(19) Europäisches Patentamt
European Patent Office
Office européen des brevets

(11) EP 4 446 325 A1

(12) **EUROPEAN PATENT APPLICATION**
published in accordance with Art. 153(4) EPC

(43) Date of publication:
**16.10.2024 Bulletin 2024/42**

(21) Application number: **22903595.1**

(22) Date of filing: **09.12.2022**

(51) International Patent Classification (IPC):
***C07D 487/04*** (2006.01) ***C07D 487/14*** (2006.01)
***C07D 491/048*** (2006.01) ***C07D 495/04*** (2006.01)
***C07D 495/14*** (2006.01) ***A61K 31/517*** (2006.01)
***A61K 31/519*** (2006.01) ***A61P 35/00*** (2006.01)
***A61P 37/00*** (2006.01) ***A61P 25/00*** (2006.01)
***A61P 31/12*** (2006.01)

(52) Cooperative Patent Classification (CPC):
**A61K 31/517; A61K 31/519; A61P 25/00; A61P 31/12; A61P 35/00; A61P 37/00; C07D 487/04; C07D 487/14; C07D 491/048; C07D 495/04; C07D 495/14**

(86) International application number:
**PCT/CN2022/137824**

(87) International publication number:
**WO 2023/104178 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.12.2021 CN 202111509875**
**28.01.2022 CN 202210106980**

(71) Applicant: **Shandong Luye Pharmaceutical Co., Ltd.**
**Shandong 264670 (CN)**

(72) Inventors:
- **TIAN, Jingwei**
**Yantai, Shandong 264670 (CN)**
- **YE, Liang**
**Yantai, Shandong 264670 (CN)**
- **LU, Jing**
**Yantai, Shandong 264670 (CN)**
- **ZHANG, Jianzhao**
**Yantai, Shandong 264670 (CN)**
- **LEI, Hui**
**Yantai, Shandong 264670 (CN)**
- **BAI, Xinfa**
**Yantai, Shandong 264670 (CN)**
- **WANG, Wenyan**
**Yantai, Shandong 264670 (CN)**
- **ZHANG, Rui**
**Yantai, Shandong 264670 (CN)**

(74) Representative: **karo IP**
**Patentanwälte PartG mbB**
**Steinstraße 16-18**
**40212 Düsseldorf (DE)**

(54) **PROTEIN KINASE INHIBITOR, PREPARATION METHOD THEREFOR, AND APPLICATION THEREOF**

(57) The present invention relates to a series of compounds for regulating activity of a protein kinase, in particular a compound for inhibiting activity of PLK1. The present invention further provides a pharmaceutical composition containing the compound, and a use of the compound and the pharmaceutical composition thereof in preparing a drug for preventing or treating diseases caused by and/or related to dysregulation of PLK1 kinase activity.

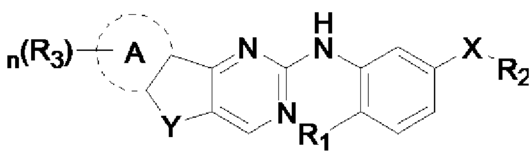

(I)

EP 4 446 325 A1

## Description

### Technical Field

The present invention belongs to the technical field of medicines, and relates to compounds and pharmaceutical compositions for the prevention or treatment of diseases related to dysregulation of protein kinase activity, as well as a method of using same and the use thereof. In particular, the present invention provides compounds that can serve as inhibitors of protein kinases, especially PLK1 kinase, and the use thereof.

### Background Art

PLK1 is a serine/threonine kinase widely present in eukaryotic cells. PLK1 is involved in most processes of cell division, mainly including the entry of cell division, rupture of the nuclear membrane, centriole duplication, complete arrangement of chromosomes, initiation of mitotic checkpoints, and cytokinesis and other biological processes.

In G2 phase, PLK1 plays an important role in the activation of cyclin B-CDK1 complex through at least two mechanisms. First, it activates CDC25C phosphatase, which in turn eliminates inhibitory phosphorylation of CDK1, thereby activating CDK1. Secondly, PLK1 induces the phosphorylation-dependent degradation of WEE1, thereby preventing further phosphorylation of CDK1 and allowing cells to enter M phase. In addition, PLK1 is also involved in the regulation of DNA damage checkpoints in G2 and M phases.

Studies have confirmed that PLK1 is overexpressed in a variety of tumor tissues. Overexpression of wild-type PLK1 may lead to multinucleation, and the expression of overactive PLK1 may allow cells to bypass the DNA damage-induced G2 arrest checkpoint. In addition, PLK1 is closely related to the occurrence and development of tumors, and the expression level of PLK1 is associated with poor prognosis of clinical patients. Therefore, PLK1 inhibitors are expected to become effective anti-tumor drug targets.

Volasertib and Onvansertib are ATP-competitive PLK1 small molecule inhibitors with rapid clinical trial progress at present. Whether used as single agents or in combination, they have shown significant anti-tumor effects in a variety of preclinical models or clinical studies. Volasertib is an injectable formulation for which an increased rate of higher-grade adverse events are observed in clinical trials. Onvansertib is an oral formulation that has shown low bioavailability in preclinical studies and a narrow safety margin in clinical trials. Therefore, there is still a need in the art for compounds with high selectivity, high activity and better safety to meet clinical needs.

### Summary of the Invention

In one embodiment of the present invention, there is provided a compound of formula (I), or a pharmaceutically acceptable salt or stereoisomer thereof:

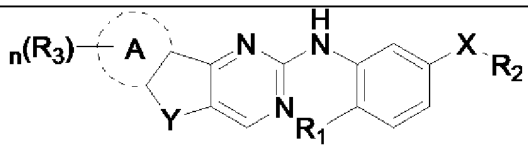

(I)

wherein:

X is independently selected from: divalent group of piperidine, piperazine, 3,8-diazabicyclo[3.2.1]octane, $-CH_2$-piperazine-, -NR-pyrrolidine-, -NR-azetidine-, or $-NR-CH_2$-pyrrolidine-; the R is independently selected from: H, D, or $C_1$-$C_6$ alkyl;

Y is independently selected from: divalent group of $-C(R_4)_2-C(R_4)_2-$, $-CR_4 = CR_4-$, $-C(R_4)_2-NR_4-$, $-NR_4-C(R_4)_2-$, $-CR_4=N-$ or $-N=CR_4-$, each of the $R_4$ is independently selected from: H, D, or Ci-Ce alkyl;

ring A is selected from the structure represented by formula (A):

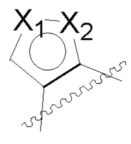

(A) ;

$X_1$ is independently selected from: C, N, O or S atoms;
$X_2$ is independently selected from: C, N, O or S atoms;
$R_1$ is independently selected from: H, D, $C_1$-$C_6$ alkyl, $C_1$-$C_6$ haloalkyl, $C_1$-$C_6$ alkoxy, or $C_1$-$C_6$ haloalkoxy;
$R_2$ is independently selected from: H, D, $C_1$-$C_6$ alkyl, wherein the $C_1$-$C_6$ alkyl is optionally substituted with one or more halogen, OH, $NH_2$, or CN group;
Each $R_3$ is independently selected from: H, D, $C_1$-$C_6$ alkyl, $C_3$-$C_6$ cycloalkyl, -C(O)NHR$_5$, wherein the $C_1$-$C_6$ alkyl is optionally substituted with one or more halogen, OH, $NH_2$, CN, or $C_1$-$C_6$ alkoxy, the $R_5$ is independently selected from: H, D, $C_1$-$C_6$ alkyl, $C_1$-$C_6$ alkoxy, HO-$C_1$-$C_6$ alkoxy, OH, $NH_2$, -NHC(O)$NH_2$, or $R_6$-S(O)$_2$-, the $R_6$ is independently selected from: $C_1$-$C_6$ alkyl, $C_1$-$C_6$ haloalkyl, or $C_3$-$C_6$ cycloalkyl.

In some embodiments of the present invention, in the compound of formula (I), or the pharmaceutically acceptable salt or stereoisomer thereof, X is preferably independently selected from:

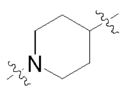 , 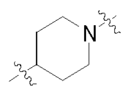

, 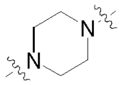 , 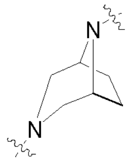 , 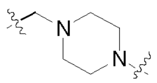 , 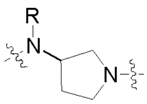 , 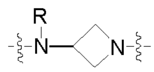 , or 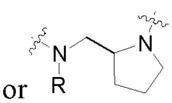 ;

the R is independently selected from: H, D, methyl, ethyl, n-propyl, or isopropyl.

In some embodiments of the present invention, in the compound of formula (I), a pharmaceutically acceptable salt or stereoisomer thereof, Y is preferably independently selected from: divalent group of - $CH_2$-$CH_2$-, -CH = CH-, -$CH_2$-NH-, -NH-$CH_2$-, -$CH_2$-N($CH_3$)-, -N($CH_3$)-$CH_2$-, -CH=N- or -N=CH-; and more preferably, -CH=CH-.

In some embodiments of the present invention, in the compound of formula (I), a pharmaceutically acceptable salt or stereoisomer thereof, $R_1$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, n-propoxy, isopropoxy, n-butoxy, sec-butoxy, tert-butoxy, n-pentyloxy, S-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, or pentachloroethoxy.

In some embodiments of the present invention, in the compound of formula (I), a pharmaceutically acceptable salt or stereoisomer thereof, $R_2$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl, wherein the terminal position of methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl is optionally substituted with one or more F, Cl, Br, OH, $NH_2$, or CN group. In some embodiments of the present invention, in the compound of formula (I), a pharmaceutically acceptable salt or stereoisomer thereof, ring A is preferably selected from a structure represented by formula (A-1), formula (A-2), formula (A-3), formula (A-4) or formula (A-5):

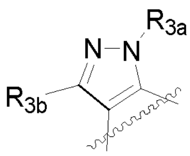
(A-1) ,
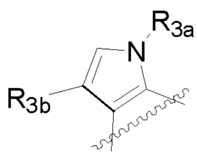
(A-2) ,
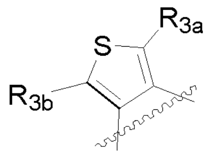
(A-3) ,
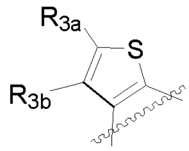
(A-4) ,
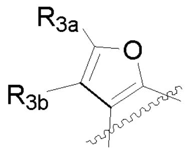
(A-5) ;

$R_{3a}$ is independently selected from: H, D, $C_1$-$C_6$ alkyl, $C_3$-$C_6$ cycloalkyl, wherein the $C_1$-$C_6$ alkyl is optionally substituted with one or more halogen, OH, $NH_2$, CN, or $C_1$-$C_6$ alkoxy, $R_{3b}$ is independently selected from: -C(O)NHR$_5$, the $R_5$ is independently selected from: H, D, $C_1$-$C_6$ alkyl, $C_1$-$C_6$ alkoxy, HO-$C_1$-$C_6$ alkoxy, OH, $NH_2$, -NHC(O)NH$_2$, or $R_6$-S(O)$_2$-, the $R_6$ is independently selected from: $C_1$-$C_6$ alkyl, $C_1$-$C_6$ haloalkyl, or $C_3$-$C_6$ cycloalkyl.

In some embodiments of the present invention, in the compound of formula (I), a pharmaceutically acceptable salt or stereoisomer thereof, $R_{3a}$ is preferably selected from: H, D, $C_1$-$C_6$ linear alkyl, $C_3$-$C_6$ cycloalkyl; the terminal position of the $C_1$-$C_6$ linear alkyl is optionally substituted with one or more F, Cl, Br, OH, $NH_2$, CN, methoxy, or ethoxy.

In some embodiments of the present invention, in the compound of formula (I), a pharmaceutically acceptable salt or stereoisomer thereof, $R_{3a}$ is particularly preferably selected from: methyl, ethyl, n-propyl, n-butyl, n-pentyl, n-hexyl, cyclopropyl, cyclobutyl, cyclopentyl or cyclohexyl, wherein the terminal position of the methyl, ethyl, n-propyl, n-butyl, n-pentyl or n-hexyl is optionally substituted with one or more F, Cl, Br, OH, $NH_2$, CN, methoxy or ethoxy.

In some embodiments of the present invention, in the compound of formula (I), a pharmaceutically acceptable salt or stereoisomer thereof, $R_{3b}$ is independently selected from: -C(O)NHR$_5$, the $R_5$ is preferably selected from: H, D, $C_1$-$C_6$ linear alkyl, $C_1$-$C_6$ linear alkoxy, HO-$C_1$-$C_6$ linear alkoxy, OH, $NH_2$, - NHC(O)NH$_2$, or $R_6$-S(O)$_2$-; the $R_6$ is independently selected from: $C_1$-$C_6$ alkyl, $C_1$-$C_6$ haloalkyl, or $C_3$-$C_6$ cycloalkyl.

In some embodiments of the present invention, in the compound of formula (I), a pharmaceutically acceptable salt or stereoisomer thereof, $R_{3b}$ is independently selected from: -C(O)NHR$_5$, the $R_5$ is particularly preferably selected from: H, D, methyl, ethyl, n-propyl, n-butyl, n-pentyl, n-hexyl, methoxy, ethoxy, n-propoxy, n-butoxy, n-pentyloxy, n-hexyloxy, HO-methoxy, HO-ethoxy, HO-n-propoxy, HO-n-butoxy, HO-n-pentyloxy, HO-n-hexyloxy, OH, $NH_2$, -NHC(O)NH$_2$, $R_6$-S(O)$_2$-, the $R_6$ is independently selected from: methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, cyclopropyl, cyclobutyl, cyclopentyl, or cyclohexyl.

In another embodiment of the present invention, there is provided a compound of formula (II), a pharmaceutically acceptable salt or stereoisomer thereof:

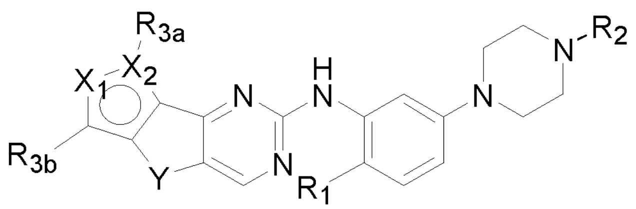
(II)

wherein:

Y is independently selected from: divalent group of -C(R$_4$)$_2$-C(R$_4$)$_2$-, -CR$_4$ = CR$_4$-, -C(R$_4$)$_2$-NR$_4$-, -NR$_4$-C(R$_4$)$_2$-, -CR$_4$=N- or -N=CR$_4$-, each of the $R_4$ is independently selected from: H, D, or Ci-Ce alkyl;

$X_1$ is independently selected from: C, N, O or S atoms;

$X_2$ is independently selected from: C, N, O or S atoms;

$R_1$ is independently selected from: H, D, $C_1$-$C_6$ alkyl, $C_1$-$C_6$ haloalkyl, $C_1$-$C_6$ alkoxy, or $C_1$-$C_6$ haloalkoxy;

$R_2$ is independently selected from: H, D, $C_1$-$C_6$ alkyl, wherein the $C_1$-$C_6$ alkyl is optionally substituted with one or more halogen, OH, $NH_2$, or CN group;

$R_{3a}$ is independently selected from: H, D, $C_1$-$C_6$ alkyl, or $C_3$-$C_6$ cycloalkyl, wherein the Ci-Ce alkyl is optionally substituted with one or more halogen, OH, $NH_2$, or CN.

$R_{3b}$ is independently selected from: -C(O)NHR$_5$,

the $R_5$ is independently selected from: H, D, $C_1$-$C_6$ alkyl, $C_1$-$C_6$ alkoxy, HO-$C_1$-$C_6$ alkoxy, OH, $NH_2$, - NHC(O)NH$_2$, or $R_6$-S(O)$_2$-, the $R_6$ is independently selected from: $C_1$-$C_6$ alkyl, $C_1$-$C_6$ haloalkyl, or $C_3$-$C_6$ cycloalkyl.

In some embodiments of the present invention, in the compound of formula (II), a pharmaceutically acceptable salt or stereoisomer thereof, Y is preferably independently selected from: divalent group of - $CH_2$-$CH_2$-, -CH = CH-, -$CH_2$-NH-, -NH-$CH_2$-, -$CH_2$-N($CH_3$)-, -N($CH_3$)-$CH_2$-, -CH=N- or -N=CH-; and more preferably, -CH=CH-.

In some embodiments of the present invention, in the compound of formula (II), a pharmaceutically acceptable salt or stereoisomer thereof, $R_1$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, n-propoxy, isopropoxy, n-butoxy, sec-butoxy, tert-butoxy, n-pentyloxy, S-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, or pentachloroethoxy.

In some embodiments of the present invention, in the compound of formula (II), a pharmaceutically acceptable salt or stereoisomer thereof, $R_2$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl, wherein the terminal position of methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl is optionally substituted with one or more F, Cl, Br, OH, $NH_2$, or CN group. In some embodiments of the present invention, in the compound of formula (II), a pharmaceutically acceptable salt or stereoisomer thereof, $R_{3a}$ is preferably selected from: H, D, $C_1$-$C_6$ linear alkyl, $C_3$-$C_6$ cycloalkyl; the terminal position of the Ci-Ce linear alkyl is optionally substituted with one or more F, Cl, Br, OH, $NH_2$, or CN group.

In some embodiments of the present invention, in the compound of formula (II), a pharmaceutically acceptable salt or stereoisomer thereof, $R_{3a}$ is particularly preferably selected from: methyl, ethyl, n-propyl, n-butyl, n-pentyl, n-hexyl, cyclopropyl, cyclobutyl, cyclopentyl or cyclohexyl, wherein the terminal position of the methyl, ethyl, n-propyl, n-butyl, n-pentyl or n-hexyl is optionally substituted with one or more F, Cl, Br, OH, $NH_2$, or CN group.

In some embodiments of the present invention, in the compound of formula (II), a pharmaceutically acceptable salt or stereoisomer thereof, $R_{3b}$ is independently selected from: -C(O)NH$R_5$, the $R_5$ is preferably selected from: H, D, $C_1$-$C_6$ linear alkyl, $C_1$-$C_6$ linear alkoxy, HO-$C_1$-$C_6$ linear alkoxy, OH, $NH_2$, - NHC(O)$NH_2$, or $R_6$-S$(O)_2$-; the $R_6$ is independently selected from: $C_1$-$C_6$ alkyl, $C_1$-$C_6$ haloalkyl, or $C_3$-$C_6$ cycloalkyl.

In some embodiments of the present invention, in the compound of formula (II), a pharmaceutically acceptable salt or stereoisomer thereof, $R_{3b}$ is independently selected from: -C(O)NH$R_5$, the $R_5$ is particularly preferably selected from: H, D, methyl, ethyl, n-propyl, n-butyl, n-pentyl, n-hexyl, methoxy, ethoxy, n-propoxy, n-butoxy, n-pentyloxy, n-hexyloxy, HO-methoxy, HO-ethoxy, HO-n-propoxy, HO-n-butoxy, HO-n-pentyloxy, HO-n-hexyloxy, OH, $NH_2$, -NHC(O)$NH_2$, $R_6$-S$(O)_2$-, the $R_6$ is independently selected from: methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, cyclopropyl, cyclobutyl, cyclopentyl, or cyclohexyl.

In another embodiment of the present invention, there is provided a compound of formula (IIa), formula (IIb), formula (IIc), formula (IId) or formula (IIe), or a pharmaceutically acceptable salt or stereoisomer thereof:

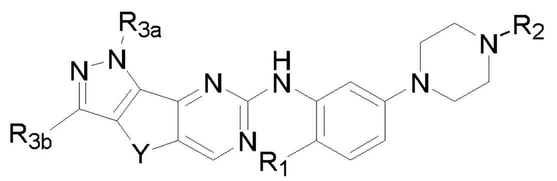
(IIa)
,

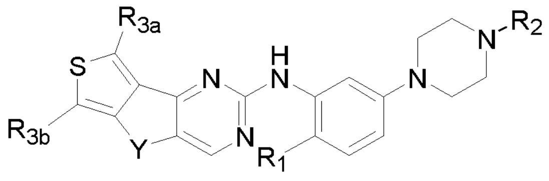
(IIb)
,

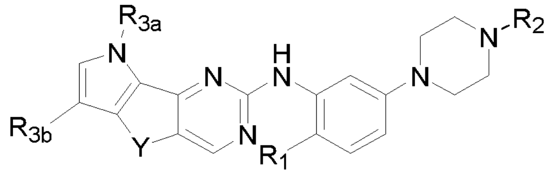
(IIc)
,

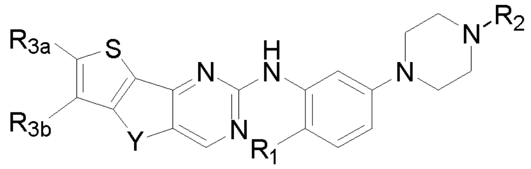
(IId)
,

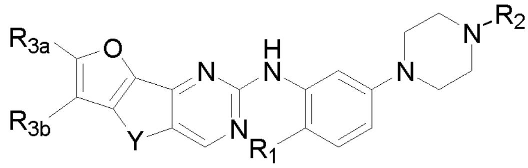
(IIe)
,

wherein:

Y is independently selected from: divalent group of $-C(R_4)_2-C(R_4)_2-$, $-CR_4 = CR_4-$, $-C(R_4)_2-NR_4-$, $-NR_4-C(R_4)_2-$, $-CR_4=N-$ or $-N=CR_4-$, each of the $R_4$ is independently selected from: H, D, or Ci-Ce alkyl;
$R_1$ is independently selected from: H, D, $C_1$-$C_6$ alkyl, $C_1$-$C_6$ haloalkyl, $C_1$-$C_6$ alkoxy, or $C_1$-$C_6$ haloalkoxy;
$R_2$ is independently selected from: H, D, $C_1$-$C_6$ alkyl, wherein the $C_1$-$C_6$ alkyl is optionally substituted with one or more halogen, OH, $NH_2$, or CN group;
$R_{3a}$ is independently selected from: H, D, $C_1$-$C_6$ alkyl, or $C_3$-$C_6$ cycloalkyl, wherein the Ci-Ce alkyl is optionally substituted with one or more halogen, OH, $NH_2$, or CN.
$R_{3b}$ is independently selected from: $-C(O)NHR_5$,
the $R_5$ is independently selected from: H, D, $C_1$-$C_6$ alkyl, $C_1$-$C_6$ alkoxy, HO-$C_1$-$C_6$ alkoxy, OH, $NH_2$, - $NHC(O)NH_2$, or $R_6$-$S(O)_2$-, the $R_6$ is independently selected from: $C_1$-$C_6$ alkyl, $C_1$-$C_6$ haloalkyl, or $C_3$-$C_6$ cycloalkyl.

In some embodiments of the present invention, in the compound represented by formula (IIa), formula (IIb), formula (IIc), formula (IId) or formula (IIe), a pharmaceutically acceptable salt or stereoisomer thereof, Y is preferably independently selected from: divalent group of $-CH_2-CH_2-$, $-CH = CH-$, $-CH_2-NH-$, $-NH-CH_2-$, $-CH_2-N(CH_3)-$, $-N(CH_3)-CH_2-$, $-CH=N-$ or $-N=CH-$; and more preferably, $-CH=CH-$.

In some embodiments of the present invention, in the compound represented by formula (IIa), formula (IIb), formula (IIc), formula (IId) or formula (IIe), a pharmaceutically acceptable salt or stereoisomer thereof, $R_1$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, n-propoxy, isopropoxy, n-butoxy, sec-butoxy, tert-butoxy, n-pentyloxy, S-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, or pentachloroethoxy.

In some embodiments of the present invention, in the compound represented by formula (IIa), formula (IIb), formula (IIc), formula (IId) or formula (IIe), a pharmaceutically acceptable salt or stereoisomer thereof, $R_2$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl, wherein the terminal position of methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl is optionally substituted with one or more F, Cl, Br, OH, $NH_2$, or CN group.

In some embodiments of the present invention, in the compound represented by formula (IIa), formula (IIb), formula (IIc), formula (IId) or formula (IIe), a pharmaceutically acceptable salt or stereoisomer thereof, $R_{3a}$ is preferably

selected from: H, D, $C_1$-$C_6$ linear alkyl, $C_3$-$C_6$ cycloalkyl; the terminal position of the $C_1$-$C_6$ linear alkyl is optionally substituted with one or more F, Cl, Br, OH, $NH_2$, or CN group.

In some embodiments of the present invention, in the compound represented by formula (IIa), formula (IIb), formula (IIc), formula (IId) or formula (IIe), a pharmaceutically acceptable salt or stereoisomer thereof, $R_{3a}$ is particularly preferably selected from: methyl, ethyl, n-propyl, n-butyl, n-pentyl, n-hexyl, cyclopropyl, cyclobutyl, cyclopentyl or cyclohexyl, wherein the terminal position of the methyl, ethyl, n-propyl, n-butyl, n-pentyl or n-hexyl is optionally substituted with one or more F, Cl, Br, OH, $NH_2$, or CN group.

In some embodiments of the present invention, in the compound represented by formula (IIa), formula (IIb), formula (IIc), formula (IId) or formula (IIe), a pharmaceutically acceptable salt or stereoisomer thereof, $R_{3b}$ is independently selected from: -C(O)NHR$_5$, the $R_5$ is preferably selected from: H, D, $C_1$-$C_6$ linear alkyl, $C_1$-$C_6$ linear alkoxy, HO-$C_1$-$C_6$ linear alkoxy, OH, $NH_2$, -NHC(O)$NH_2$, or $R_6$-S(O)$_2$-; the $R_6$ is independently selected from: $C_1$-$C_6$ alkyl, $C_1$-$C_6$ haloalkyl, or $C_3$-$C_6$ cycloalkyl.

In some embodiments of the present invention, in the compound represented by formula (IIa), formula (IIb), formula (IIc), formula (IId) or formula (IIe), a pharmaceutically acceptable salt or stereoisomer thereof, $R_{3b}$ is independently selected from: -C(O)NHR$_5$, the $R_5$ is particularly preferably selected from: H, D, methyl, ethyl, n-propyl, n-butyl, n-pentyl, n-hexyl, methoxy, ethoxy, n-propoxy, n-butoxy, n-pentyloxy, n-hexyloxy, HO-methoxy, HO-ethoxy, HO-n-propoxy, HO-n-butoxy, HO-n-pentyloxy, HO-n-hexyloxy, OH, $NH_2$, -NHC(O)$NH_2$, $R_6$-S(O)$_2$-, the $R_6$ is independently selected from: methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, cyclopropyl, cyclobutyl, cyclopentyl, or cyclohexyl.

In some embodiments of the present invention, in the compound of formula (IIa), a pharmaceutically acceptable salt or stereoisomer thereof, Y is -CR$_4$= CR$_4$-; Each of the $R_4$ is independently selected from: H, D, or Ci-Ce alkyl; preferably Y is -CH=CH-.

$R_1$ is independently selected from: H, D, $C_1$-$C_6$ alkyl, $C_1$-$C_6$ haloalkyl, $C_1$-$C_6$ alkoxy, or Ci-Ce haloalkoxy.

$R_2$ is independently selected from: H, D, $C_1$-$C_6$ alkyl, wherein the $C_1$-$C_6$ alkyl is optionally substituted with one or more halogen, OH, $NH_2$, or CN group.

$R_{3a}$ is independently selected from: H, D, $C_1$-$C_6$ alkyl, or $C_3$-$C_6$ cycloalkyl, wherein the Ci-Ce alkyl is optionally substituted with one or more halogen, OH, $NH_2$, or CN.

$R_{3b}$ is independently selected from: -C(O)NHR$_5$, the $R_5$ is independently selected from: H, D, $C_1$-$C_6$ alkyl, $C_1$-$C_6$ alkoxy, HO-$C_1$-$C_6$ alkoxy, OH, $NH_2$, -NHC(O)$NH_2$, or $R_6$-S(O)$_2$-, the $R_6$ is independently selected from: $C_1$-$C_6$ alkyl, $C_1$-$C_6$ haloalkyl, or $C_3$-$C_6$ cycloalkyl.

In some embodiments of the present invention, in the compound of formula (IIa), a pharmaceutically acceptable salt or stereoisomer thereof, $R_1$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, n-propoxy, isopropoxy, n-butoxy, sec-butoxy, tert-butoxy, n-pentyloxy, S-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, or pentachloroethoxy.

In some embodiments of the present invention, in the compound of formula (IIa), a pharmaceutically acceptable salt or stereoisomer thereof, $R_2$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl, wherein the terminal position of methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl is optionally substituted with one or more F, Cl, Br, OH, $NH_2$, or CN group. In some embodiments of the present invention, in the compound of formula (IIa), a pharmaceutically acceptable salt or stereoisomer thereof, $R_{3a}$ is preferably selected from: H, D, $C_1$-$C_6$ linear alkyl, $C_3$-$C_6$ cycloalkyl; the terminal position of the Ci-Ce linear alkyl is optionally substituted with one or more F, Cl, Br, OH, $NH_2$, or CN group.

In some embodiments of the present invention, in the compound of formula (IIa), a pharmaceutically acceptable salt or stereoisomer thereof, $R_{3a}$ is preferably selected from: methyl, ethyl, n-propyl, n-butyl, n-pentyl, n-hexyl, cyclopropyl, cyclobutyl, cyclopentyl or cyclohexyl, wherein the terminal position of the methyl, ethyl, n-propyl, n-butyl, n-pentyl or n-hexyl is optionally substituted with one or more F, Cl, Br, OH, $NH_2$, or CN group; $R_{3a}$ is particularly preferably selected from methyl, ethyl, cyclopropyl, -$CH_2CH_2F$, -$CH_2CF_3$, or -$CH_2CH_2OH$.

In some embodiments of the present invention, in the compound of formula (IIa), a pharmaceutically acceptable salt or stereoisomer thereof, $R_{3b}$ is independently selected from: -C(O)NHR$_5$, the $R_5$ is preferably selected from: H, D, $C_1$-$C_6$ linear alkyl, $C_1$-$C_6$ linear alkoxy, HO-$C_1$-$C_6$ linear alkoxy, OH, $NH_2$, - NHC(O)$NH_2$, or $R_6$-S(O)$_2$-, the $R_6$ is inde-

pendently selected from: $C_1$-$C_6$ alkyl, $C_1$-$C_6$ haloalkyl, or $C_3$-$C_6$ cycloalkyl.

In some embodiments of the present invention, in the compound of formula (IIa), a pharmaceutically acceptable salt or stereoisomer thereof, $R_{3b}$ is independently selected from: -C(O)NHR$_5$, the $R_5$ is particularly preferably selected from: H, D, methyl, ethyl, n-propyl, n-butyl, n-pentyl, n-hexyl, methoxy, ethoxy, n-propoxy, n-butoxy, n-pentyloxy, n-hexyloxy, HO-methoxy, HO-ethoxy, HO-n-propoxy, HO-n-butoxy, HO-n-pentyloxy, HO-n-hexyloxy, OH, $NH_2$, -NHC(O)$NH_2$, $R_6$-S(O)$_2$-, the $R_6$ is independently selected from: methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, cyclopropyl, cyclobutyl, cyclopentyl, or cyclohexyl.

In some aspects of the present invention, the compound of formula (I) is selected from:

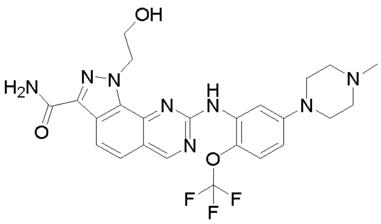
1

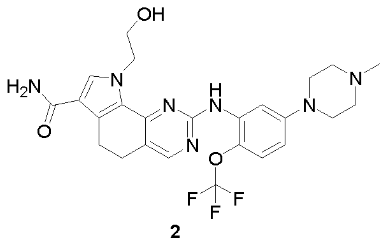
2

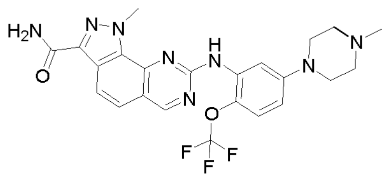
3

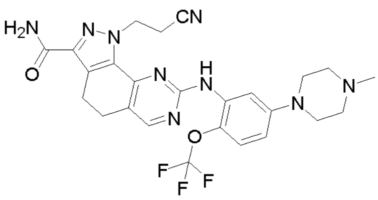
4

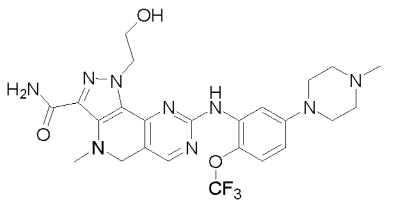
5

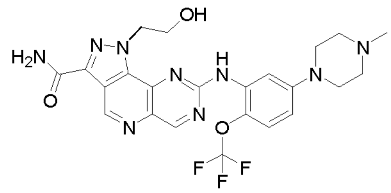
6

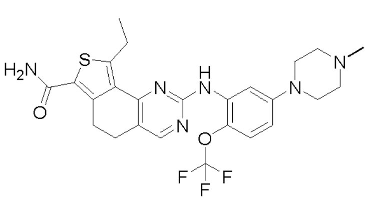
7

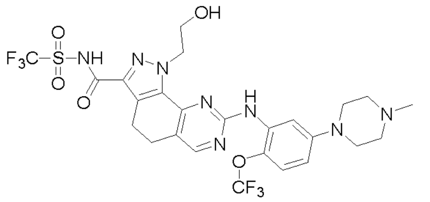
8

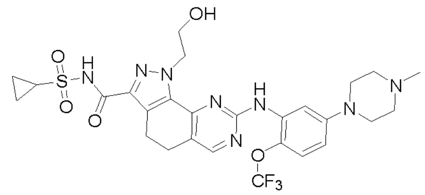
9

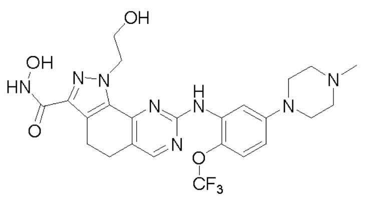
10

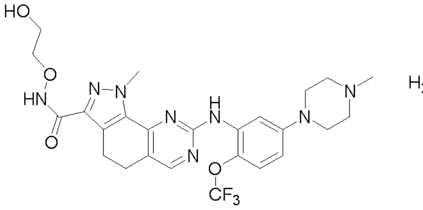
11

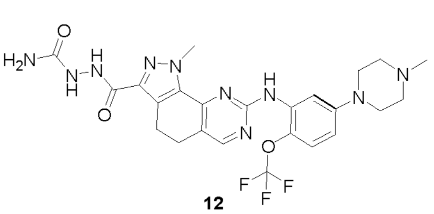
12

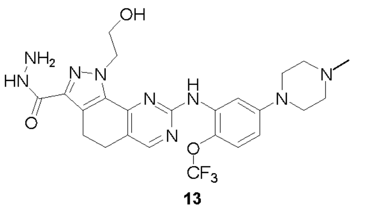
13

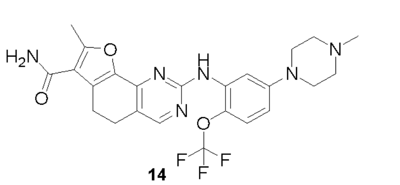
14

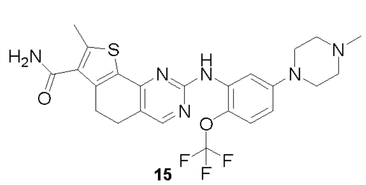
15

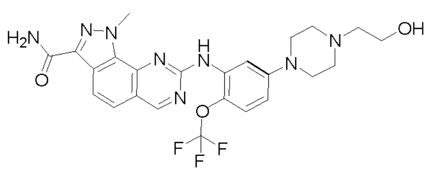
16

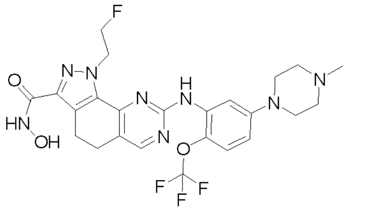
17

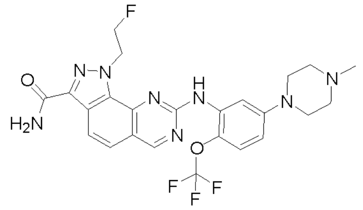
18

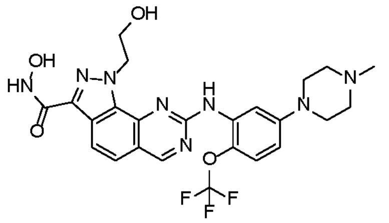
19

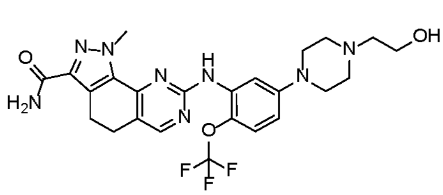
20

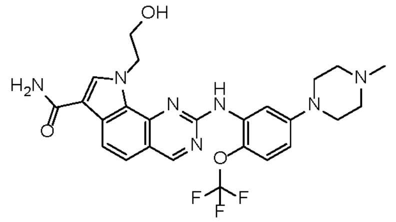
21

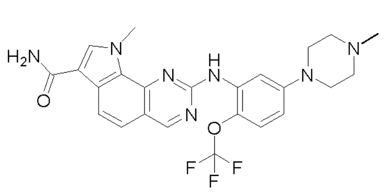
22

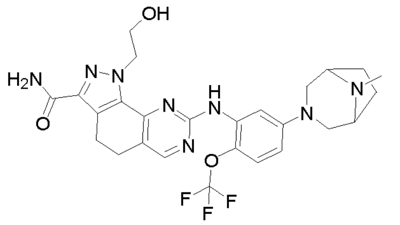
23

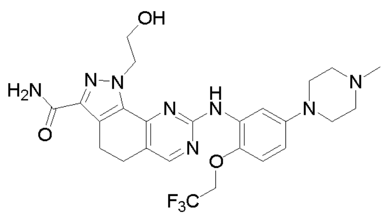
24

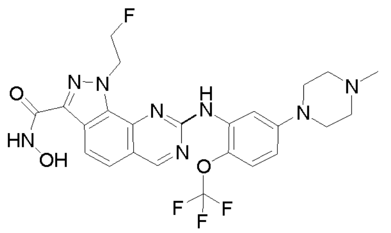
25

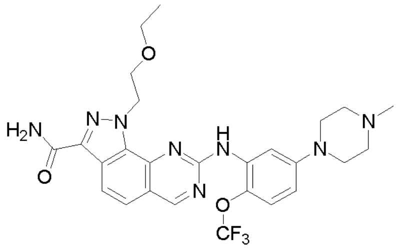
26

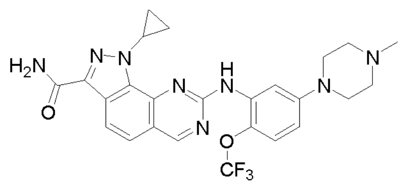
27

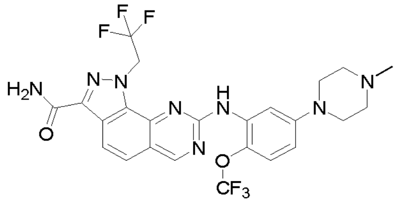
28

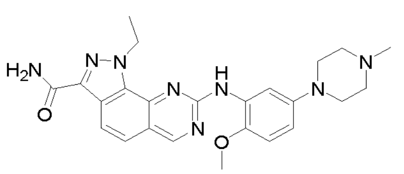
29

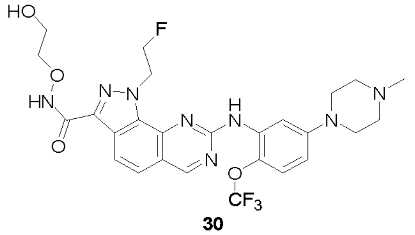
30

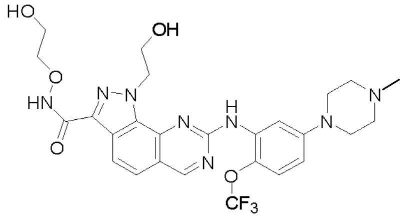
31

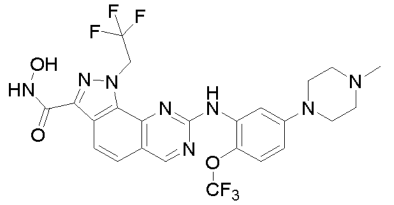
32

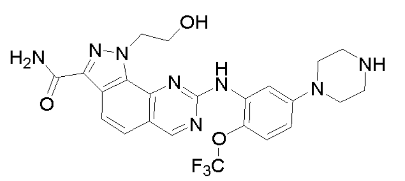
33

.

The present invention also provides a pharmaceutical composition, including any one of the above compounds or the pharmaceutically acceptable salt thereof and a pharmaceutically acceptable carrier. The pharmaceutical composition can be prepared into various pharmaceutically acceptable dosage forms, such as tablets, capsules, oral liquid preparations, granules, injections or various sustained and controlled release preparations. The pharmaceutical composition can be administered by oral administration or parenteral mode (such as intravenous, subcutaneous or topically). Dosage of administration can be appropriately adjusted according to the age, gender and disease type of the patients, and the daily dosage is generally about 1-200 mg. The present invention also provides the use of the above-mentioned compound or the pharmaceutically acceptable salt threof or the pharmaceutical composition in preparing a drug for the prevention or treatment of diseases caused by and/or related to dysregulation of protein kinase activity, preferably in preparing a drug for the prevention or treatment of diseases caused by and/or related to dysregulation of PLK1 kinase activity. The disease is selected from a cancer, a cell proliferative disease, a viral infection, an autoimmune and neurodegenerative disease.

Such a cancer includes, but not limited to: a cancer such as bladder cancer, breast cancer, colon cancer, kidney cancer, liver cancer, lung cancer (including small cell lung cancer), esophageal cancer, gallbladder cancer, ovarian cancer, pancreatic cancer, gastric cancer, cervical cancer, thyroid cancer, prostate cancer and skin cancer (including squamous cell cancer); hematopoietic tumors of the lymphoid system, including leukemia, acute lymphocitic leukemia, acute lymphoblastic leukemia, B-cell lymphoma, T-cell lymphoma, Hodgkin's lymphoma, non-Hodgkin's lymphoma, hairy cell lymphoma, and Burkett's lymphoma; hematopoietic tumors of the myeloid system, including acute and chronic myeloid leukemias, myelodysplastic syndromes, and promyelocytic leukemia; tumors of mesenchymal origin, including fibrosarcoma and rhabdomyosarcoma; tumors of the central and peripheral nervous system, including astrocytomas, neuroblastomas, gliomas, and schwannomas; other tumors, including melanoma, seminoma, teratoma, osteosarcoma, xeroderma pigmentosum, keratoacanthoma, follicular thyroid carcinoma, and Kaposi's sarcoma.

Such cell proliferative diseases are such as benign prostatic hyperplasia, familial adenoma, polyposis, neurofibromatosis, psoriasis, vascular smooth cell proliferation associated with atherosclerosis, pulmonary fibrosis, arthritis, glomerulonephritis, and postoperative stenosis and restenosis.

**Definition and Description**

Unless otherwise stated, the following terms and phrases used herein are intended to have the following meanings. A specific term or phrase should not be considered uncertain or unclear unless specifically defined, but should be understood in its ordinary meaning. When a trade name appears herein, it is intended to refer to the corresponding commodity or an active ingredient thereof.

The "pharmaceutically acceptable" in the present invention refers to compounds, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The "pharmaceutically acceptable salt" in the present invention refers to a salt of the compound of the present invention, which is prepared from the compound having specific substituents found in the present invention with relatively non-toxic acids or bases. When compounds of the present invention contain relatively basic functional groups, acid addition salts can be obtained by contacting the neutral form of such compounds with a sufficient amount of acid, either in pure solution or a suitable inert solvent. Examples of pharmaceutically acceptable acid addition salts include inorganic acid salts or organic acid salts; and also include salts of amino acids (such as arginine), and salts of organic acids such as glucuronic acid. Certain specific compounds of the present invention contain acidic functional groups and thus can be converted to any acid addition salt.

Certain compounds of the present invention may have asymmetric carbon atoms (optical centers) or double bonds. Racemates, diastereomers, geometric isomers and individual isomers are encompassed within the scope of the present invention.

The compounds of the present invention may exist in specific geometric or stereoisomeric forms. The present invention contemplates all such compounds, including cis and trans isomers, (-)- and (+)-enantiomers, (*R*)- and (*S*)-enantiomers, diastereomers, (*D*)-isomers, (*L*)-isomers, and racemic mixtures and other mixtures thereof, such as enantiomerically or diastereomerically enriched mixtures, all of which fall within the scope of the present invention. Additional asymmetric carbon atoms may be present in a substituent such as an alkyl group. All these isomers and mixtures thereof are encompassed within the scope of the present invention.

The pharmaceutically acceptable salts of the present invention can be synthesized from a parent compound containing acid radicals or base radicals by conventional chemical methods. In general, a method for preparing such salts comprises: in water or an organic solvent or a mixture of both, reacting these compounds in free acid or base forms with a stoichiometric amount of a suitable base or acid to prepare the salts.

The term "pharmaceutically acceptable carrier" refers to any preparation or carrier medium that can deliver an

effective amount of active substances in the present invention, does not interfere with the biological activity of the active substances, and has no toxic or side effects on a host or patient. Representative carriers include, but are not limited to: a binder, a filler, a lubricant, a disintegrant, a wetting agent, a dispersing agent, a solubilizer, a suspending agent, etc.

The present invention is intended to include all isotopes of atoms present in the compounds of the present invention. The isotopes comprise those atoms with the same atomic number but different mass numbers. By way of general example, and not as a limitation, isotopes of hydrogen comprise deuterium and tritium. Isotopes of carbon comprise $^{13}C$ and $^{14}C$. Isotope-labeled compounds of the present invention can generally be prepared by using an appropriate isotope-labeled reagent in place of a non-labeled reagent otherwise used by means of conventional techniques known to a person skilled in the art or by means of methods analogous to those described herein.

Unless otherwise specified, the term "alkyl" is used to represent a linear or branched saturated hydrocarbon group, which may be monosubstituted (e.g., $-CH_2F$) or polysubstituted (e.g., $-CF_3$) and may be monovalent (e.g., methyl), divalent (e.g., methylene) or polyvalent (e.g., methine). For example, $C_1$-$C_6$ represents 1 to 6 carbons, $C_{1-6}$ is selected from $C_1$, $C_2$, $C_3$, $C_4$, $C_5$, or $C_6$; Examples of alkyl include methyl (Me), ethyl (Et), propyl (e.g., n-propyl and isopropyl), butyl (e.g., n-butyl, isobutyl, s-butyl and t-butyl), pentyl (e.g., n-pentyl, isopentyl, neopentyl and 1-ethylpropyl), hexyl (e.g., n-hexyl, isohexyl, 1,1-dimethylbutyl, 2,2-dimethylbutyl, 3,3-dimethylbutyl and 2-ethylbutyl), etc.

Unless otherwise specified, the term "halo" or "halogen" by itself or as part of another substituent means a fluorine, chlorine, bromine or iodine atom.

"Haloalkyl" is used to represent a monohalogenated/polyhalogenated linear or branched alkyl group. Typical haloalkyl includes $C_{1-6}$ haloalkyl, for example: $C_1$, $C_2$, $C_3$, $C_4$, $C_5$, or $C_6$ haloalkyl. For example, examples of $C_1$-$C_6$ haloalkyl includes but not limited to: fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, and pentachloroethyl.

Unless otherwise specified, the "alkoxy" represents the above alkyl (including cycloalkyl or haloalkyl) having a specific number of carbon atoms and connected via an oxygen bridge. Typical alkoxy includes $C_{1-6}$ alkoxy, such as: $C_1$, $C_2$, $C_3$, $C_4$, $C_5$, or $C_6$ alkoxy, $C_3$, $C_4$, $C_5$, or $C_6$ cycloalkoxy, $C_1$, $C_2$, $C_3$, $C_4$, $C_5$, or $C_6$ haloalkoxy. Examples of alkoxy include, but are not limited to: methoxy, ethoxy, n-propoxy, isopropoxy, n-butoxy, sec-butoxy, tert-butoxy, n-pentoxy, S-pentoxy, hexyloxy, or 2-ethylbutoxy. Examples of cycloalkoxy include, but are not limited to: cyclopropoxy, cyclobutoxy, cyclopentoxy, and cyclohexoxy. Examples of haloalkoxy include, but are not limited to: fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, or pentachloroethoxy.

Unless otherwise specified, cycloalkyl includes any stable cyclic or polycyclic hydrocarbon group, and any carbon atom is saturated, which may be monosubstituted or polysubstituted and may be monovalent, divalent or polyvalent. Examples of these cycloalkyl groups include, but are not limited to, cyclopropyl, cyclobutyl, cyclopentyl, cyclohexyl, and the like.

Compounds are named by hand or ChemDraw® software, and commercially available compounds are named by the supplier catalog names.

## Brief Description of the Drawings

Fig. 1 shows in vivo pharmacodynamic study of compound 1 in mice - tumor growth curve in animal.
Fig. 2. shows in vivo pharmacodynamic study of compound 1 in mice - body weight during test in animal
Fig. 3. shows in vivo pharmacodynamic study of compound 18 in mice - tumor growth curve in animal
Fig. 4. shows in vivo pharmacodynamic study of compound 18 in mice - body weight during test in animal

## Detailed Description of Embodiments

The present invention is further described below in conjunction with specific examples and test examples, but the scope of the present invention is not limited in any way.

In the following examples, the following reagent abbreviations have the following meanings. If not defined, they have meanings well-known in the art.

| Abbreviations | Reagent meaning/Chinese name |
|---|---|
| PTSA | sodium p-toluenesulfonate |
| LiHMDS | lithium bis(trimethylsilyl)amide |

(continued)

| Abbreviations | Reagent meaning/Chinese name |
|---|---|
| DMF-DMA | N,N-dimethylformamide dimethyl acetal |
| DMF | N,N-dimethylformamide |
| TFA | trifluoroacetic acid |
| TEMPO | 2,2,6,6-tetramethylpiperidine oxide |
| TosMIC | p-toluenesulfonylmethyl isonitrile |
| TFAA | trifluoroacetic anhydride |
| HOBt | 1-hydroxybenzotriazole |
| DIEA | N,N-diisopropylethylamine |
| EDCI | 1-ethyl-(3-dimethylaminopropyl)carbodiimide hydrochloride |
| DDQ | 2,3-dichloro-5,6-dicyano-1,4-benzoquinone |
| CAN | ceric ammonium nitrate |
| NMP | N-methylpyrrolidone |
| DMAP | 4-(dimethylamino)pyridine |
| CDI | 1,1'-carbonyldiimidazole |
| DAST | diethylamine sulfur trifluoride |
| HATU | 2-(7-azabenzotiiazol-1-yl)-N,N,N',N'-tetramethyluronium hexafluorophosphate |
| TBDPSCI | tert-butyldiphenylchlorosilane |
| DavePhos | 2-dicyclohexylphosphino-2'-(N,N-dimethylamine)biphenyl |
| Pd2(dba)3 | tris(dibenzylideneacetone)dipalladium |
| KHMDS | potassium bis(trimethylsilyl)amide |
| TMSCI | trimethylchlorosilane |
| BFMO | N1,N2-bis(furan-2-ylmethyl)oxalamide |
| DCM | dichloromethane |

**Example 1: Synthesis of compound 1**

Synthesis route:

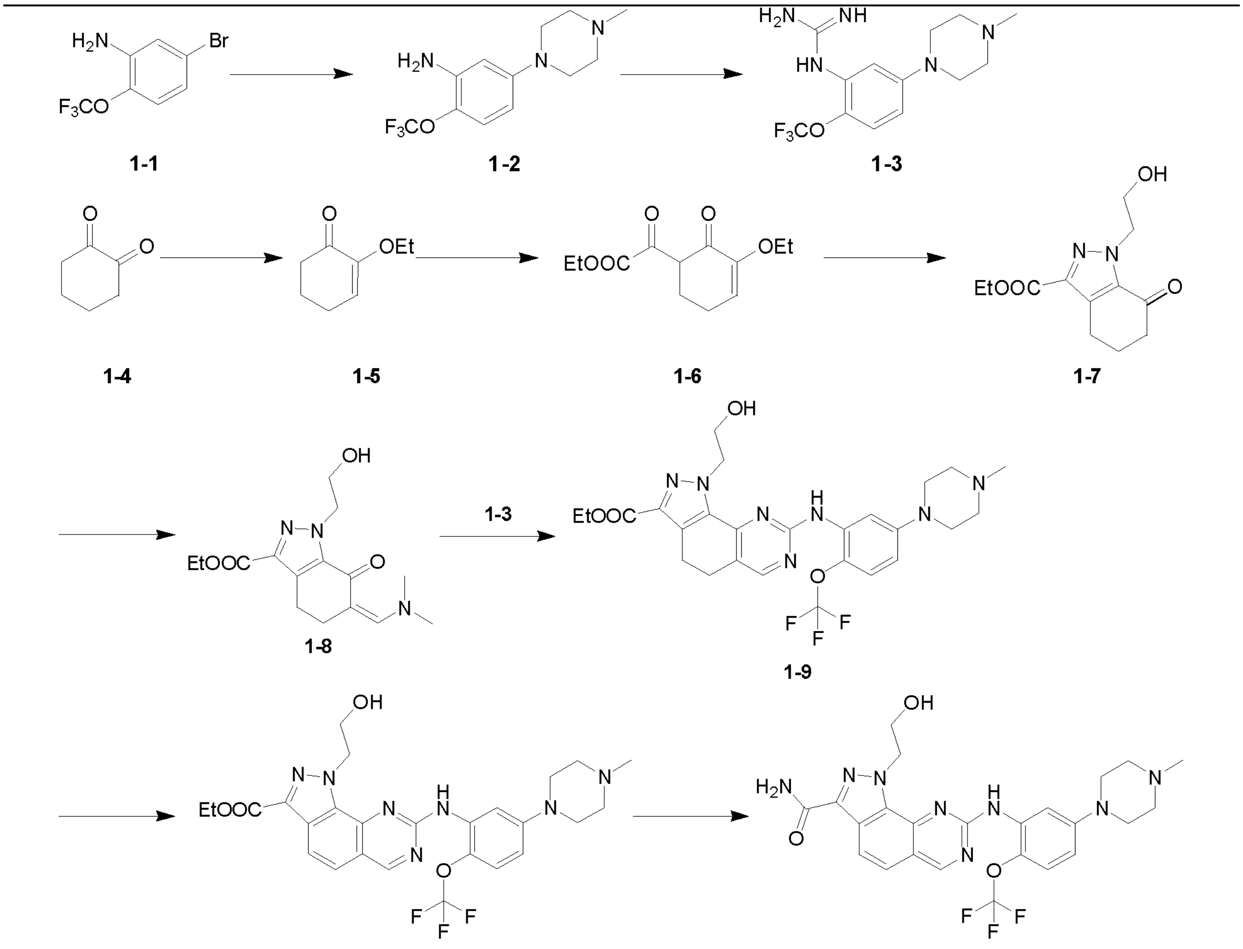

1-1 1-2 1-3

1-4 1-5 1-6 1-7

1-8 1-9

1-10 1

1.1 Synthesis of intermediate **1-2**

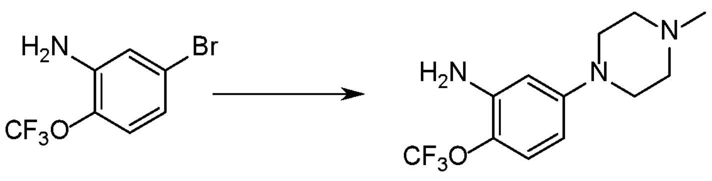

**1-1** **1-2**

Intermediate **1-1** (20.0 g, 78.1 mmol), bis(dibenzylideneacetone)palladium (0.715 g, 0.78 mmol), and 2-dicyclohexylphosphino-2'-(N,N-dimethylamine)-biphenyl (0.615 g, 1.56 mmol) were dissolved in tetrahydrofuran (32 mL), and N-methylpiperazine (12.5 g, 125 mmol) and lithium bis(trimethylsilyl)amide (1.00 M, 187 mL) were added at 0°C. The mixture was reacted at 70°C for 3 h. Water (200 mL) was added to quench the reaction, and dichloromethane (100 mL × 3) was added for extraction. The organic phases were combined, dried over anhydrous sodium sulfate, filtered, and concentrated under reduced pressure. The residue was subjected to silica gel column chromatography (methanol/dichloromethane = 1%-10%) to obtain intermediate **1-2** (15.5 g, yield 72.2%, brown solid). MS (ESI) m/z = 276.0 $[M+H]^+$.

1.2 Synthesis of intermediate **1-3**

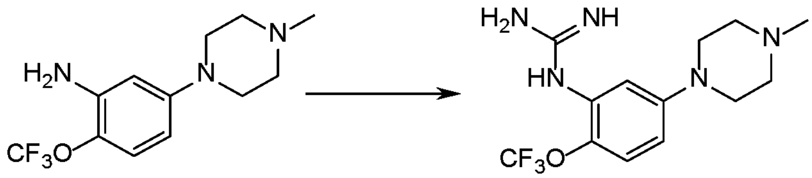

1-2 1-3

Intermediate **1-2** (0.372 g, 1.35 mmol) was dissolved in 6 N HCl (2 mL), and cyanamide (0.455 g, 10.8 mmol) was added. The mixture was reacted at 80°C for 12 h. The reaction liquid was concentrated under reduced pressure, and the residue was subjected to column chromatography (C18, acetonitrile/water = 0%-10%). The intermediate **1-3** (0.19 g, yield 45%, brown solid) was obtained. MS (ESI) m/z =318.1 $[M+H]^+$.

1.3 Synthesis of intermediate **1-5**

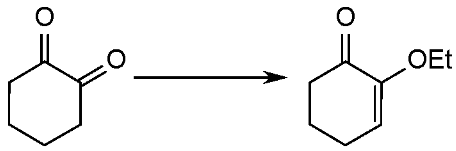

**1-4** **1-5**

Intermediate **1-4** (100 g, 892 mmol) was dissolved in toluene (1000 mL) and ethanol (123 g, 2680 mmol), PTSA (15.4 g, 89.2 mmol) was added, and the mixture was reacted at 120°C for 12 h. Water (1000 mL × 2) was added for extraction. The organic phases were collected, dried over anhydrous sodium sulfate, filtered, and the organic phase was concentrated under reduced pressure. The residue was subjected to silica gel column chromatography (ethyl acetate/petroleum ether = 0%-50%) to obtain intermediate **1-5** (57.9 g, yield 46.4%, oil).

Synthesis of intermediate **1-6**

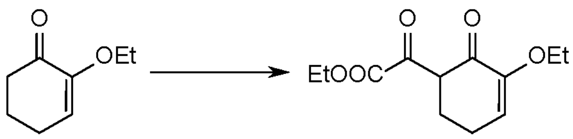

**1-5** **1-6**

Intermediate **1-5** (30.0 g, 214 mmol) was dissolved in tetrahydrofuran (450 mL), LiHMDS (1 M, 256 mL) was added dropwise at -50.0°C, and the mixture was stirred for 30 min. Diethyl oxalate (37.8 g, 258 mmol) was added and the mixture was reacted at room temperature for 16 h. Saturated ammonium chloride (500 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-20%) to obtain intermediate 1-6 (25.0 g, yield 48.6%, oil). MS (ESI) m/z = 241.1 $[M+H]^+$.

1.5 Synthesis of intermediate **1-7**

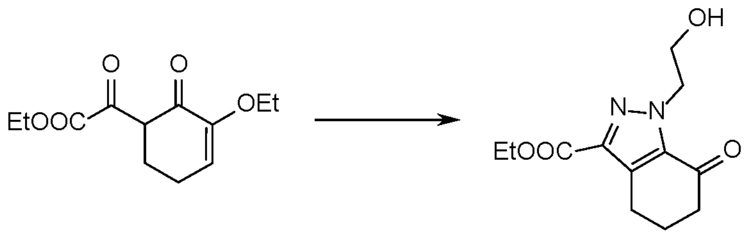

1-6 1-7

Intermediate **1-6** (25.0 g, 104 mmol) was dissolved in acetic acid (120 mL), hydroxyethylhydrazine (8.16 g, 107 mmol) was added, and the mixture was reacted at room temperature for 15 h. water (500 mL) was added to quench the reaction, saturated $Na_2CO_3$ (800 mL) was added, and dichloromethane (800 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-5%) to obtain intermediate **1-7** (19.9 g, yield 76%, yellow solid). MS (ESI) m/z = 253.0 $[M+H]^+$.

1.6 Synthesis of intermediate **1-8**

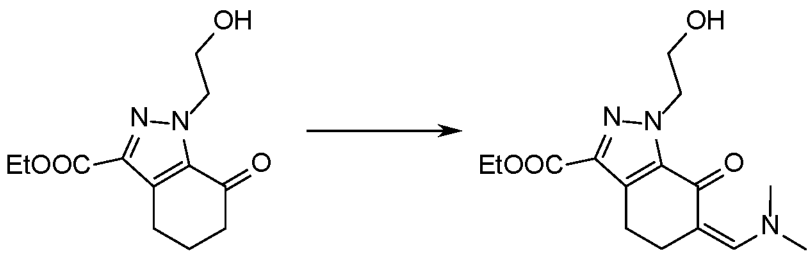

1-7 1-8

Intermediate **1-7** (10.0 g, 39.6 mmol) was dissolved in DMF-DMA (100 mL) and the mixture was reacted at 110°C for 12 h. The reaction liquid was concentrated under reduced pressure, and the residue was dissolved in methyl tertiary ether and the mixture was stirred for 3 h. The resulting product was filtered, the filter cake was collected, and dried to obtain intermediate **1-8** (6.0 g, crude product), which was directly used in the next step.

1.7 Synthesis of intermediate **1-9**

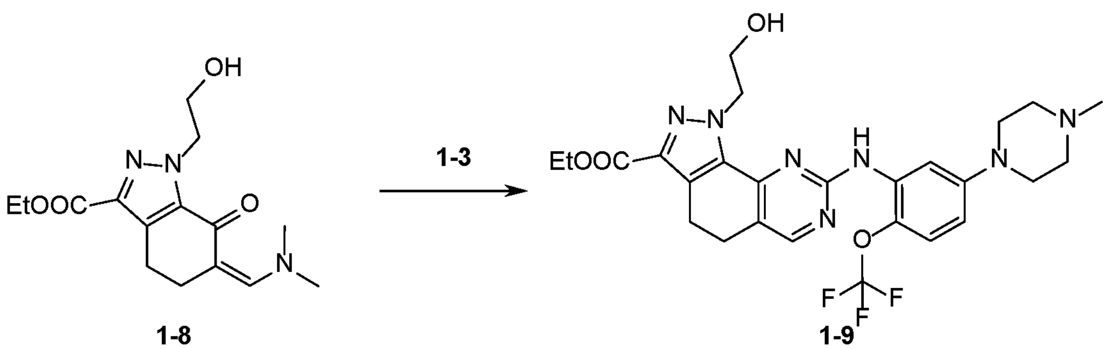


1-8 1-9

Intermediate **1-8** (1.95 g, 6.34 mmol) and intermediate **1-3** (2.03 g, 6.41 mmol) were dissolved in DMF (20 mL) and the mixture was reacted at 110°C for 12 h. The reaction liquid was concentrated under reduced pressure, and the residue was subjected to silica gel column chromatography (ethyl acetate/petroleum ether = 0%-5%) to obtain intermediate **1-9** (1.90 g, yield 53.3%, yellow solid). MS (ESI) m/z = 562.2 $[M+H]^+$.

1.8 Synthesis of intermediate **1-10**

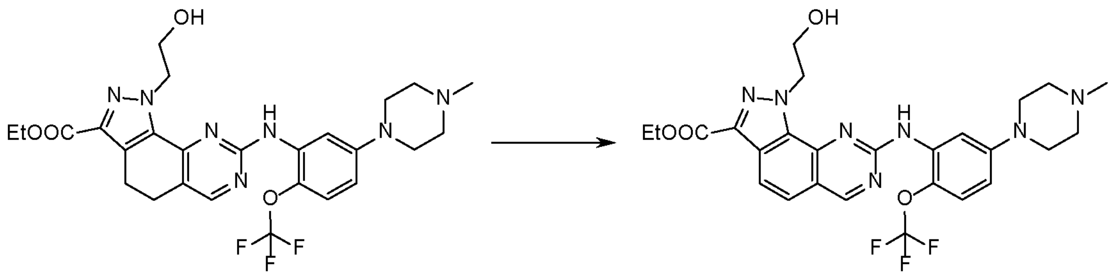

**1-9** **1-10**

Intermediate **1-9** (100 mg, 178 μmol) and DDQ (100 mg, 440 μmol) were dissolved in dioxane (20 mL) and the mixture was reacted at 100°C for 12 h. Saturated $NaHCO_3$(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatogram (C18 150*40 mm* 10 μm; mobile phase: [water (TFA)-acetonitrile]; B%: 25%-55%, 10 min). Intermediate **1-10** (10 mg, yield 10%, brown solid) was obtained. MS (ESI) m/z =560.1 $[M+H]^+$.

1.9 Synthesis of compound 1

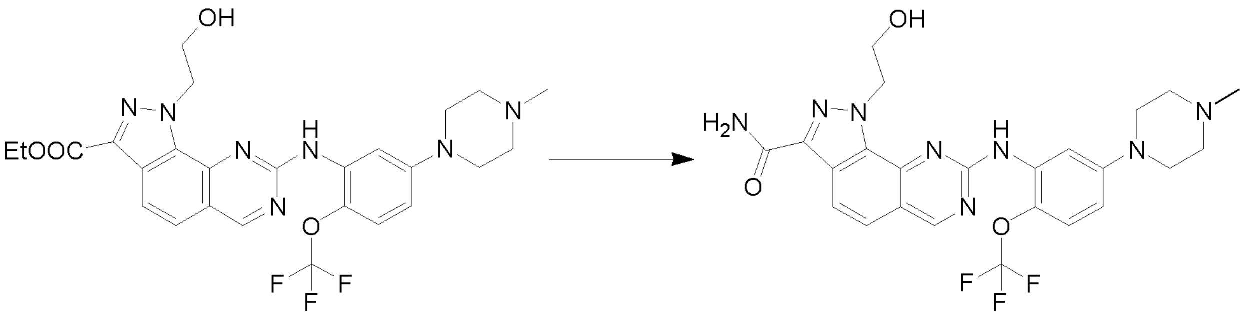

**1-10** **1**

Intermediate **1-10** (160 mg, 285 μmol) was dissolved in NMP (6 mL), and $NH_3$/MeOH (50.0 mL) was added. The mixture was reacted for 48 h under sealed conditions at 80.0°C. The reaction liquid was concentrated under reduced pressure, and the residue was purified by preparative liquid chromatogram (C18 30* 150 mm*7 μm; mobile phase: [water (TFA)-acetonitrile]; B%: 10%-80%, 40 min). Compound 1 (56.7 mg, yield 37.5%, yellow solid) was obtained. MS (ESI) m/z =531.2 $[M+H]^+$. $^1H$ NMR (400 MHz, DMSO-*d6*) δ 9.56 (s, 1H), 9.34 (s, 1H), 7.89 (d, *J* = 8.6 Hz, 1H), 7.67 (d, *J* = 8.8 Hz, 1H), 7.33 - 7.20 (m, 2H), 6.88 - 6.91 (m, 1H), 4.91 (t, *J* = 4.9 Hz, 2H), 4.65 (d, *J* = 5.3 Hz, 1H), 4.41 (q, *J* = 7.1 Hz, 2H), 3.70 - 3.54 (m, 3H), 3.28 - 3.10 (m, 3H), 3.10 - 3.09 (m, 1H), 3.16 - 3.09 (m, 1H), 2.34 - 2.19 (m, 3H), 1.39 (t, *J* = 7.1 Hz, 3H), 1.36 - 1.36 (m, 1H), 1.31 (t, *J* = 7.1 Hz, 1H), 1.26 - 1.12 (m, 1H).

**Example 2: Synthesis of compound 2**

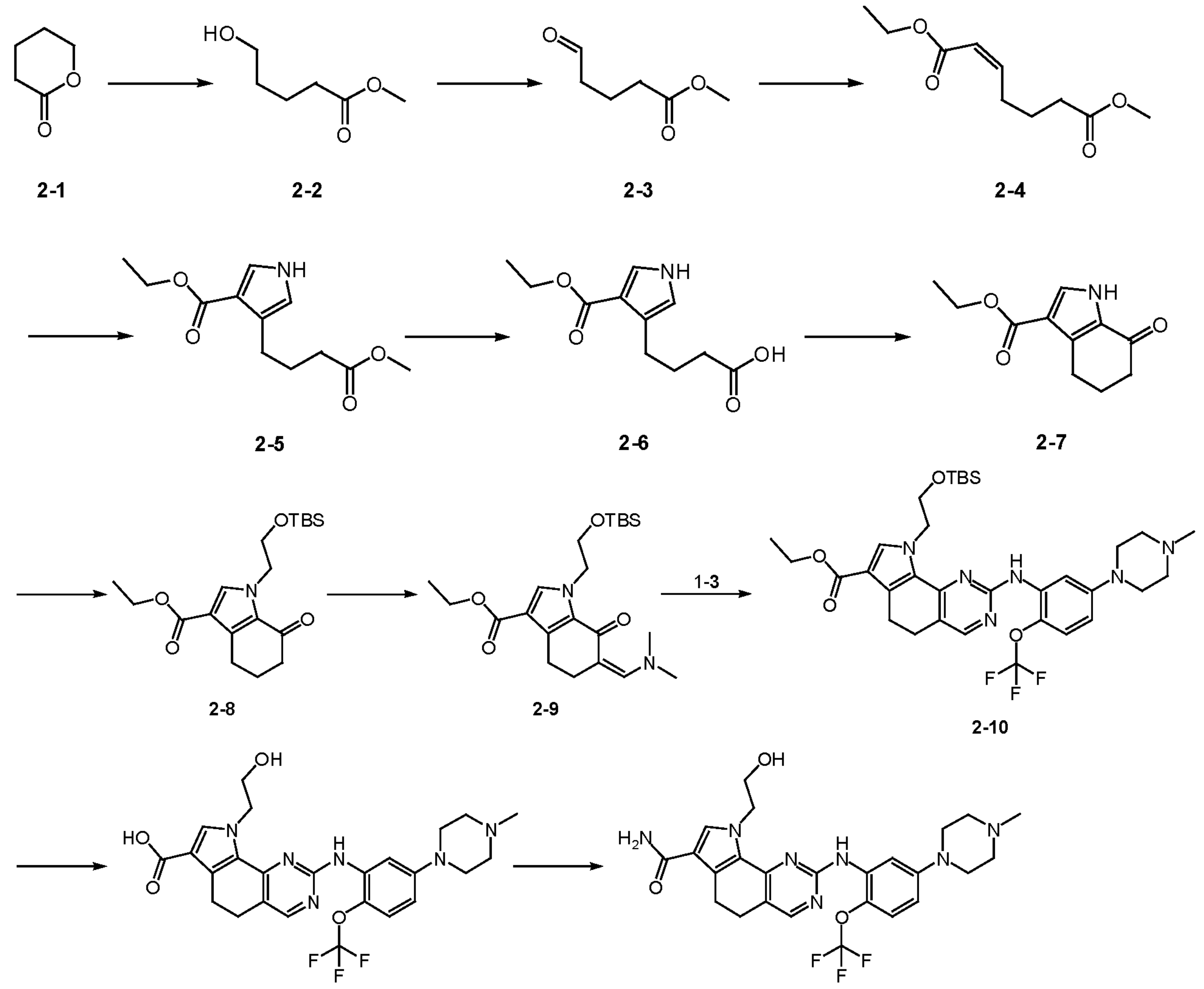


2-1 2-2 2-3 2-4

2-5 2-6 2-7

2-8 2-9 2-10

2-11 2

2.1 Synthesis of compound **2-2**

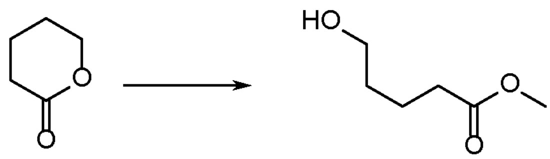

**2-1** **2-2**

Intermediate **2-1** (5.00 g, 49.9 mmol) was dissolved in methanol (20 mL), concentrated sulfuric acid (0.33 mL) was added dropwise, and the mixture was reacted at 65°C for 48 h under nitrogen protection. Potassium carbonate (2.00 g) was added and the mixture was stirred at room temperature for 0.5 h. The reaction liquid was filtered, and the filtrate was collected and concentrated under reduced pressure. The residue was dissolved in water (50 mL), and extracted with ethyl acetate (50 mL × 3). The organic phases were combined, dried over anhydrous sodium sulfate, filtered, and the organic phase was concentrated under reduced pressure. The intermediate **2-2** (5.48 g, crude product) was obtained, which was used directly in the next step. MS (ESI) m/z = 133.2 $[M+H]^+$.

2.2 Synthesis of intermediate **2-3**

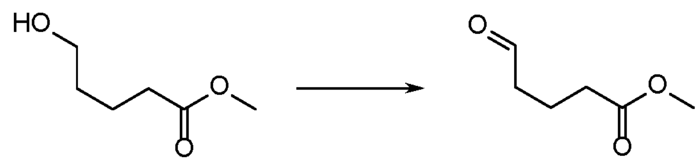

2-2 2-3

Intermediate **2-2** (5.85 g, 44.3 mmol), TEMPO (0.35 g, 2.20 mmol) and KBr (0.53 g, 4.40 mmol) were dissolved in dichloromethane (60 mL), NaClO (14.3 g, 23% aqueous solution) was added at -5°C, and the mixture was reacted at 0°C for 1 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. Intermediate **2-3** (5.46 g, crude product, oil) was obtained and directly used in the next step.

2.3 Synthesis of intermediate **2-4**

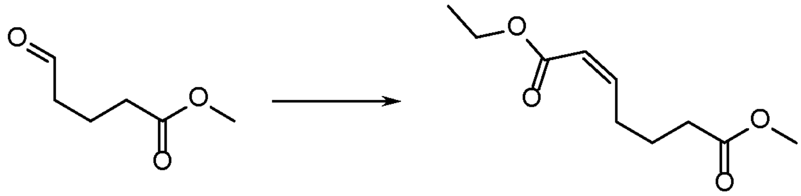

2-3 2-4

Intermediate **2-3** (5.46 g, 42.0 mmol) and ethyl (triphenylphosphaalkene)acetate (14.9 g, 42.8 mmol) were dissolved in toluene (50 mL) and the mixture was reacted at 120°C for 12 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-15%) to obtain intermediate **2-4** (2.18 g, yield 21.7%, oil). MS (ESI) m/z = 201.2 $[M+H]^+$.

2.4 Synthesis of intermediate **2-5**

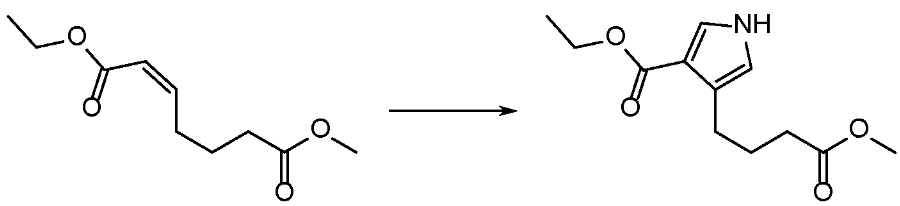

2-4 2-5

Under nitrogen protection and -78°C, a solution of 1 M LiHMDS in tetrahydrofuran (10.8 mL, 10.8 mmol) was added dropwise to 30 mL of a solution of TosMIC (2.11 g, 10.8 mmol) in tetrahydrofuran. The obtained product was stirred for 40 min at -78°C, a solution of intermediate **2-4** (1.10 g, 5.90 mmol) in tetrahydrofuran (30 mL) was added dropwise, and the mixture was reacted under stirring for 20 min and then brought to room temperature for reaction. After the reaction was completed by detection, water (100 mL) was added to quench the reaction, and dichloromethane (100 mL × 3) was added for extraction. The organic phases were combined, dried over anhydrous sodium sulfate, filtered, and the organic phase was concentrated under reduced pressure. The residue was subjected to silica gel column chromatography (ethyl acetate:petroleum ether = 30%) to obtain intermediate **2-5** (1.82 g, yield 70.6%, oil). MS (ESI) m/z = 240.0 $[M+H]^+$.

2.5 Synthesis of intermediate **2-6**

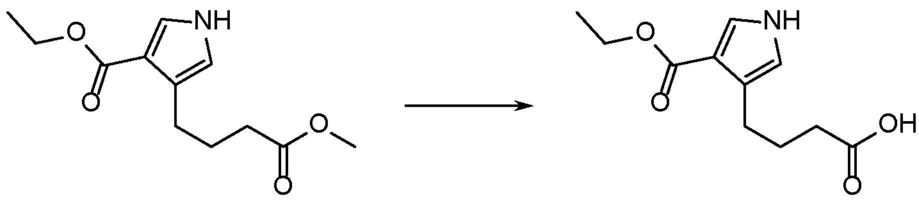

**2-5** **2-6**

Intermediate **2-5** (1.00 g, 4.20 mmol) was dissolved in dioxane (20 mL) and water (5 mL), lithium hydroxide (0.100 g, 0.420 mmol) was added, and the mixture was reacted at room temperature for 12 h. The pH was adjusted to acidic with 1 N HCl, water (50 mL) was added, and ethyl acetate (50 mL × 3) was added for extraction. The organic phases were combined, dried over anhydrous sodium sulfate, filtered, and the organic phase was concentrated under reduced pressure. The intermediate **2-6** (0.974 g, crude product) was obtained, which was used directly in the next step. MS (ESI) m/z = 226.0 $[M+H]^+$.

2.6 Synthesis of intermediate **2-7**

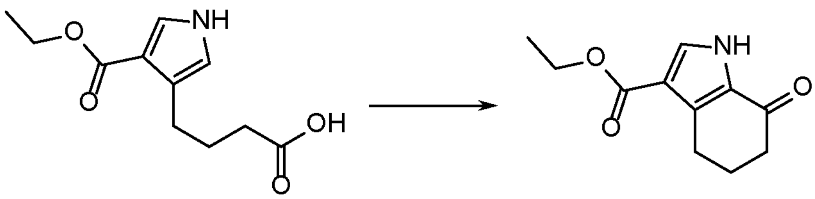

**2-6** **2-7**

Intermediate **2-6** (0.974 g, 4.32 mmol) was dissolved in trifluoroacetic acid (5 mL), TFAA (0.908 g, 4.32 mmol) was added, and the mixture was reacted at room temperature for 1 h. The reaction liquid was concentrated under reduced pressure. The residue was subjected to silica gel column chromatography (ethyl acetate/petroleum ether = 0%-32%) to obtain intermediate **2-7** (0.46 g, yield 50.8%, oil). MS (ESI) m/z = 208.0 $[M+H]^+$.

2.7 Preparation of intermediate **2-8**

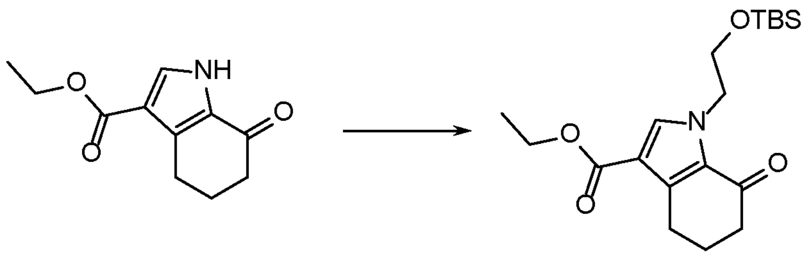

**2-7** **2-8**

Intermediate **2-7** (0.44 g, 2.12 mmol) and (2-bromoethoxy)-tert-butyldimethylsilane (0.61 g, 2.55 mmol) were dissolved in DMF (5 mL), $K_2CO_3$ (0.587 g, 4.24 mmol) was added, and the mixture was reacted at room temperature for 12 h. Water (50 mL) was added to quench the reaction, and ethyl acetate (50 mL × 3) was added for extraction. The

organic phases were combined, dried over anhydrous sodium sulfate, filtered, and the organic phase was concentrated under reduced pressure. The residue was subjected to silica gel column chromatography (ethyl acetate/petroleum ether = 0%-20%) to obtain intermediate **2-8** (0.64 g, yield 83.7%, oil). MS (ESI) m/z = 366.2 [M+H]$^+$.

2.8 Synthesis of intermediate **2-9**

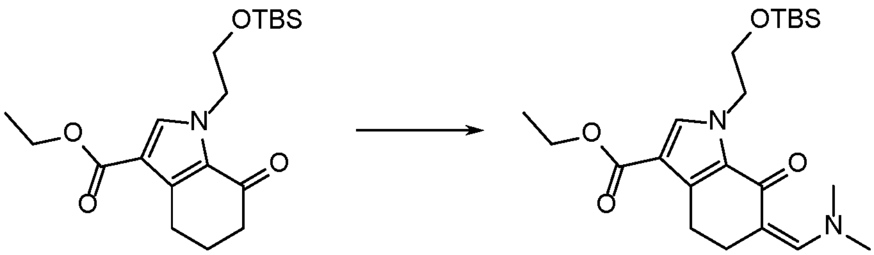

**2-8** **2-9**

Intermediate **2-8** (0.5 g, 1.37 mmol) was dissolved in DMF (5 mL), tert-butoxy bis(dimethylamino)methane (953 mg, 5.47 mmol) was added, and the mixture was reacted under microwave at 150°C for 4 h. The mixture was cooled to room temperature, water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The intermediate **2-9** (0.6 g, crude product) was obtained, which was used directly in the next step.

2.9 Synthesis of intermediate **2-10**

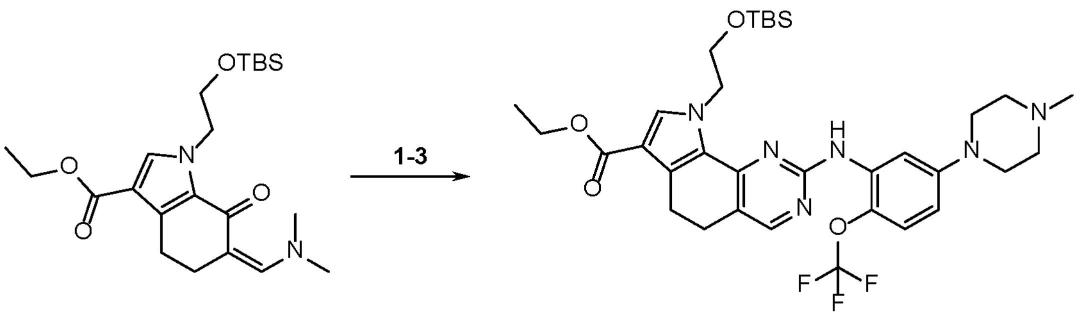


**2-9** **2-10**

Intermediates **2-9** (0.45 g, 1.07 mmol) and **1-3** (0.34 g, 1.07 mmol) were dissolved in DMF (10 mL). The mixture was reacted at 120°C for 12 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (methanol/dichloromethane = 0%-6%) to obtain intermediate **2-10** (0.30 g, yield 42.1%, brown solid). MS (ESI) m/z = 675.4 [M+H]$^+$.

2.10 Synthesis of intermediate **2-11**

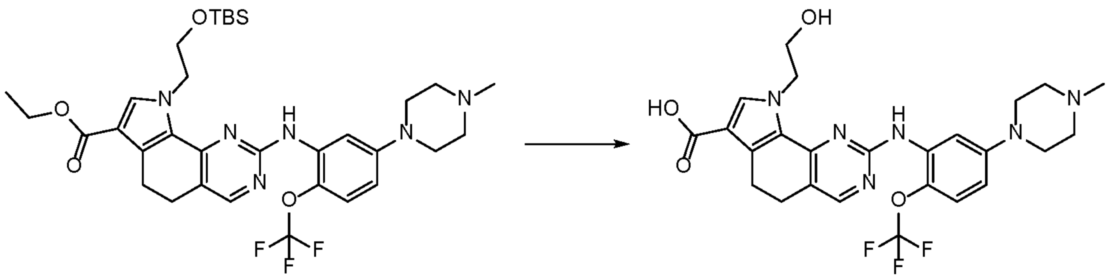

2-10 2-11

Intermediate **2-10** (0.289 g, 0.428 mmol) and LiOH (0.103 g, 4.28 mmol) were dissolved in methanol (7 mL) and water (3 mL). The mixture was reacted at 50°C for 24 h. The reaction liquid was adjusted to acidic pH with dilute hydrochloric acid and concentrated under reduced pressure. The residue was subjected to column chromatography (C18, acetonitrile/water = 0%-20%) to obtain intermediate **2-11** (0.16 g, yield 72.7%, pink solid). MS (ESI) m/z = 533.1$[M+H]^+$.

2.11 Synthesis of compound **2**

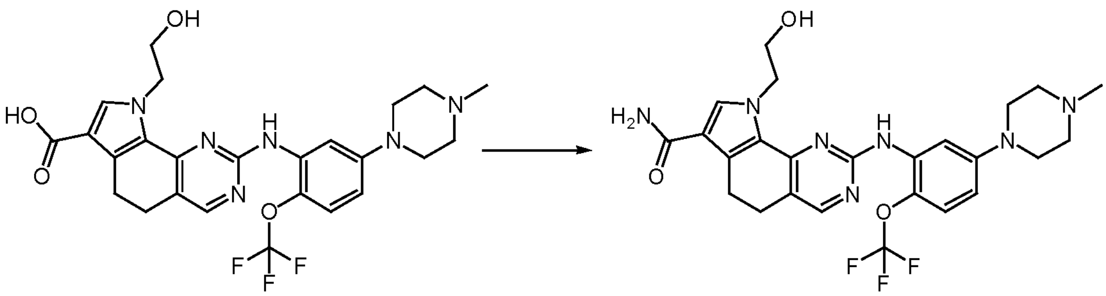

2-11 2

Intermediate **2-11** (0.30 g, 0.563 mmol), ammonium chloride (0.302 g, 5.63 mmol), HOBt (0.0989 g, 0.732 mmol) and DIEA (0.291 g, 2.25 mmol) were dissolved in dichloromethane (10 mL), and EDCI (0.14 g, 0.732 mmol) was added. The mixture was reacted at 50°C for 12 h. The reaction liquid was concentrated under reduced pressure. The residue was subjected to column chromatography (C18, acetonitrile/water (0.1% FA) = 38%) to obtain compound 2 (0.10 g, yield 34.2%, yellow solid). MS (ESI) m/z = 532.2 $[M+H]^+$. $^1$H NMR(400 MHz, DMSO-$d_6$)δ 8.52 (s, 1H), 8.11 (s, 1H), 7.52 (s, 1H), 7.36 - 7.14 (m, 3H), 6.94 - 6.66 (m, 2H), 4.68 (t, $J$ = 4.8 Hz, 1H), 4.32 (t, $J$ = 4.8 Hz, 2H), 3.42 - 3.37 (m, 2H), 3.17 - 3.09 (m, 4H), 2.99 - 2.92 (m, 2H), 2.73 - 2.66 (m, 2H), 2.46 - 2.41 (m, 4H), 2.22 (s, 3H).

**Example 3: Synthesis of compound 3**

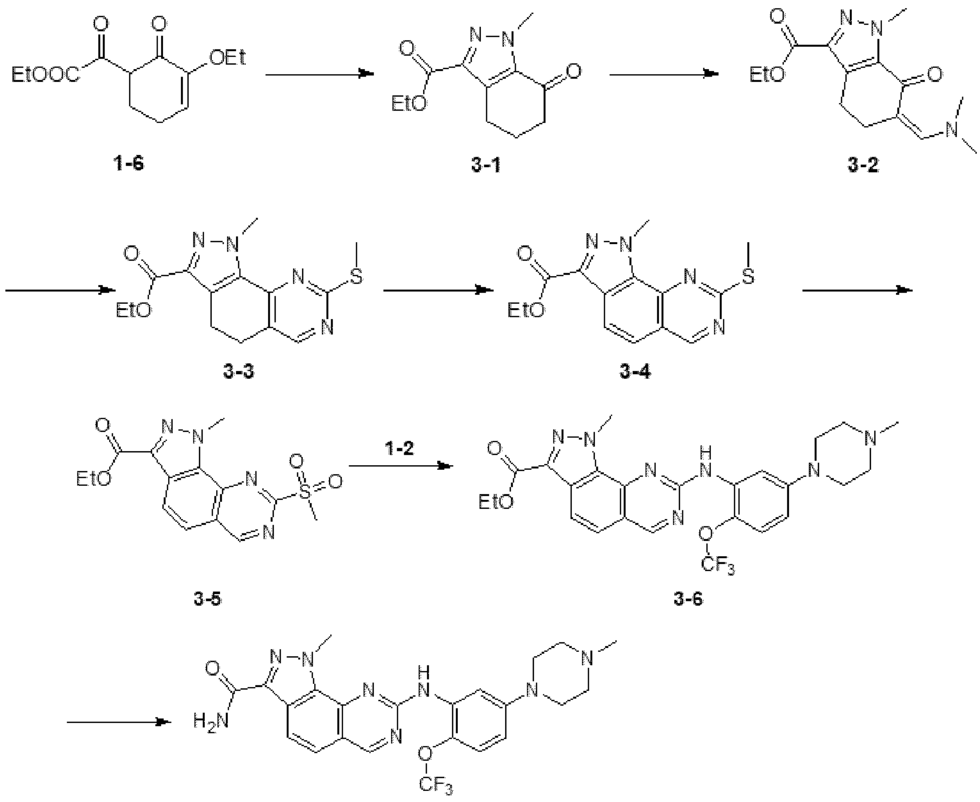


1-6 3-1 3-2

3-3 3-4

3-5 3-6

3

3.1 Synthesis of intermediate **3-1**

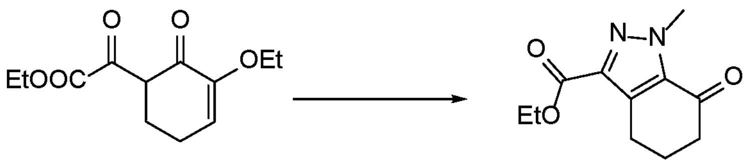

**1-6** **3-1**

Intermediate **1-6** (20 g, 83.2 mmol) was dissolved in acetic acid (100 mL), methylhydrazine (6.2 g, 53.8 mmol) was added at 0°C, and the mixture was reacted at room temperature for 2 h. The reaction liquid was concentrated under reduced pressure, and the residue was dissolved in water (500 mL) and adjusted to pH 7-8 with saturated $NaHCO_3$. Dichloromethane (100 mL × 3) was added for extraction, the organic phases were combined, dried over anhydrous sodium sulfate, and filtered, the organic phase was concentrated under reduced pressure, and the residue was subjected to silica gel column chromatography (dichloromethane 100%) to obtain the intermediate **3-1** (13.0 g, yield 70.3%, off-white solid). MS (ESI) m/z =223.2 $[M+H]^+$.

3.2 Synthesis of intermediate **3-2**

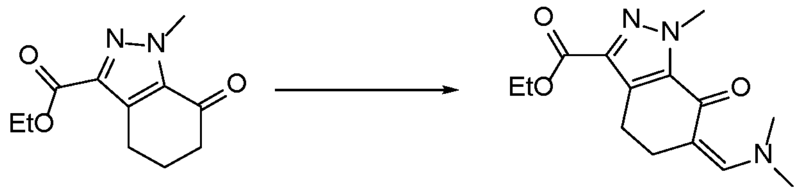

3-1 3-2

Intermediate **3-1** (1 g, 4.50 mmol) was dissolved in DMF (5 mL), tert-butoxy bis(dimethylamino)methane (1.83 g, 9.00 mmol) was added, and the mixture was reacted at 80°C for 4 h. The reaction liquid was concentrated under reduced pressure to obtain intermediate **3-2** (1.2 g, crude product), which was directly used in the next step.

3.3 Synthesis of intermediate **3-3**

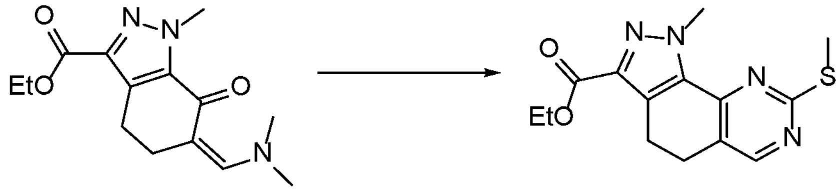

3-2 3-3

Intermediate **3-2** (5 g, 18.0 mmol) was dissolved in DMF (50 mL), 2-methyl-2-mercaptourea sulfate (3.01 g, 10.8 mmol) and potassium acetate ( 1.77 g, 18.0 mmol) were added, and the mixture was reacted 95°C for 4 h. Water (200 mL) was added to quench the reaction and the solid was precipitated, filtered and collected. The solid was purified by silica gel column chromatography (petroleum ether : ethyl acetate = 2 : 1) to obtain intermediate **3-3**(2.76 g, yield 50.5%, yellow solid). MS (ESI) m/z = 305.1 $[M+H]^+$.

3.4 Synthesis of intermediate **3-4**

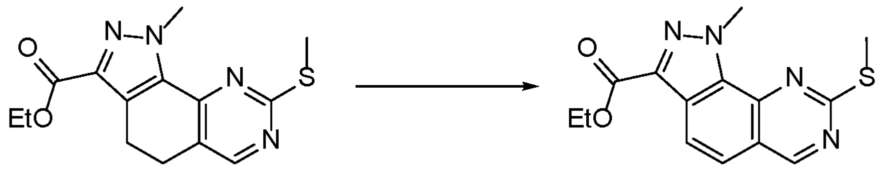

3-3 3-4

Intermediate **3-3** (1.32 g, 4.34 mmol) was dissolved in chlorobenzene (50 mL), and DDQ (1.97 g, 8.67 mmol) was added. The mixture was reacted at 135°C for 16 h. The reaction liquid was concentrated under reduced pressure, and the residue was purified by silica gel column chromatography (dichloromethane) to obtain intermediate **3-4** (0.76 g, yield 57.8%, brown solid). MS (ESI) m/z =302.9 $[M+H]^+$.

3.5 Synthesis of intermediate **3-5**

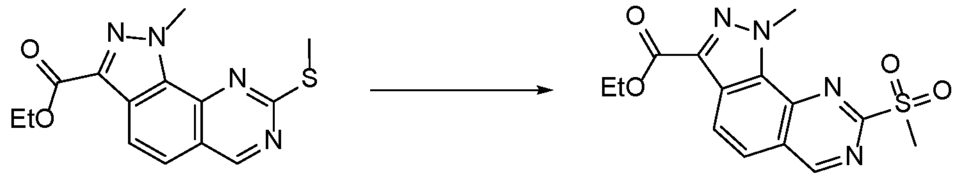

3-4 3-5

Intermediate **3-4** (0.86 g, 2.84 mmol) was dissolved in DMF (30 mL), potassium peroxymonosulfonate (5.25 g, 8.53 mmol) was added, and the mixture was reacted at room temperature for 16 h. Ice water was added to quench the reaction, the solid was precipitated, and filtered. The filter cake was dissolved in tetrahydrofuran (30 mL), and the solution was concentrated under reduced pressure. These operations were repeated three times. Intermediate **3-5** (0.80 g, crude product, white solid) was obtained, which was used directly in the next step. MS (ESI) m/z = 335.1 [M+H]+.

3.6 Synthesis of intermediate **3-6**

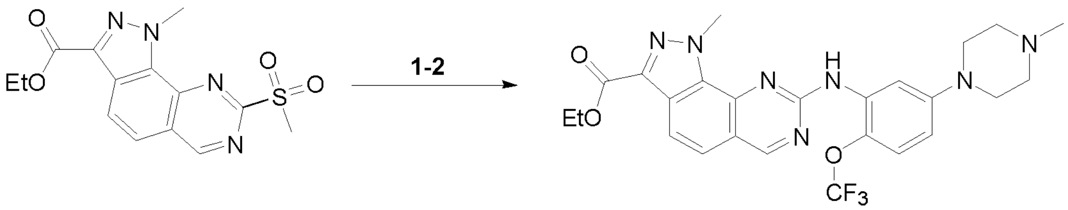


3-5 3-6

Intermediate **1-2** (0.284 g, 1.03 mmol) was dissolved in tetrahydrofuran (10 mL), LiHMDS (1 M, 1.55 mL) was added, and the mixture was reacted under stirring at -78°C for 0.5 h. Intermediate **3-5** (0.23 g, 0.688 mmol) was added and the mixture was reacted at room temperature for 12 h. Water (100 mL) was added to quench the reaction, dichloromethane (50 mL × 3) was added for extraction, the organic phases were combined, dried over anhydrous sodium sulfate, and filtered, the organic phase was concentrated under reduced pressure, and the residue was subjected to column chromatograph (C18 150*40 mm* 10 μm; mobile phase: [$H_2O(NH_3H_2O+NH_4HCO_3)$-ACN]; B%: 50%-90 %, 10 min) to obtain intermediate **3-6** (0.11 g, yield 20.5%, yellow solid). MS (ESI) m/z =530.3 [M+H]+.

3.7 Synthesis of compound **3**

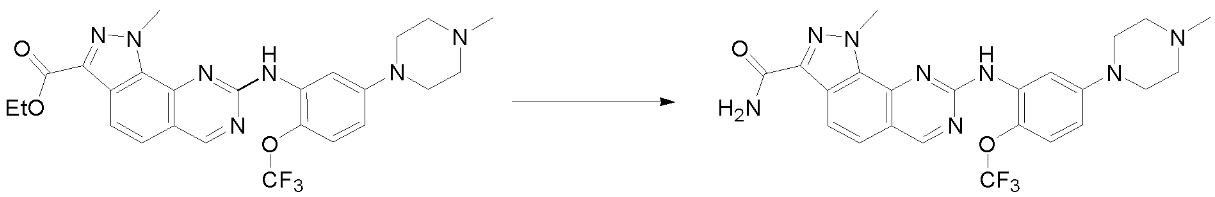

3-6 3

Compound **3-6** (40 mg, 75.5 umol) was dissolved in DMF (10 mL) and a solution of ammonia in methanol (75 mL) was added. The mixture was reacted for 48 h under sealed conditions at 135°C. The reaction liquid was concentrated under reduced pressure, and the residue was subjected to column chromatography (C18 75*30 mm*3 μm; mobile phase: [$H_2O(NH_4HCO_3)$-ACN]; B%: 30%-90%, 28 min). Compound **3** (3.9 mg, yield 10.4%, white solid) was obtained. MS (ESI) m/z =501.1 [M+H]+. $^1$H NMR (400 MHz, $CD_3OD$) δ 9.03 - 9.09 (m, 1 H). 7.98 - 8.08 (m, 2 H) 7.49 - 7.54 (m, 1 H) 7.19 - 7.27 (m, 1 H) 6.76 - 6.82 (m, 1 H) 4.71 - 4.75 (m, 3 H) 4.53 - 4.57 (m, 4 H) 4.30 (br, 1 H) 3.29 (br, 4 H) 2.59 - 2.70 (m, 4 H) 2.31 - 2.40 (m, 3 H).

**Example 4: Synthesis of compo und 4**

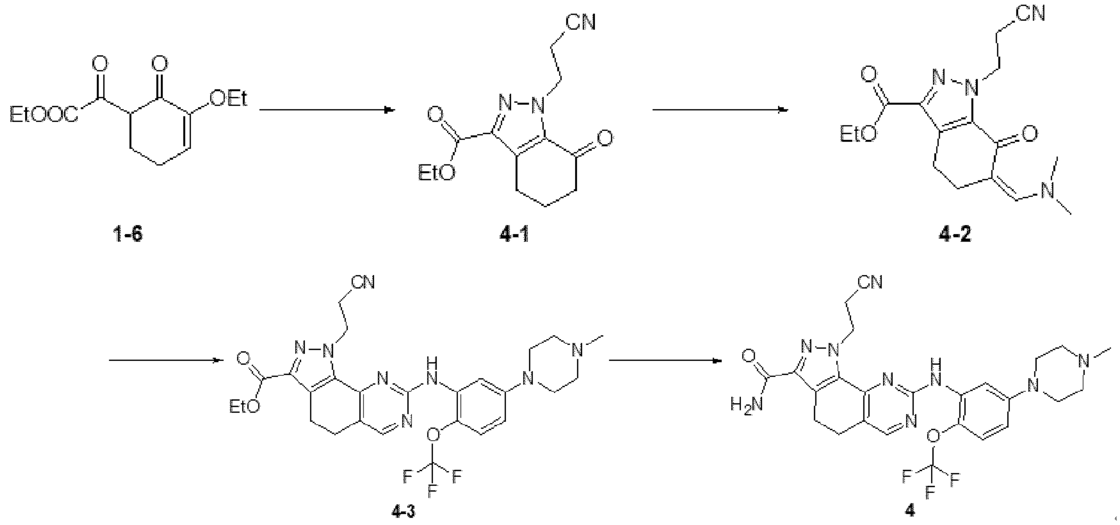

1-6 4-1 4-2 4-3 4

4.1 Synthesis of intermediate 4-1

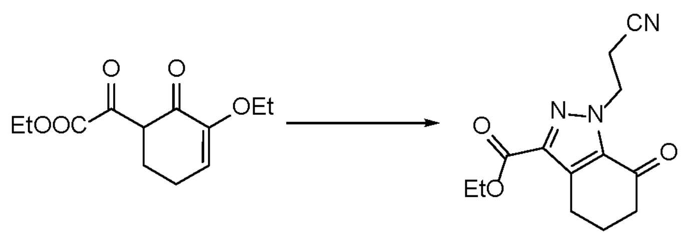

**1-6** **4-1**

Intermediate **1-6** (10.0 g, 41.6 mmol) was dissolved in acetic acid, 2-cyanoethylhydrazine (4.25 g, 50.0 mmol) was added, and the mixture was reacted at 25°C for 6 h. The reaction liquid was concentrated under reduced pressure, and the residue was purified by silica gel column chromatography (ethyl acetate:petroleum ether = 0-10%) to obtain intermediate **4-1** (5.4 g, yield 49.6%, white solid). MS (ESI) m/z =261.9 $[M+H]^+$.

4.2 Synthesis of intermediate **4-2**

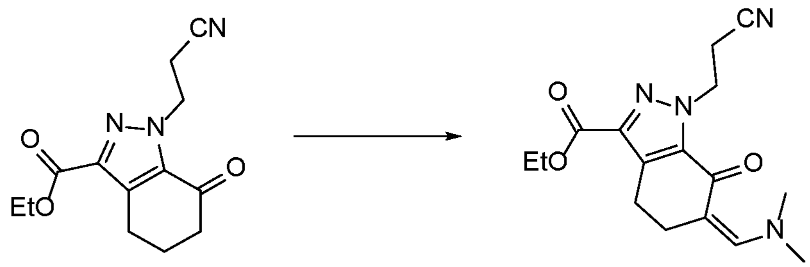

**4-1** **4-2**

Intermediate **4-1** (0.50 g, 1.91 mmol) was dissolved in DMF (10 mL), tert-butoxy bis(dimethylamino)methane (0.95 g, 3.83 mmol) was added, and the mixture was reacted at 110°C for 16 h. The reaction liquid was concentrated under reduced pressure, and the residue was dissolved in methyl tert-butyl ether (50 mL) and the mixture was stirred at 25°C for 0.5 h to precipitate a solid. After suction filtration, the filter cake was dried to obtain intermediate **4-2** (1.2 g, crude product), which was directly used in the next step.

4.3 Synthesis of intermediate **4-3**

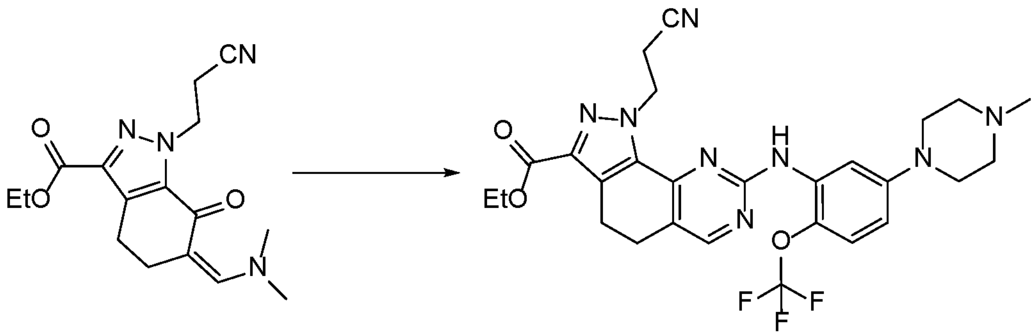

**4-2** **4-3**

Intermediate **4-2** (1.2 g, 1.91 mmol) was dissolved in DMF (10 mL), intermediate **1-3** (0.90 g, 2.84 mmol) was added, and the mixture was reacted at 110°C for 6 h. Water (200 mL) was added to quench the reaction, ethyl acetate (200 mL × 3) was added for extraction, the organic phases were combined, dried over anhydrous sodium sulfate, and filtered, the organic phase was concentrated under reduced pressure, and the residue was subjected to silica gel column chromatograph (methanol : dichloromethane = 0-10%). The intermediate **4-3** (0.47 g, yield 43.5%, brown solid) was obtained. MS (ESI) m/z = 571.3 $[M+H]^+$.

4.4 Synthesis of compound 4

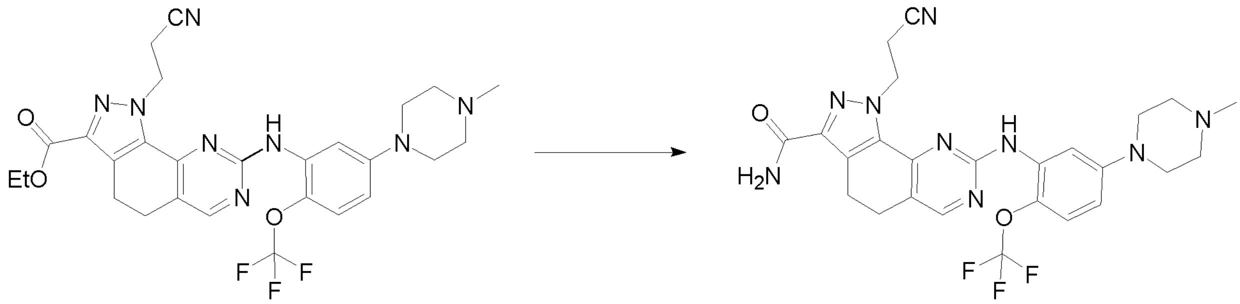

**4-3** **4**

Intermediate **4-3** (0.10 g, 0.175 mmol) was dissolved in methanol (5 mL), ammonia water (2 mL) was added, and the mixture was reacted under sealed conditions at 85°C for 48 h. The reaction liquid was concentrated under reduced pressure, and the residue was dissolved in ethyl acetate (50 mL) and the obtained product was washed with water (50 mL × 2). The organic phase was collected, dried over anhydrous sodium sulfate, filtered, and concentrated under reduced pressure. The residue was subjected to column chromatography (C18 75*30 mm*3 μm; mobile phase: [$H_2O$(0.05%$NH_3H_2O$ 10mM $NH_4HCO_3$)-ACN]; B%: 29%-59%, 11 min). Compound **4** (0.02 g, yield 20.6%, white solid) was obtained. MS (ESI) m/z = 542.3 $[M+H]^+$. $^1H$ NMR (400 MHz, DMSO-*d6*) δ 9.03 - 9.06 (m, 1 H). 9.00 - 9.07 (m, 1 H). 8.33 - 8.38 (m, 1 H). 7.43 - 7.50 (m, 1 H). 7.33 - 7.38 (m, 1 H). 7.18 - 7.28 (m, 2 H). 6.75 - 6.83 (m, 1 H). 4.72 - 4.81 (m, 2H). 3.11 - 3.18 (m, 4 H). 2.94 - 3.02 (m, 2 H). 2.91 (t, *J* = 6.40 Hz, 2 H) 2.76 - 2.83 (m, 2 H). 2.29 (br, 2 H). 2.25 - 2.39 (m, 1 H). 2.17 - 2.24 (m, 3 H).

**Example 5: Synthesis of compound 5**

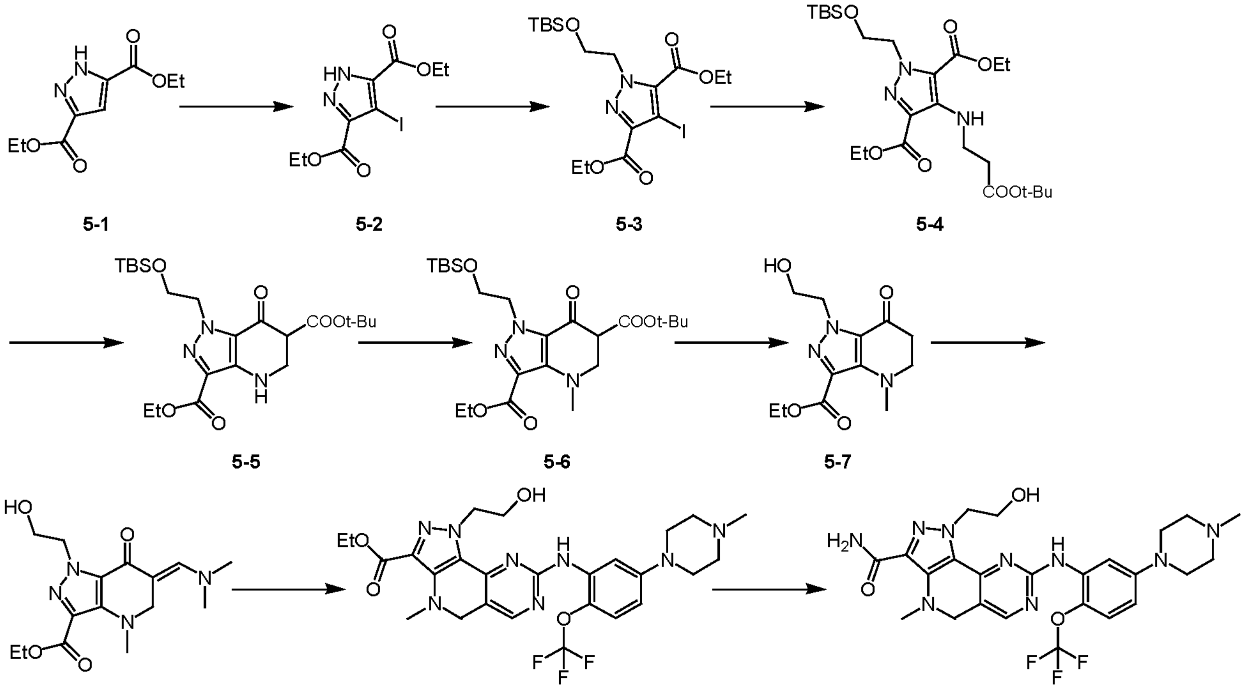

5-1 5-2 5-3 5-4

5-5 5-6 5-7

5-8 5-9 5

5.1 Synthesis of intermediate **5-2**

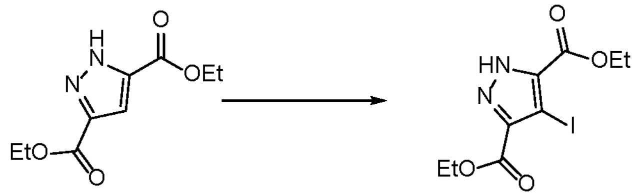

5-1 5-2

Intermediate **5-1** (30.0 g, 141 mmol) was dissolved in acetonitrile (500 mL), CAN (38.8 g, 70.7 mmol) and iodine (21.5 g, 84.8 mmol) were added, and the mixture was reacted at 80°C for 16 h. Sodium thiosulfate saturated solution (500 mL) was added to quench the reaction, and ethyl acetate (300 mL × 3) was added for extraction. The organic phases were combined, dried over anhydrous sodium sulfate, filtered, and the organic phase was concentrated under reduced pressure. The residue was subjected to silica gel column chromatography (ethyl acetate:petroleum ether = 0-30%) to obtain intermediate **5-2** (37.4 g, yield 78.3%, yellow solid). MS (ESI) m/z = 339.0 [M+H]$^+$.

5.2 Synthesis of intermediate **5-3**

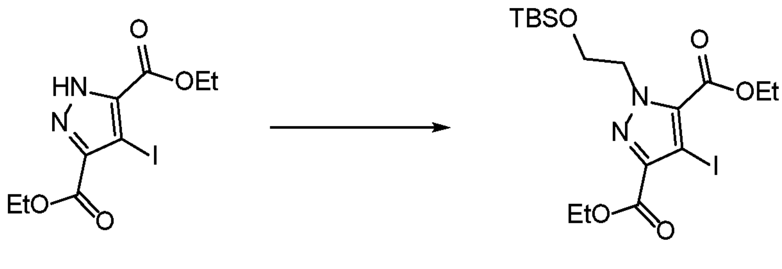

5-2 5-3

Intermediate **5-2** (40.0 g, 118 mmol) was dissolved in acetonitrile (400 mL), $K_2CO_3$ (24.5 g, 177 mmol ) and (2-bromoethoxy)-tert-butyldimethylsilane (34.0 g, 142 mmol) were added and the mixture was reacted at 80°C for 16 h. The reaction liquid was filtered, the filtrate was concentrated under reduced pressure, and the residue was subjected to silica gel column chromatography (ethyl acetate : petroleum ether = 0-20%). The intermediate **5-3** (54.4 g, yield 92.9%, white solid) was obtained. MS (ESI) m/z = 497.1 $[M+H]^+$.

5.3 Synthesis of intermediate **5-4**

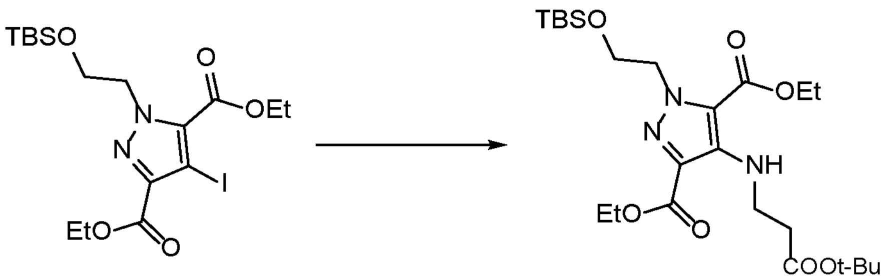

5-3 5-4

Intermediate **5-3** (50.0 g, 101 mmol), tert-butyl 3-aminopropionate hydrochloride (27.5 g, 151 mmol), cesium carbonate (131 g, 403 mmol) and 4,5-bisdiphenylphosphine-9,9-dimethylxanthene (2.91 g, 5.04 mmol) was dissolved in dioxane (500 mL), and $Pd_2(dba)_3$ (4.61 g, 5.04 mmol) was added, the mixture was reacted under nitrogen protection at 110°C for 16 h. Ammonium chloride saturated solution (500 mL) was added to quench the reaction, ethyl acetate (500 mL × 3) was added for extraction, the organic phases were combined, dried over anhydrous sodium sulfate, and filtered, and the organic phase was concentrated under reduced pressure. The residue was subjected to silica gel column chromatography (ethyl acetate : petroleum ether = 0-20%) to obtain intermediate **5-4** (38.9 g, yield 75.0%, oil). MS (ESI) m/z = 514.3 $[M+H]^+$.

5.4 Synthesis of intermediate **5-5**

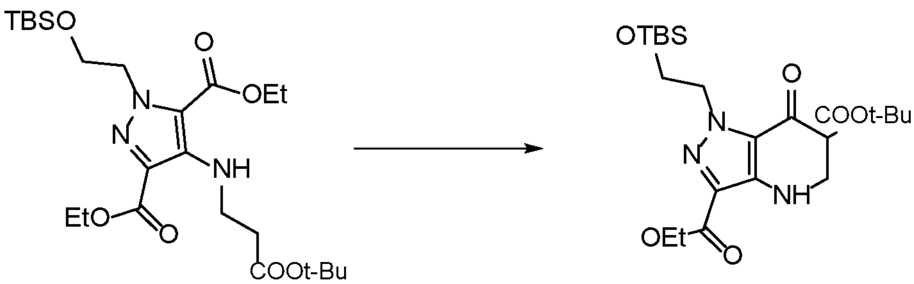

5-4 5-5

Intermediate **5-4** (24.0 g, 46.7 mmol) was dissolved in tetrahydrofuran (300 mL), potassium tert-butoxide (10.5 g, 93.4 mmol) was added, and the mixture was stirred at -20°C for 2 h. Ammonium chloride saturated solution (300 mL) was added to quench the reaction, ethyl acetate (300 mL × 3) was added for extraction, the organic phases were combined, dried over anhydrous sodium sulfate, and filtered, and the organic phase was concentrated under reduced pressure. The residue was subjected to silica gel column chromatography (ethyl acetate : petroleum ether = 0-30%) to obtain intermediate **5-5** (8.9 g, yield 40.6%, yellow solid). MS (ESI) m/z = 468.2 $[M+H]^+$.

5.5 Synthesis of intermediate **5-6**

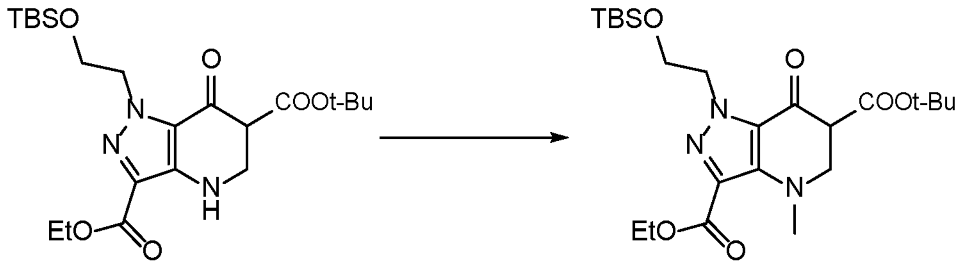

**5-5** **5-6**

Intermediate **5-5** (5.00 g, 10.7 mmol) was dissolved in acetic acid (50 mL), formaldehyde solution (2.60 g, 32.1 mmol) and $NaBH_3CN$ (1.34 g, 21.4 mmol) were added at 0°C. The mixture was reacted at room temperature for 2 h. $Na_2CO_3$ saturated solution (10 mL) was added to quench the reaction, ethyl acetate (10 mL × 2) was added for extraction, the organic phases were combined, dried over anhydrous sodium sulfate, and filtered, and the organic phase was concentrated under reduced pressure. The residue was subjected to silica gel column chromatography (ethyl acetate : petroleum ether = 30%) to obtain intermediate **5-6** (3.50 g, yield 68.0%, yellow solid). MS (ESI) m/z = 482.3 $[M+H]^+$.

5.6 Synthesis of intermediate **5-7**

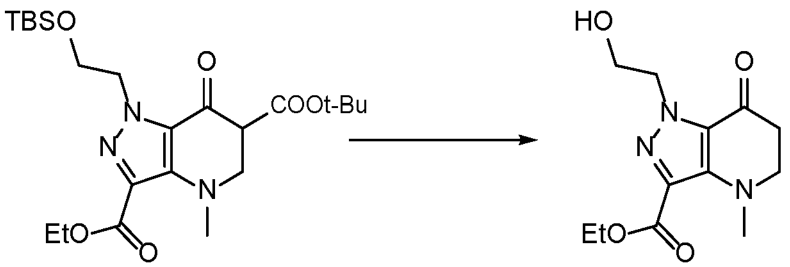

**5-6** **5-7**

Intermediate **5-6** (1.00 g, 2.08 mmol) was dissolved in ethanol (5 mL) and hydrochloric acid (3 M, 15.0 mL). The mixture was reacted at 60°C for 16 h. Sodium carbonate saturated solution (50 mL) was added to quench the reaction, ethyl acetate (50 mL × 3) was added for extraction, the organic phases were combined, dried over anhydrous sodium sulfate, and filtered, and the organic phase was concentrated under reduced pressure. The residue was subjected to silica gel column chromatography (ethyl acetate : petroleum ether = 0-100%) to obtain intermediate **5-7** (0.35 g, yield 63.1%, white solid). MS (ESI) m/z = 268.1 $[M+H]^+$.

5.7 Synthesis of intermediate **5-8**

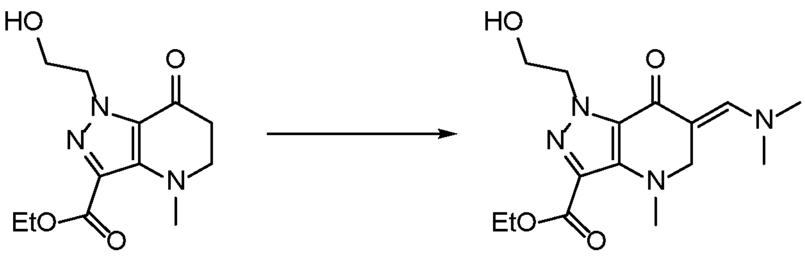

5-7 5-8

Intermediate **5-7** (0.30 g, 1.12 mmol) was dissolved in DMF (3 mL), tert-butoxy bis(dimethylamino)methane (0.456 g, 2.24 mmol) was added. The mixture was reacted at 60°C for 16 h. The reaction liquid was concentrated under reduced pressure to obtain intermediate **5-8** (0.25 g, crude product), which was directly used in the next step.

5.8 Synthesis of intermediate **5-9**

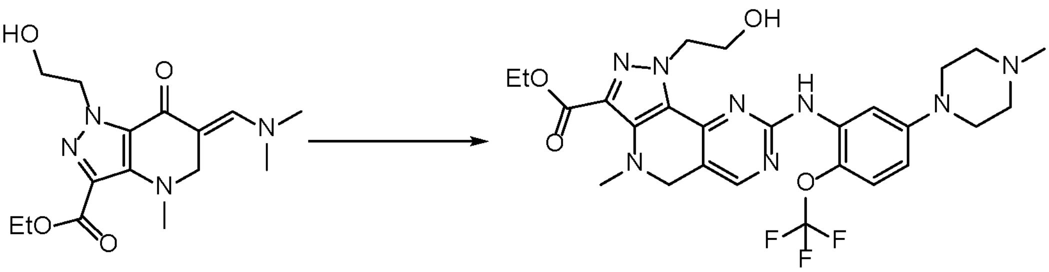

5-8 5-9

Intermediate **5-8** (0.10 g, 0.31 mmol), intermediate 1-3 (0.13 g, 0.31 mmol) and sodium ethoxide (0.025 g, 0.37 mmol) were dissolved in ethanol (10 mL). The mixture was reacted under microwave at 120°C for 1.5 h. The reaction liquid was concentrated under reduced pressure, and the residue was subjected to column chromatography (C18 150*40 mm* 10 μm; mobile phase: [$H_2O$ (10 mM $NH_4HCO_3$)-ACN]; B%: 20%-50%, 8 min), and the intermediate **5-9** (0.0034 g, yield 1.88%) was obtained. MS (ESI) m/z = 577.2 $[M+H]^+$.

5.9 Synthesis of compound **5**

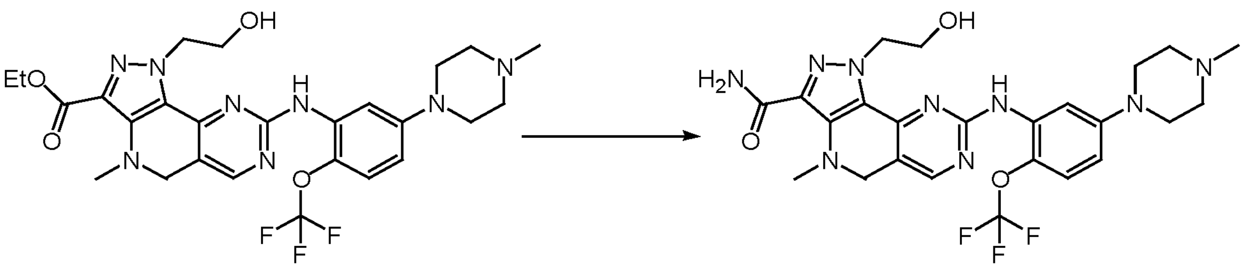

5-9 5

Intermediate **5-9** (5.00 mg, 8.67 μmol) was dissolved in tetrahydrofuran (1 mL), $NH_4Cl$ (1.45 mg, 27.1 μmol) was added, and LiHMDS (1 M, 54.2 μL) was added at 0°C. The mixture was reacted at room temperature for 16 h. Water (2 mL) was added to quench the reaction, ethyl acetate (5 mL * 3) was added for extraction, the organic phases were combined, dried over anhydrous sodium sulfate, and filtered, and the organic phase was concentrated under reduced pressure. The residue was subjected to column chromatography (C18 150*40 mm* 10 μm; mobile phase: [$H_2O$ (10 mM $NH_4HCO_3$)-ACN]; B%: 20%-50%, 8 min), and the compound **5** (1.61 mg, yield 32.4%) was obtained. MS (ESI) m/z = 548.1 $[M+H]^+$. $^1H$ NMR (400 MHz, DMSO-$d_6$) δ 8.88 (s, 1H), 8.24 (s, 1H), 7.42 (br s, 1H), 7.14 - 7.32 (m, 3H),

6.75 - 5.77 (m, 1H), 4.64 (br, 1H), 4.52 (br, 2H), 4.09 (s, 2H), 3.64 (br, 2H), 3.31 (br, 3H), 3.10 - 3.17 (m, 4H), 2.94 (s, 3H), 2.43 (br, 4H).

**Example 6: Synthesis of compound 6**

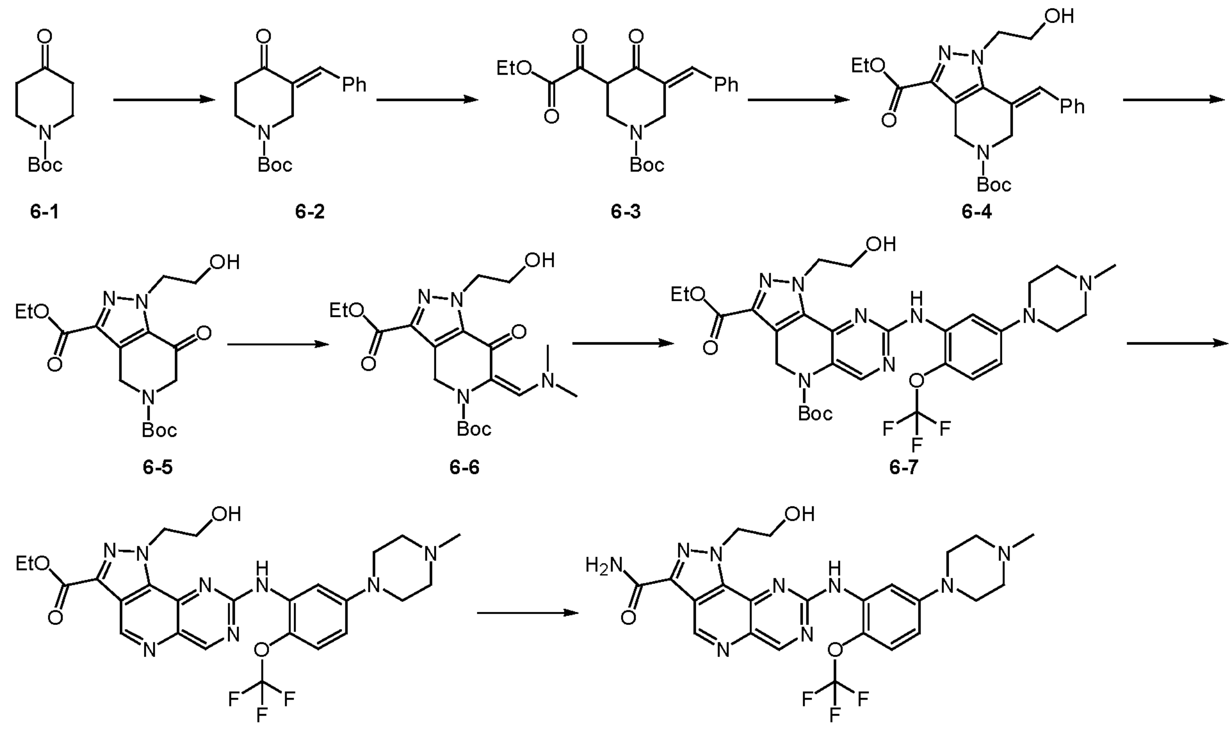

6-1 6-2 6-3 6-4

6-5 6-6 6-7

6-8 6

6.1 Synthesis of intermediate **6-2**

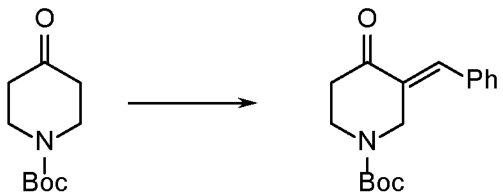

**6-1** **6-2**

Intermediate **6-1** (8.0 g, 40.1 mmol) was dissolved in toluene (100 mL), and morpholine (5.8 g, 66.4 mmol), p-toluenesulfonic acid (0.034 g, 0.2 mmol) and benzaldehyde (4.0 mL, 40.0 mmol) were added and the mixture was reacted at 110°C for 16 h. The reaction liquid was concentrated under reduced pressure, and the residue was subjected to silica gel column chromatography (ethyl acetate : petroleum ether = 25%) to obtain intermediate **6-2** (2.9 g, yield 25%, yellow solid). MS (ESI) m/z = 288.2 [M+H]$^+$.

6.2 Synthesis of intermediate **6-3**

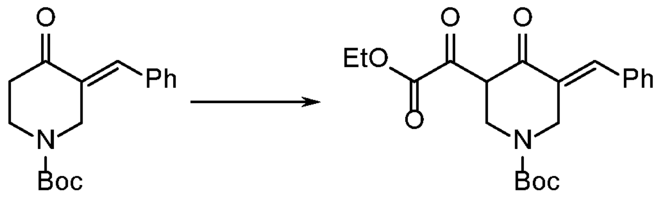

**6-2** **6-3**

Intermediate **6-2** (2.87 g, 10 mmol) was dissolved in tetrahydrofuran (50 mL), LiHMDS (1 M, 12 mL) was added dropwise at -50.0°C, and the mixture was stirred for 30 min. Diethyl oxalate (1.75 g, 12 mmol) was added and reacted at room temperature for 16 h. Saturated ammonium chloride (50 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-20%) to obtain intermediate **6-3** (2.8 g, yield 73.7%, oil). MS (ESI) m/z = 388.2 $[M+H]^+$.

6.3 Synthesis of intermediate **6-4**

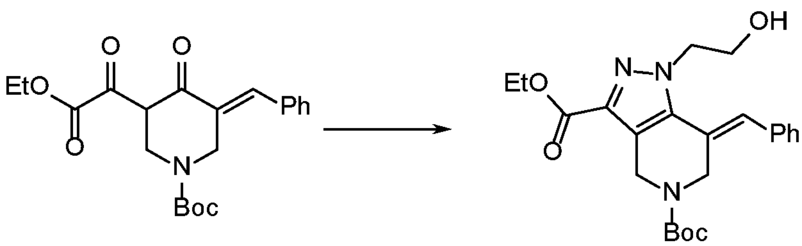

**6-3** **6-4**

Intermediate **6-3** (2.85 g, 7.36 mmol) was dissolved in acetic acid (10 mL), hydroxyethylhydrazine (0.57 g, 7.57 mmol) was added, and the mixture was reacted at room temperature for 15 h. water (50 mL) was added to quench the reaction, saturated $Na_2CO_3$ (60 mL) was added, and dichloromethane (50 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-50%) to obtain intermediate **6-4** (1.59 g, yield 50.7%, yellow solid). MS (ESI) m/z = 428.2 $[M+H]^+$.

6.4 Synthesis of intermediate **6-5**

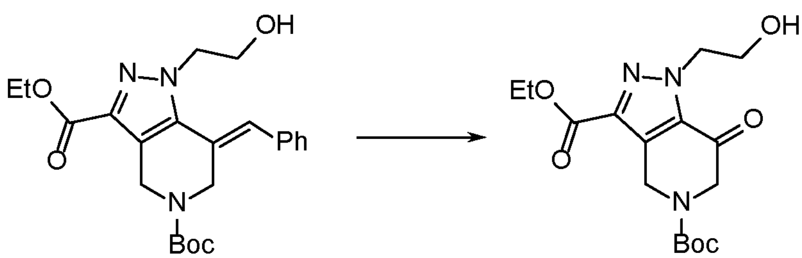

**6-4** **6-5**

Intermediate **6-4** (1.58 g, 3.70 mmol) was dissolved in tetrahydrofuran (16 mL) and water (16 mL), and $NaIO_4$ (1.58 g, 7.40 mmol) and $K_2OsO_4.2H_2O$ (0.11 g, 0.37 mmol) were added. The mixture was reacted at room temperature for 5 h. Water (50 mL) was added to quench the reaction, and ethyl acetate (50 mL × 3) was added for extraction. The organic phases were combined, dried over anhydrous sodium sulfate, filtered, and the organic phase was concentrated

under reduced pressure. The residue was subjected to silica gel column chromatography (ethyl acetate/petroleum ether = 0%-30%) to obtain intermediate **6-5** (0.313 g, yield 24%, yellow solid). MS (ESI) m/z = 354.2 $[M+H]^+$.

6.5 Synthesis of intermediate **6-6**

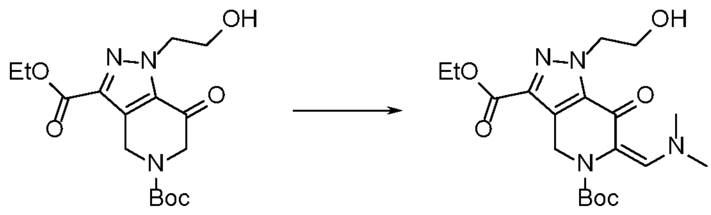

**6-5** **6-6**

Intermediate **6-5** (0.313 g, 0.89 mmol) was dissolved in DMF-DMA (10 mL) and the mixture was reacted at 110°C for 12 h. The reaction liquid was concentrated under reduced pressure, and the residue was dissolved in methyl tertiary ether and the mixture was stirred for 3 h. The resulting product was filtered, the filter cake was collected, and dried to obtain intermediate **6-6** (0.36 g, crude product), which was directly used in the next step.

6.6 Synthesis of intermediate **6-7**

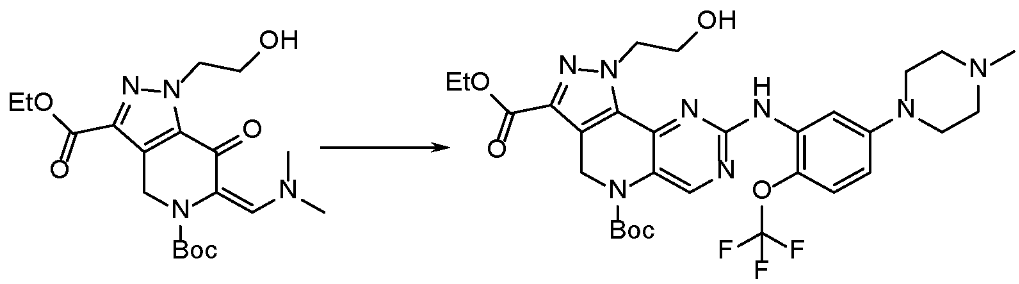

**6-6** **6-7**

Intermediate **6-6** (0.16 g, 0.40 mmol) and intermediate **1-3** (0.14 g, 0.44 mmol) were dissolved in DMF (5 mL) and the mixture was reacted at 110°C for 12 h. The reaction liquid was concentrated under reduced pressure, and the residue was subjected to silica gel column chromatography (ethyl acetate/petroleum ether = 0%-100%) to obtain intermediate **6-7** (0.14 g, yield 53.3%, yellow solid). MS (ESI) m/z = 663.2 $[M+H]^+$.

6.7 Synthesis of intermediate **6-8**

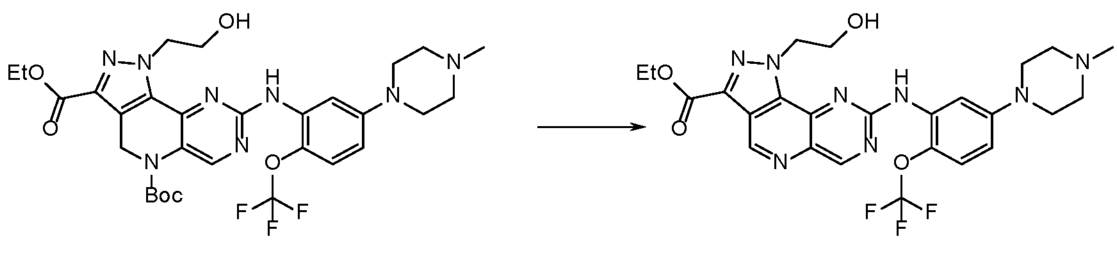

**6-7** **6-8**

Intermediate **6-7** (0.14 g, 0.21 mmol) was dissolved in dichloromethane (5 mL), trifluoroacetic acid (0.5 mL)

was added, and the mixture was reacted at room temperature for 4 h. The reaction liquid was concentrated under reduced pressure to obtain intermediate **6-8** (0.12 g, crude product), which was directly used in the next step.

6.8 Synthesis of compound **6**

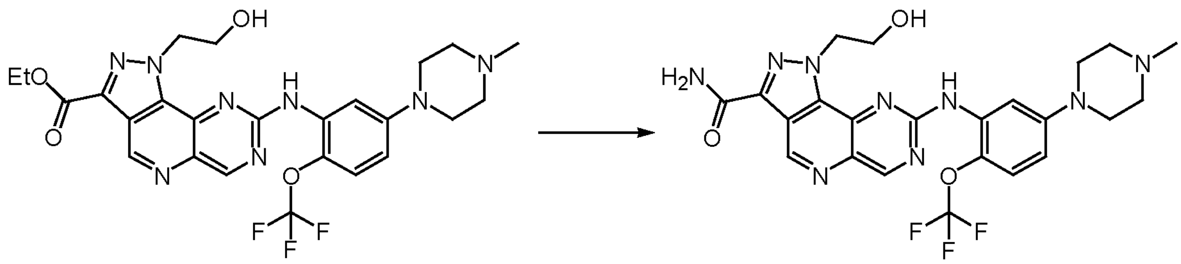

**6-8** **6**

Intermediate **6-8** (0.12 g, 0.21 mmol) was dissolved in NMP (6 mL), and $NH_3$/MeOH (5 mL) was added. The mixture was reacted for 48 h under sealed conditions at 80.0°C. The reaction liquid was concentrated under reduced pressure, and the residue was purified by preparative liquid chromatogram (C18 30* 150 mm*7 $\mu$m; mobile phase: [water (TFA)-acetonitrile]; B%: 10%-80%, 40 min). Compound **6** (0.045 g, yield 40%, yellow solid) was obtained. MS (ESI) m/z = 532.2 $[M+H]^+$. $^1$H NMR (400 MHz, DMSO-*d6*) $\delta$ 9.66 (s, 1H), 9.35 (d, *J*= 1.7 Hz, 2H), 7.97 (s, 1H), 7.68 (s, 1H), 7.27 - 7.30 (m, 2H), 6.87 - 6.90 (m, 1H), 4.88 - 4.90 (m, 2H), 4.65 - 4.67 (m, 1H), 3.77 (d, *J*= 5.5 Hz, 2H), 3.17 - 3.20 (m, 4H), 2.45 - 2.47 (m, 4H), 2.23 (s, 3H).

**Example 7: Synthesis of compound 7**

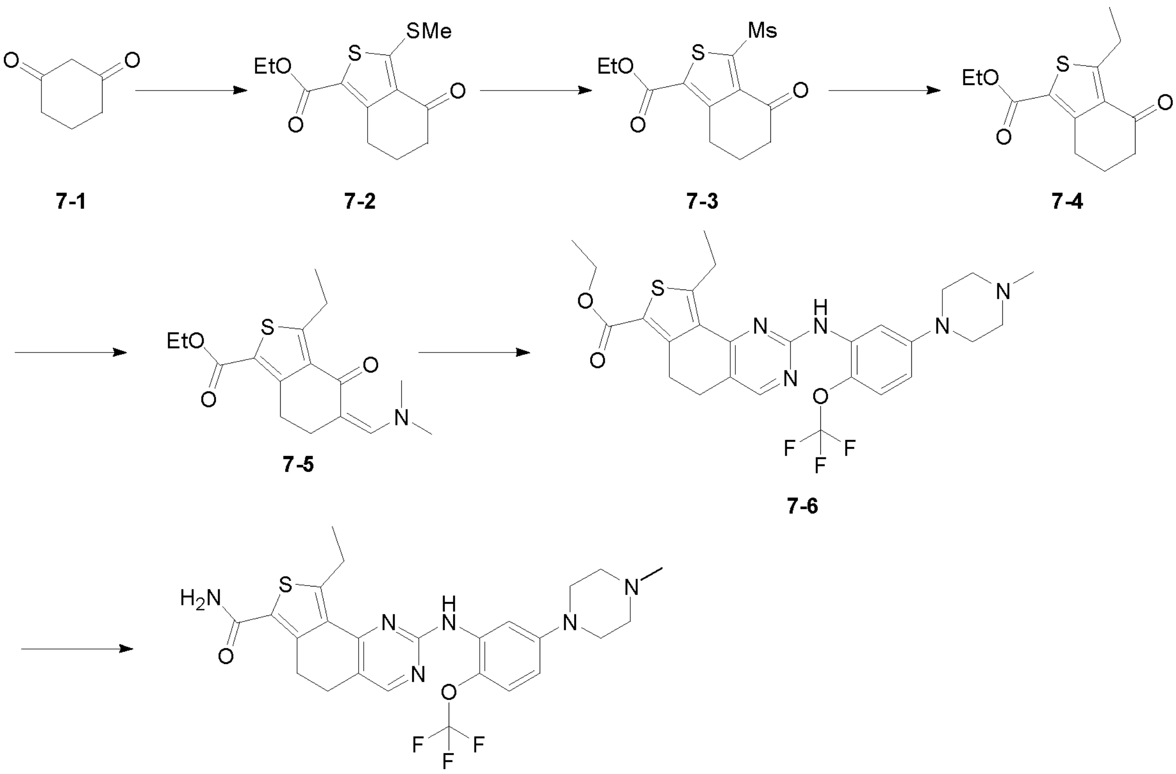

**7-1** **7-2** **7-3** **7-4**

**7-5**

**7-6**

**7**

7.1 Synthesis of intermediate **7-2**

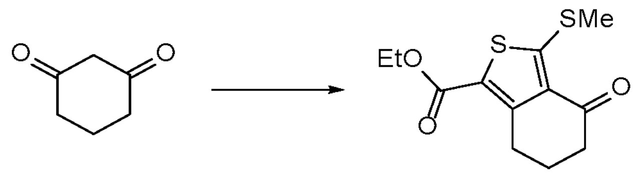

7-1 7-2

$K_2CO_3$ (206 g, 1.50 mol) was dissolved in DMF (450 mL), intermediate **7-1** (55.9 g, 0.50 mol) was added, and the mixture was reacted under stirring at room temperature for 10 min. CS2 (56.9 g, 0.75 mol) was added and the mixture was reacted under stirring at room temperature for 10 min. Then, a solution of ethyl bromoacetate (83.3 g, 0.50 mol) in DMF (500 mL) was added dropwise at 0°C, and the reaction was carried out at 0°C for 1 h. Finally, a solution of iodomethane (77.8 g, 0.55 mol) in DMF (200 mL) was added dropwise at 0°C, and the reaction was carried out at 0°C for 0.5 h. The reaction liquid was filtered, the filtrate was poured into water (5 L), and ethyl acetate (1 L × 5) was added for extraction. The organic phases were combined, dried over anhydrous sodium sulfate, filtered, and the organic phase was concentrated under reduced pressure. Intermediate **7-2** (720 g, crude product, oil) was obtained and directly used in the next step.

7.2 Synthesis of intermediate **7-3**

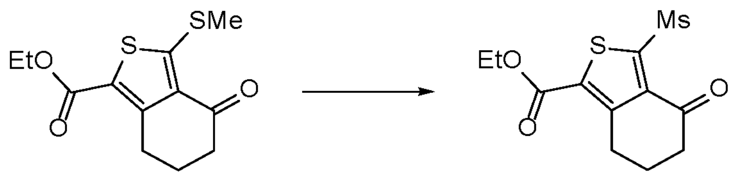

7-2 7-3

Intermediate **7-2** (53.5 g, 197 mmol) was dissolved in ethanol (650 mL) and water (650 mL), potassium monopersulfate (401 g, 2.39 mol) was added, and the mixture was reacted at 60°C 16 h. Water (1.1 L) was added, and the mixture was stirred at 50-60°C for 0.5 h and cooled to 15°C, a solid was precipitated, filtered, and the filter cake was dried. The filter cake was subjected to silica gel column chromatography (ethyl acetate:petroleum ether = 0-40%) to obtain the intermediate **7-3** (6.5 g, yield 11%, orange solid). MS (ESI) m/z = 303.0 $[M+H]^+$.

7.3 Synthesis of intermediate **7-4**

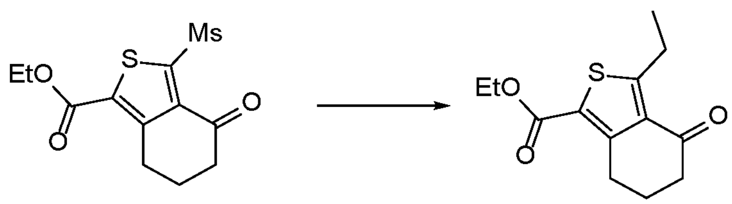

7-3 7-4

Intermediate **7-3** (9.00 g, 29.8 mmol) was dissolved in tetrahydrofuran (90 mL), and ethylmagnesium bromide (1.00 M, 32.7 mL) was added at 0°C. The mixture was reacted at 35°C for 16 h. Saturated ammonium chloride (100 mL) was added to quench the reaction, and ethyl acetate (100 mL × 3) was added for extraction. The organic phases

were combined, dried over anhydrous sodium sulfate, filtered, and the organic phase was concentrated under reduced pressure. The residue was subjected to silica gel column chromatography (ethyl acetate/petroleum ether = 0%-10%) to obtain intermediate **7-4** (1.47 g, yield 19.5%, white solid). MS (ESI) m/z = 253.1 $[M+H]^+$.

7.4 Synthesis of intermediate **7-5**

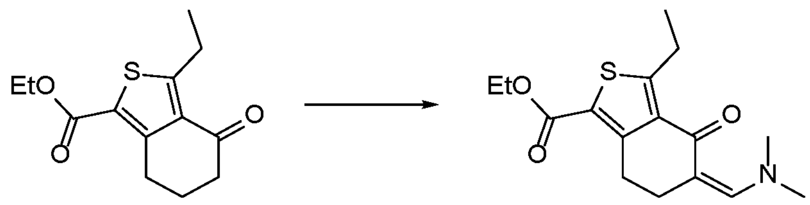

**7-4** **7-5**

Intermediate **7-4** (0.50 g, 1.98 mmol) was dissolved in DMF-DMA (4.49 g, 37.6 mmol, 5.00 mL) and the mixture was reacted at 100°C for 16 h. The reaction liquid was concentrated under reduced pressure to obtain intermediate **7-5** (0.75 g, crude product), which was directly used in the next step.

7.5 Synthesis of intermediate **7-6**

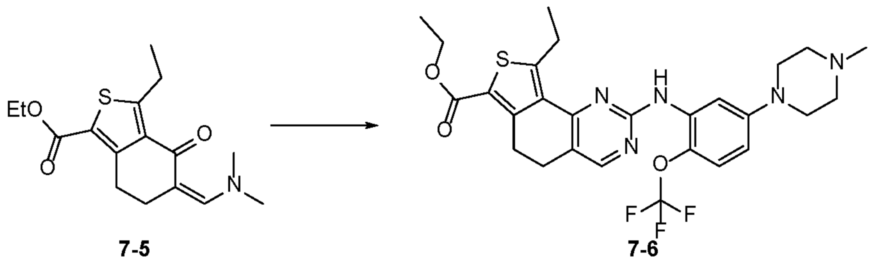

**7-5** **7-6**

Intermediate **7-5** (0.37 g, 1.20 mmol) and intermediate 1-3 (0.34 g, 1.08 mmol) were dissolved in DMF (5 mL) and the mixture was reacted at 110°C for 4 h. Water (80 mL) was added to quench the reaction, and ethyl acetate (80 mL × 4) was added for extraction. The organic phases were combined, dried over anhydrous sodium sulfate, filtered, and the organic phase was concentrated under reduced pressure. The residue was subjected to silica gel column chromatography (methanol: dichloromethane = 0%-7%) to obtain intermediate **7-6** (0.23 g, yield 34.8%, brown solid). MS (ESI) m/z = 562.2 $[M+H]^+$.

7.6 Synthesis of compound **7**

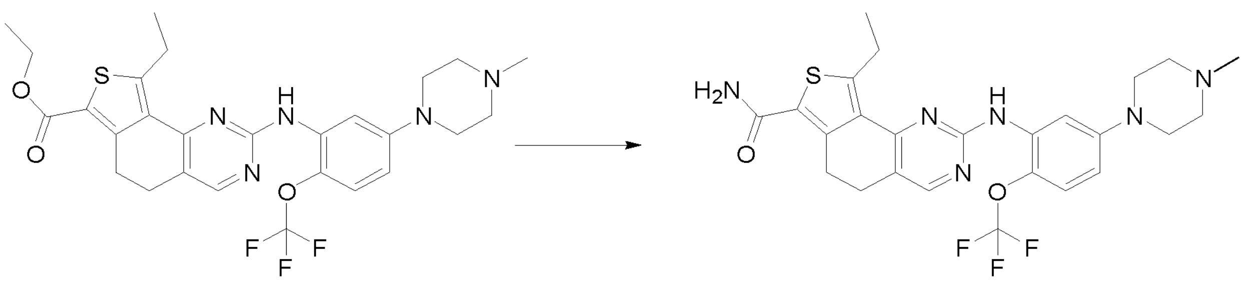

**7-6** **7**

Intermediate **7-6** (100 mg, 178 μmol) was dissolved in NMP (2 mL), and $NH_3$/MeOH (4.0 mL) was added. The mixture was reacted at 15 Psi and 100.0°C for 32 h. The reaction liquid was concentrated under reduced pressure, and the residue was purified by preparative liquid chromatogram (C18 75*30 mm*3 μm; mobile phase: [$H_2O$ ($NH_3H_2O$+$NH_4HCO_3$)-ACN]; B%: 40.0%-58.0%, 14.0 min). Compound **7** (11.6 mg, yield 95.0%, white solid) was obtained. MS (ESI) m/z = 533.2 $[M+H]^+$. $^1H$ NMR(400 MHz, DMSO-*d6*)δ 8.64 (s, 1H), 8.32 (s, 1H), 7.39 (br, 2H), 7.26 - 7.13 (m, 2H), 6.83 - 6.74 (m, 1H), 3.20 - 3.03 (m, 8H), 2.70 (br, 2H), 2.48 - 2.38 (m, 5H), 2.21 (s, 3H), 1.04 (t, *J* = 7.3 Hz, 3H).

**Example 8: Synthesis of compound 8**

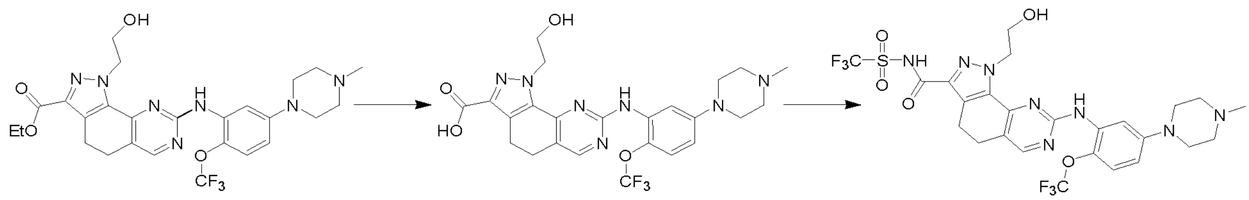

1-9 8-1 8

8.1 Synthesis of intermediate **8-1**

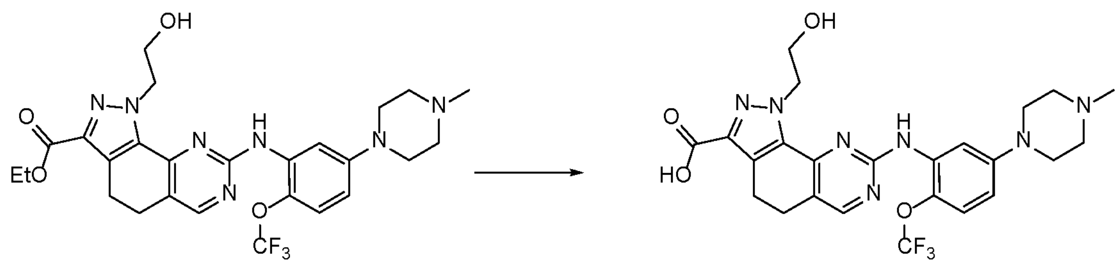

1-9 8-1

Compound **1-9** (550 mg, 0.766 mmol) was dissolved in ethanol (5 mL), and LiOH.$H_2O$ (2 M, 1.53 mL) was added dropwise. The mixture was reacted at room temperature for 5 h. Water (10 mL) was added to quench the reaction, ethyl acetate (20 mL) was added for extraction, the aqueous phase was collected and adjusted to pH 3-4 to precipitate a solid. The resulting product was filtered, the filter cake was dried to obtain intermediate **8-1** (290 mg, crude product), which was directly used in the next step. MS (ESI) m/z = 534.3 $[M+H]^+$.

8.2 Synthesis of compound **8**

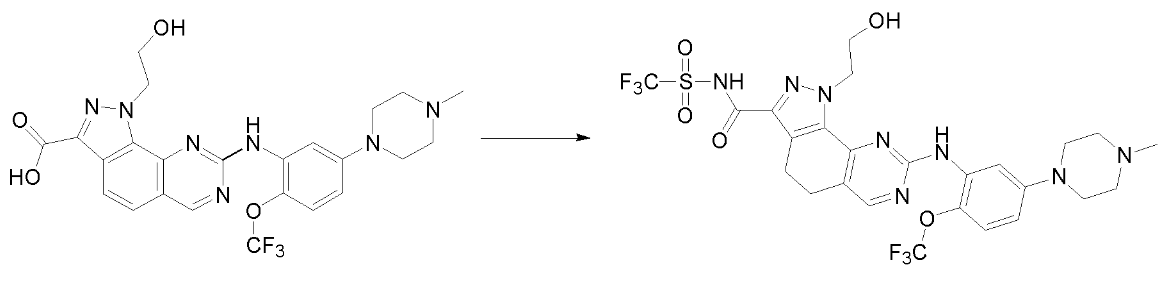

8-1 8

Intermediate **8-1** (53.1 mg, 0.10 mmol) was dissolved in dichloromethane (5 mL), trifluoromethanesulfonamide

(89.4 mg, 0.60 mmol), EDCI (76.8 mg, 0.40 mmol) and DMAP (48.8 mg, 0.40 mmol) were added. The mixture was stirred at room temperature for 16 h. The mixture was diluted with dichloromethane (10 mL), water (**20.0** mL***3**) was added for extraction, the organic phase was dried over anhydrous sodium sulfate, and filtered, and the organic phase was concentrated under reduced pressure. The residue was subjected to column chromatography (**C18 150** * **30 mm** * **5 μm;** mobile phase: [$H_2O$ ($NH_4HCO_3$)-ACN], B%: 10%-60%, 12 min), compound **8** (21.7 mg, yield 32.6%, white solid) was obtained. MS (ESI) m/z = 665.3 $[M+H]^+$. $^1H$ NMR (400 MHz DMSO-$d_6$) δ 8.82 (s, 1H), 8.29 (s, 1H), 7.23 - 7.21 (m, 2H), 6.80 - 6.78 (m, 1H),4.60 (m, 3H), 3.57 (s, 2H), 3.19 (s, 4H), 2.98 - 2.94 (m, 2H), 2.78 - 2.74 (m, 2H), 2.67 - 2.65 (m, 4H), 2.36 - 2.33 (m, 3H).

**Example 9: Synthesis of compound 9**

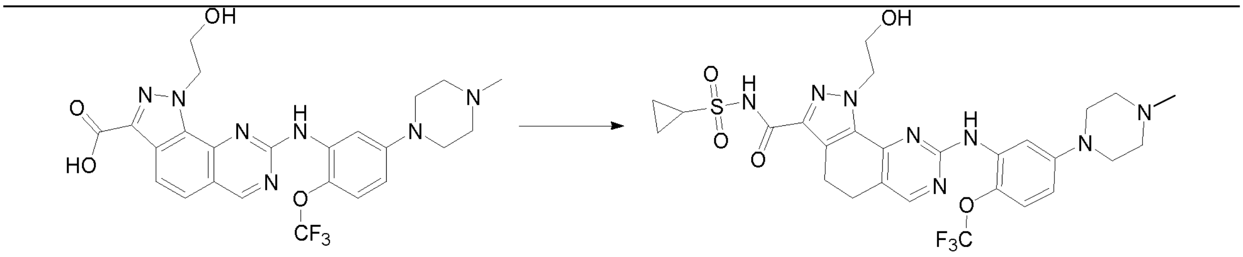

8-1 9

A method similar to Example 8 was used to prepare compound **9** (13 mg, yield 38.5%, white solid) from intermediate **8-1** and cyclopropanesulfonamide. MS (ESI) m/z = 637.2 $[M+H]^+$. $^1H$ NMR (400 MHz DMSO-$d_6$) δ 10.99 (br, 1H), 8.99 (s, 1H), 8.36 (s, 1H), 7.28 - 7.25 (m, 2H), 6.85 - 6.82 (m, 1H), 4.66 (m, 3H), 3.68 - 3.66 (m, 2H), 3.33 - 3.29 (m, 4H), 3.07 - 3.03 (m, 1H), 3.00 - 2.92 (m, 6H), 2.84 - 2.80 (m, 2H), 2.55 - 2.50 (m, 3H), 1.10 - 1.00 (m, 4H).

**Example 10: Synthesis of compound 10**

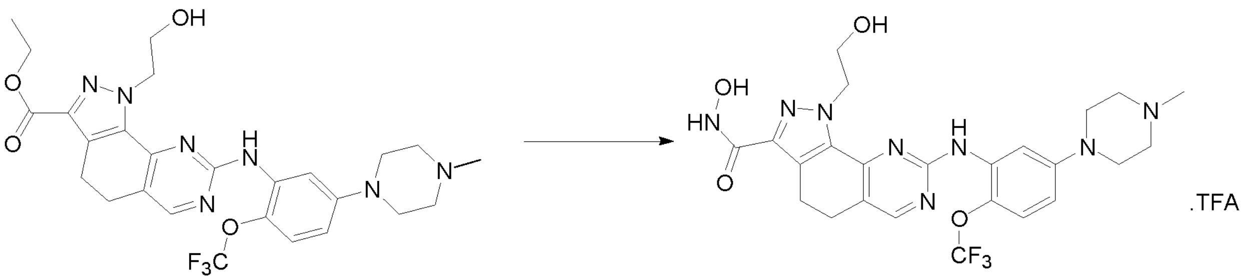


1-9 10

Na (40.9 mg, 1.78 mmol) was dissolved in methanol (1.8 mL), $HONH_2$.HCl (37.1 mg, 0.534 mmol) was added, and the mixture was stirred at room temperature for 1 h under nitrogen protection. Intermediate **1-9** (100 mg, 0.178 mmol) was added and the mixture was reacted at 65°C for 0.5 h. The reaction liquid was filtered, and the filtrate was concentrated under reduced pressure. The residue was subjected to column chromatography (C18 150*40 mm* 10 μm; mobile phase: [$H_2O$(TFA)-ACN]; B%: 10.0%-50.0%, 10 min), the compound **10** (12.0 mg, 11.3% yield, trifluoroacetate, white solid) was obtained. MS (ESI) m/z = 549.1 $[M+H]^+$. $^1H$ NMR (400 MHz DMSO-$d_6$) δ 10.89 (s, 1H), 9.86 - 9.65 (m, 1H), 9.00 (s, 1H), 8.34 (s, 1H), 7.35 - 7.26 (m, 2H), 6.85 - 6.90 (m, 1H), 4.62 (br, 2H), 3.84 (br, 2H), 3.64 (br, 3H), 3.20 - 3.10 (m, 2H), 2.94 (br, 4H), 2.86 (s, 3H), 2.83 - 2.77 (m, 2H), 2.55 (t, *J* = 5.5 Hz, 2H).

**Example 11: Synthesis of compound 11**

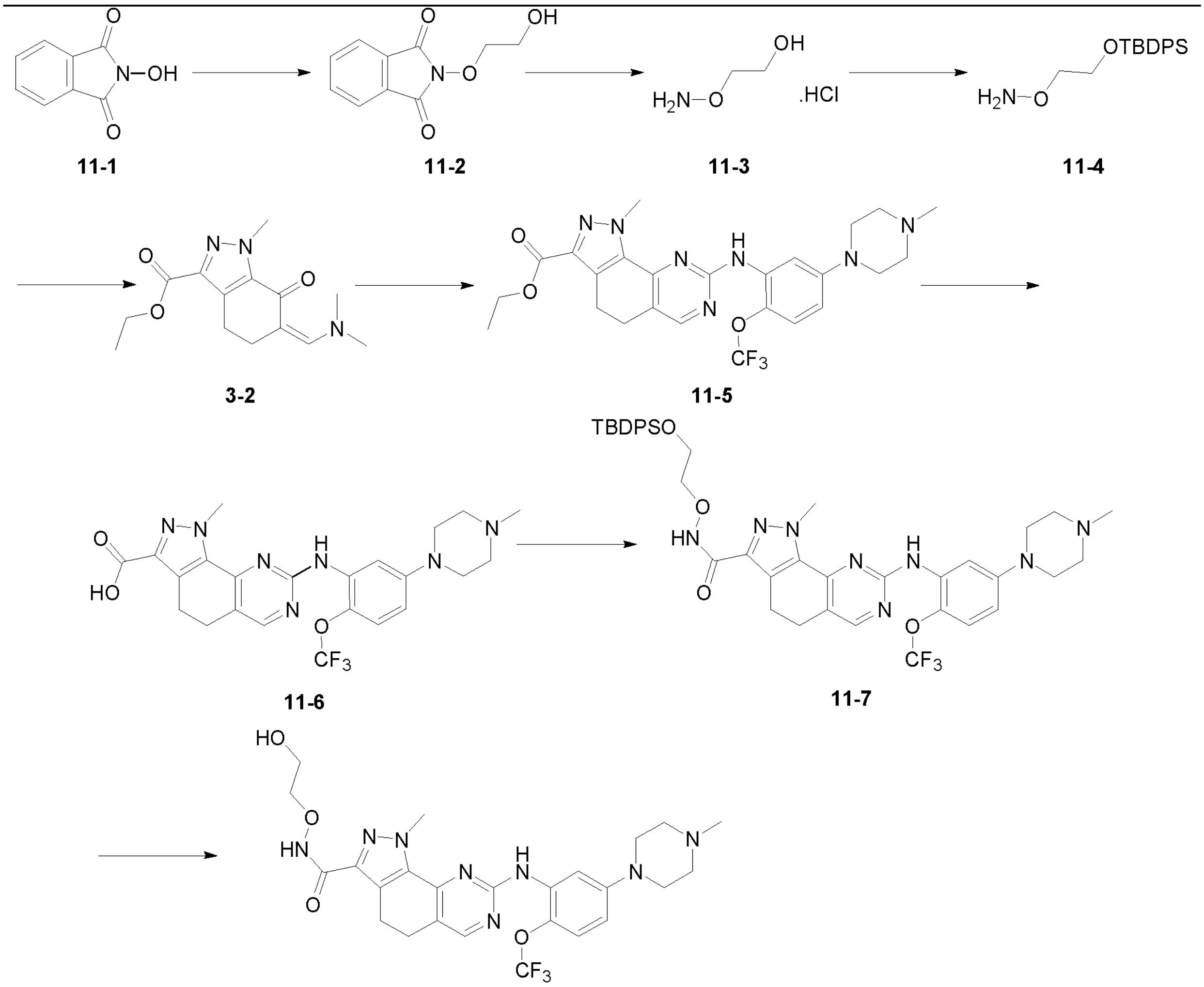

11-1 11-2 11-3 11-4

3-2 11-5

11-6 11-7

11

11.1 Synthesis of intermediate **11-2**

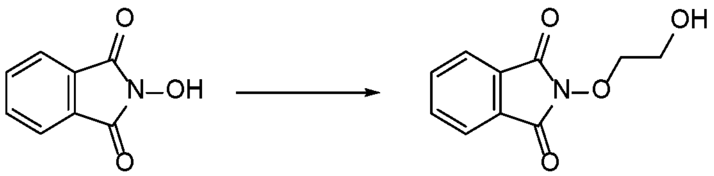

11-1 11-2

Intermediate **11-1** (10.0 g, 61.3 mmol) was dissolved in acetonitrile (20 mL), triethylamine (7.43 g, 73.4 mmol) and 2-bromoethanol (9.18 g, 73.4 mmol) were added under ice bath conditions. The mixture was reacted at 80°C for 8 h. Water (200 mL) was added to quench the reaction, and ethyl acetate (200 mL × 3) was added for extraction. The organic phases were combined, dried over anhydrous sodium sulfate, filtered, and the organic phase was concentrated under reduced pressure. The intermediate **11-2** (4.5 g, crude product) was obtained and used directly in the next step. MS (ESI) m/z =207.9 [M+H]$^+$.

11.2 Synthesis of intermediate **11-3**

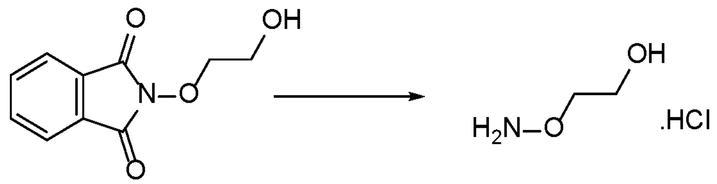

11-2 11-3

Intermediate **11-2** (2.00 g, 9.65 mmol) was dissolved in dichloromethane (40 mL), $N_2H_4 \cdot H_2O$ (1.02 g, 19.3 mmol, 987 μL) was added. The mixture was reacted at room temperature for 12 h. HCl/dioxane (2 mL, 4 M) was added and the mixture was stirred at room temperature to precipitate a solid. The resulting product was filtered, the filter cake was dried to obtain intermediate **11-3** (0.69 g, crude product), which was directly used in the next step.

11.3 Synthesis of intermediate **11-4**

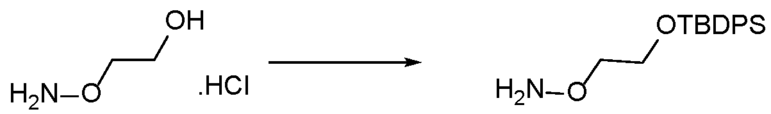

11-3 11-4

Intermediate **11-3** (2.32 g, 8.43 mmol) was dissolved in dichloromethane (40 mL), DMAP (79.2 mg, 0.648 mmol), imidazole (1.10 g, 16.2 mmol) and tert-butyl diphenylchlorosilane (1.78 g, 6.48 mmol) were added. The mixture was reacted at room temperature for 16 h. The reaction liquid was concentrated under reduced pressure, and the residue was subjected to silica gel column chromatography (ethyl acetate : petroleum ether = 0-20%) to obtain intermediate **11-4** (0.23 g, yield 11.3%, oil). MS (ESI) m/z =316.0 $[M+H]^+$.

11.4 Synthesis of intermediate **11-5**

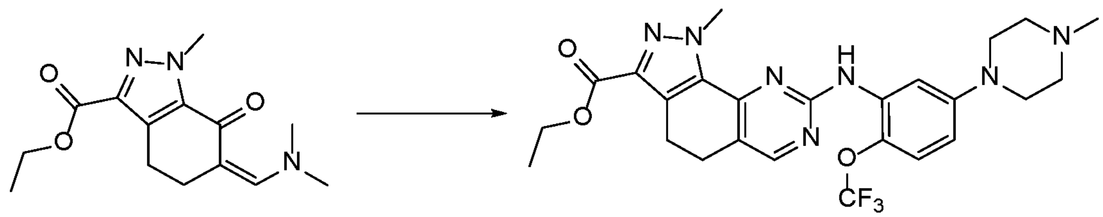

3-2 11-5

Intermediate **3-2** (800 mg, 2.88 mmol) was dissolved in DMF (20 mL), intermediate 1-3 (915 mg, 2.88 mmol) was added, and the mixture was reacted at 110°C for 16 h. Water (50 mL) was added to quench the reaction, ethyl acetate (50 mL × 3) was added for extraction, the organic phases were combined, dried over anhydrous sodium sulfate, and filtered, the organic phase was concentrated under reduced pressure, and the residue was subjected to silica gel column chromatograph (methanol : dichloromethane = 0-10%). The intermediate **11-5** (738 mg, yield 48.1%, light yellow solid) was obtained. MS (ESI) m/z =532.2 $[M+H]^+$.

11.5 Synthesis of intermediate **11-6**

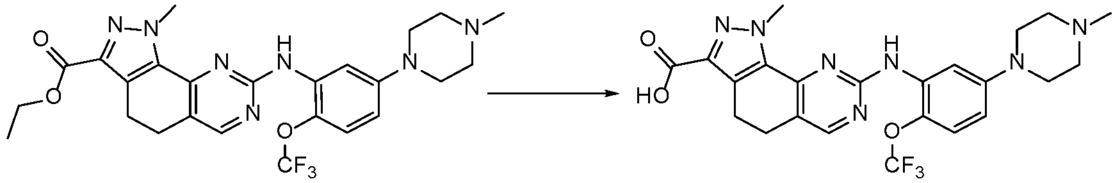

11-5 11-6

Compound **11-5** (660 mg, 1.24 mmol) was dissolved in methanol (5 mL) and tetrahydrofuran (5 mL), and a solution of $LiOH.H_2O$ (140 mg, 3.35 mmol) in water (5 mL) was added. The mixture was reacted at room temperature for 16 h. Water (10 mL) was added to quench the reaction, ethyl acetate (20 mL) was added for extraction, the aqueous phase was collected, and adjusted to pH 3-4 with dilute hydrochloric acid to precipitate a solid. The resulting product was filtered, the filter cake was dried to obtain intermediate **11-6** (531 mg, crude product), which was directly used in the next step. MS (ESI) m/z =504.1 $[M+H]^+$.

11.6 Synthesis of intermediate **11-7**

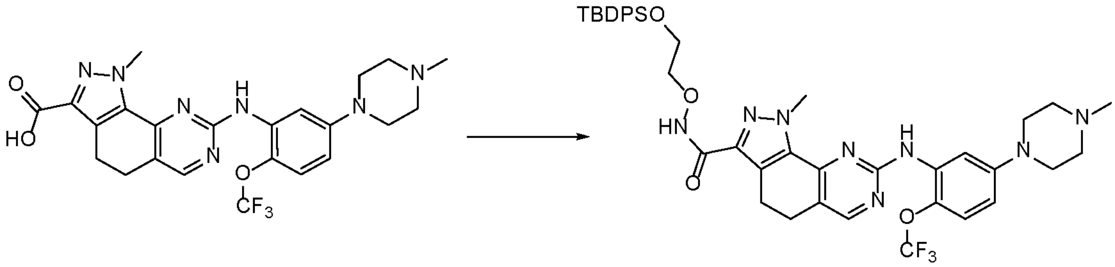

11-6 11-7

Intermediate **11-6** (125 mg, 397 μmol) was dissolved in DMF (10 mL), CDI (32.2 mg, 199 μmol) was added, and the mixture was stirred at 50°C for 2 h. Intermediate **11-4** (50 mg, 99.3 μmol) was added and the mixture was reacted at 50°C for 46 h. The reaction liquid was poured into water (100 mL), and a solid was precipitated. The obtained product was filtered, the filter cake was collected, and silica gel column chromatography (ethyl acetate:petroleum ether = 30%) was performed. The intermediate 11-7 (2.77 g, yield 55.5%, yellow solid) was obtained. MS (ESI) m/z = 801.3 $[M+H]^+$.

11.7 Synthesis of compound **11**

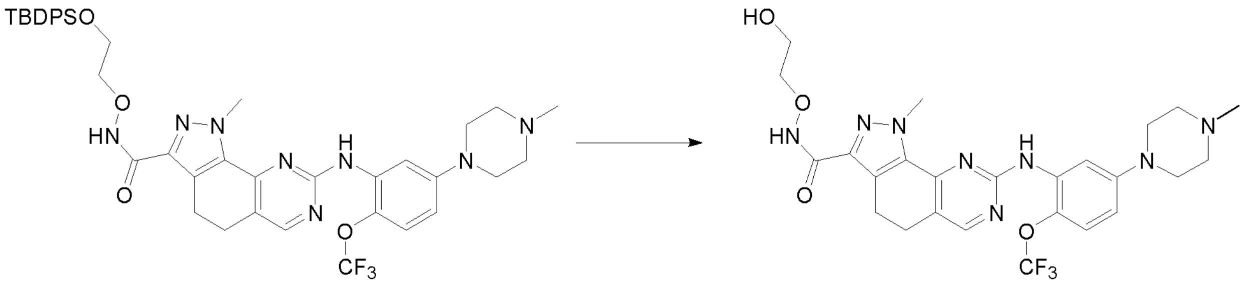

11-7 11

Intermediate **11-7** (100 mg, 125 μmol) was dissolved in dichloromethane (10 mL), $Et_3N{\cdot}3HF$ (20.1 mg, 125 μmol) was added. The mixture was reacted at room temperature for 12 h. The reaction liquid was concentrated under reduced pressure, and the residue was subjected to column chromatography (C18 30*150 mm*7 μm; mobile phase: [$H_2O$ ($NH_4HCO_3$) - MeOH]; B%: 30% - 80%, 40 min) to obtain compound **11** (33.0 mg, yield 46.0%, white solid). MS

(ESI) m/z =563.2 [M+H]+. $^1$H NMR (400 MHz, DMSO-*d6*) δ 8.90 - 8.98 (m, 1H), 8.34 - 8.37 (m, 1H), 7.25 - 7.32 (m, 1H), 7.14 - 7.23 (m, 1H), 6.76 (dd, *J* = 9.2, 2.8 Hz, 1H), 4.16 (s, 3H), 3.86 (s, 2H), 3.57 (s, 3H), 3.08 - 3.22 (m, 4H), 2.90 - 2.99 (m, 2H), 2.76 - 2.86 (m, 3H), 2.42 - 2.47 (m, 4H), 2.21 (s, 3H).

**Example 12: Synthesis of compound 12**

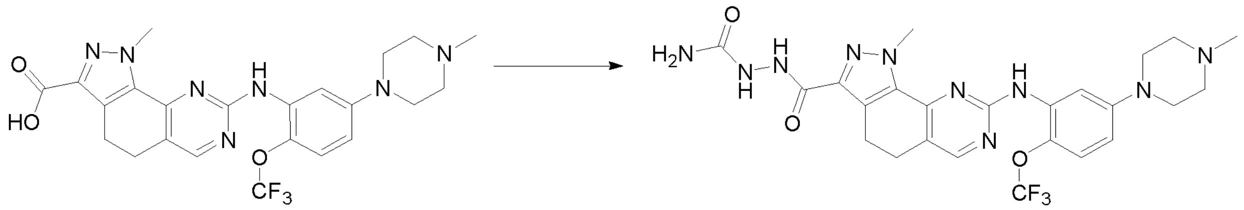

**11-6** **12**

A method similar to Example 11 was used to prepare compound **12** from the reaction of intermediate **11-6** and semicarbazide. MS (ESI) m/z = 561.2 [M+H]+. $^1$H NMR (400 MHz, $CD_3OD$) δ 8.30 (s, 1H), 7.67 (s, 1H), 7.24 (d, *J* = 8.8 Hz, 1H), 6.78 - 6.81 (m, 1H), 4.28 (s, 3H), 3.37 - 3.35 (m, 4H), 3.13 - 3.05 (m, 6H), 2.88 (t, *J* = 7.6 Hz, 2H), 2.68 (s, 3H).

**Example 13: Synthesis of compound 13**

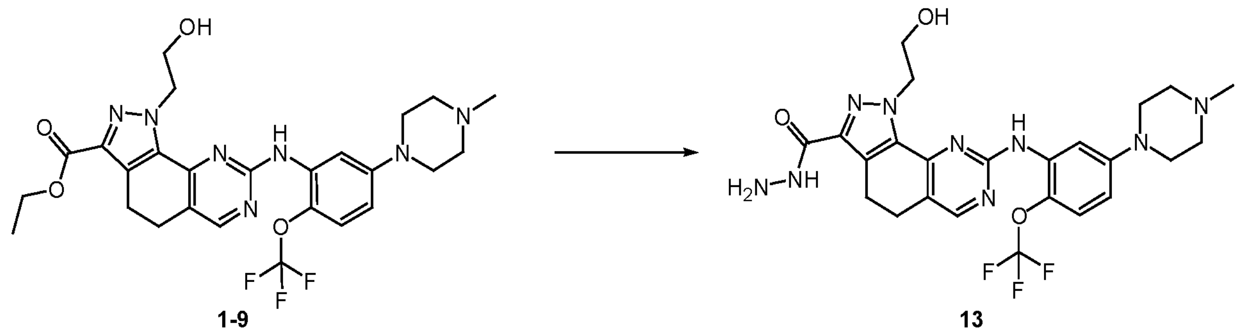

**1-9** **13**

Intermediate **1-9** (20 mg, 0.036 mmol) was dissolved in methanol (1 mL), and $N_2H_4$-$H_2O$ (54.6 mg, 1.07 mmol, 53 μL) was added. The mixture was reacted at 70.0°C for 36 h. The reaction liquid was concentrated under reduced pressure, and the residue was purified by preparative liquid chromatogram (C18 75*30 mm*3 μm; mobile phase: [$H_2O$ ($NH_3H_2O$+$NH_4HCO_3$)-ACN]; B%: 21%-41%, 10 min). Compound **13** (6.0 mg, yield 30.5%, white solid) was obtained. MS (ESI) m/z = 548.3 [M+H]+. $^1$H NMR (400 MHz, $CD_3OD$) δ 8.28 - 8.32 (m, 1 H), 7.46 - 7.54 (m, 1 H), 7.20 - 7.24 (m, 1 H), 6.80 - 6.84 (m, 1 H), 4.77 (t, *J* = 5.6 Hz, 3 H), 4.56 - 4.62 (m, 2 H), 4.51 - 4.63 (m, 3 H), 3.75 - 3.85 (m, 2 H), 3.21 - 3.27 (m, 4 H), 2.98 - 3.11 (m, 2 H), 2.85 - 2.92 (m, 2 H), 2.59 - 2.66 (m, 4 H), 2.34 - 2.39 (m, 3 H).

**Example 14: Synthesis of compound 17**

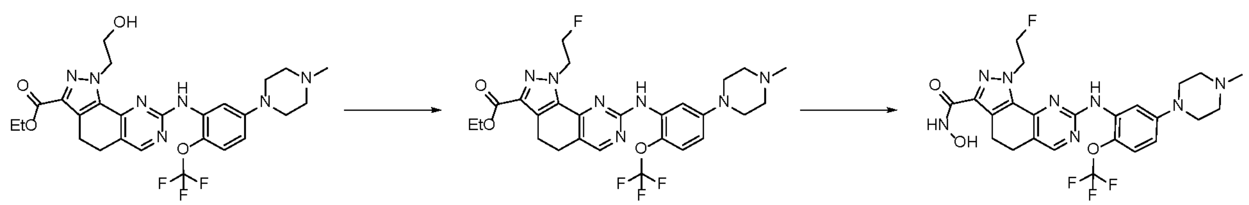

1-9 17-1 17

14.1 Synthesis of intermediate **17-1**

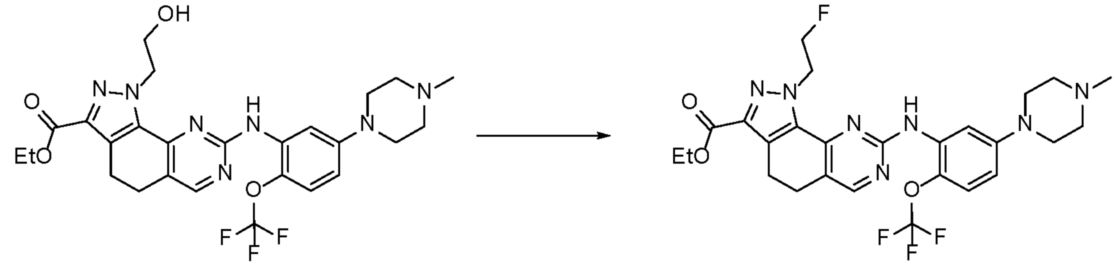

1-9 17-1

Intermediate **1-9** (200 mg, 0.36 mmol) was dissolved in dichloromethane (20 mL) and DAST (293 mg, 1.82 mmol) was added dropwise at -78°C. The mixture was reacted at room temperature for 12 h. The reaction liquid was cooled to room temperature, water (70 mL) was added to quench the reaction, and dichloromethane (50 mL × 3) was added for extraction. The organic phases were combined, dried over anhydrous sodium sulfate, filtered, and the organic phase was concentrated under reduced pressure. The residue was purified by preparative liquid chromatogram (C18 30* 150 mm*5 μm; mobile phase: [water ($NH_4HCO_3$)-acetonitrile]; B%: 45%- 82%, 12 min) to obtain intermediate**17-1** (170 mg, yield 84.8%, brown solid). MS (ESI) m/z = 564.2 $[M+H]^+$.

14.2 Synthesis of compound **17**

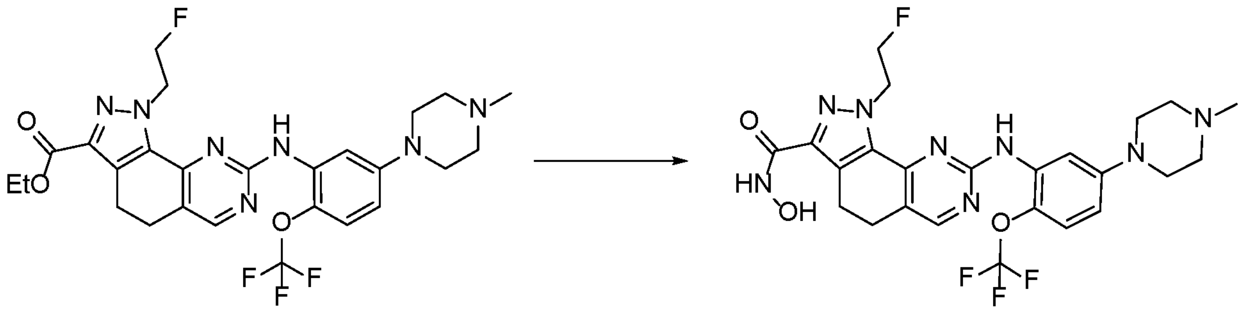

17-1 17

Na (40.9 mg, 1.78 mmol) was dissolved in methanol (1.8 mL), $HONH_2$.HCl (37.1 mg, 0.534 mmol) was added, and the mixture was stirred at room temperature for 1 h under nitrogen protection. Intermediate **17-1** (100 mg, 0.178 mmol) was added and the mixture was reacted at 65°C for 0.5 h. The reaction liquid was filtered, and the filtrate was concentrated under reduced pressure. The residue was subjected to column chromatography (C18 150*40 mm* 10 μm; mobile phase: [$H_2O$(TFA)-ACN]; B%: 10.0%-50.0%, 10 min), the compound **17** (11.1 mg, 11.3% yield, trifluoroacetate, white solid) was obtained. MS (ESI) m/z = 551.2 $[M+H]^+$. $^1H$ NMR (400 MHz DMSO-$d_6$) δ 10.90 (s, 1 H), 9.52 - 9.82 (m, 1 H), 9.10 (s, 1 H), 8.36 (s, 1 H), 7.23 - 7.36 (m, 2 H), 6.88 (dd, *J* = 9.01, 2.75 Hz, 1 H), 4.78 - 4.94 (m, 2 H), 4.65 (t, *J* = 4.63 Hz, 1 H), 4.53 (t, *J* = 4.44 Hz, 1 H), 3.84 (d, *J* = 13.01 Hz, 2 H), 3.07 - 3.26 (m, 3 H), 2.93 - 3.01 (m, 4 H), 2.78 - 2.92 (m, 6H).

**Example 15: Synthesis of compound 18**

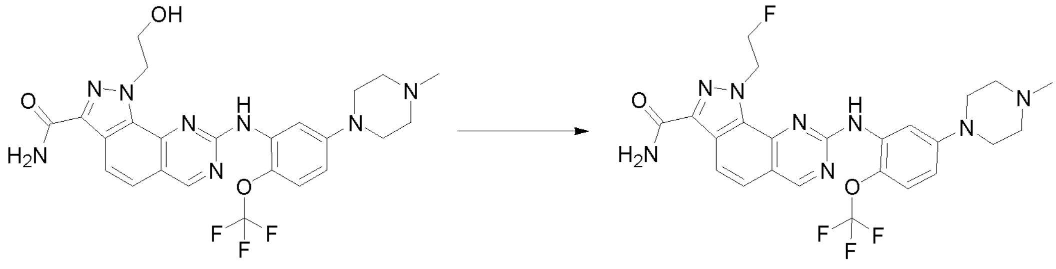

1 18

Compound **1** (50 mg, 94.2 μmol) was dissolved in dichloromethane (5 mL), and DAST (77.5 mg, 481 μmol) was added dropwise at -78°C. The mixture was reacted at room temperature for 16 h. The reaction liquid was cooled to room temperature, water (20 mL) was added to quench the reaction, and dichloromethane (10 mL × 3) was added for extraction. The organic phases were combined, dried over anhydrous sodium sulfate, filtered, and the organic phase was concentrated under reduced pressure. The residue was purified by preparative liquid chromatogram (C18 30*150 mm*5 μm; mobile phase: [water ($NH_4HCO_3$)-acetonitrile]; B%: 27%- 73%, 12 min) to obtain compound **18** (5 mg, yield 10%, brown solid). MS (ESI) m/z = 533.2 $[M+H]^+$. $^1H$ NMR: (400 MHz MeOD) δ 9.21 (s, 1H), 8.07 (d, *J* = 8.0 Hz, 1H), 7.57 - 7.49 (m, 2H), 7.32 - 7.30 (d, *J* = 8.0 Hz, 1H), 6.93 - 6.91 (m, 1H), 5.30 - 5.21 (m, 2H), 4.80 - 4.66 (m, 2H), 3.37 (m, 4H), 2.96 (m, 4H), 2.60 (s, 3H).

**Example 16: Synthesis of compound 19**

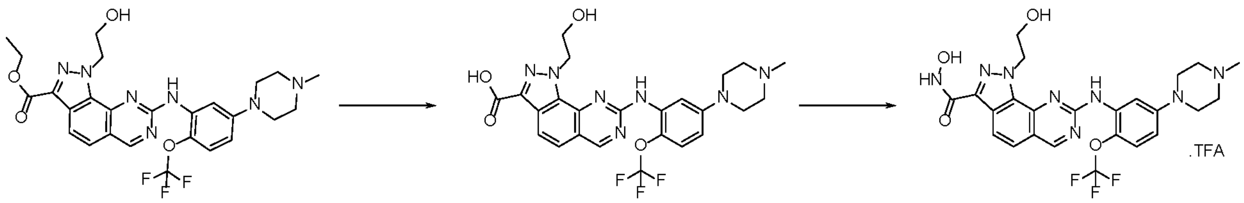

1-10 19-1 19

16.1 Synthesis of intermediate 19-1

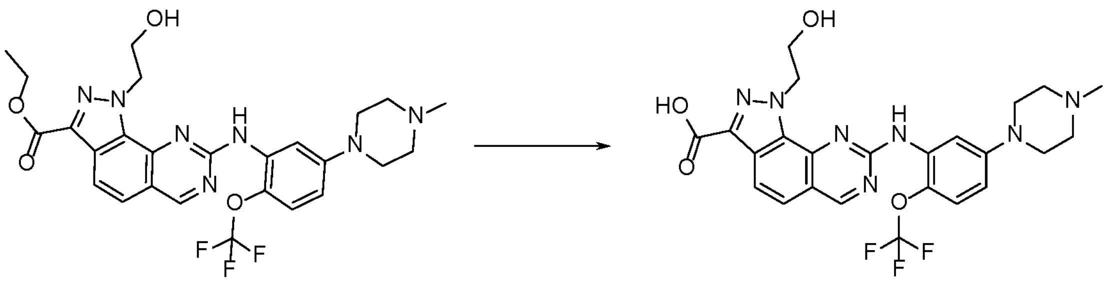

1-10 19-1

Intermediate **1-10** (100 mg, 0.178 mmol) was dissolved in methanol (2 mL) and lithium hydroxide aqueous solution (1 M, 890 μL) was added. The mixture was reacted at room temperature for 6 h. The pH was adjusted to 5-7 with hydrochloric acid at 0°C, the residue was purified by preparative liquid chromatogram (C18 150*30 mm*5 μm; mobile phase: [water (TFA)-acetonitrile]; B%: 10%- 50%, 10 min) to obtain intermediate **19-1** (37 mg, yield 39%, brown solid). MS (ESI) m/z = 532.3 $[M+H]^+$.

16.2 Synthesis of compound **19**

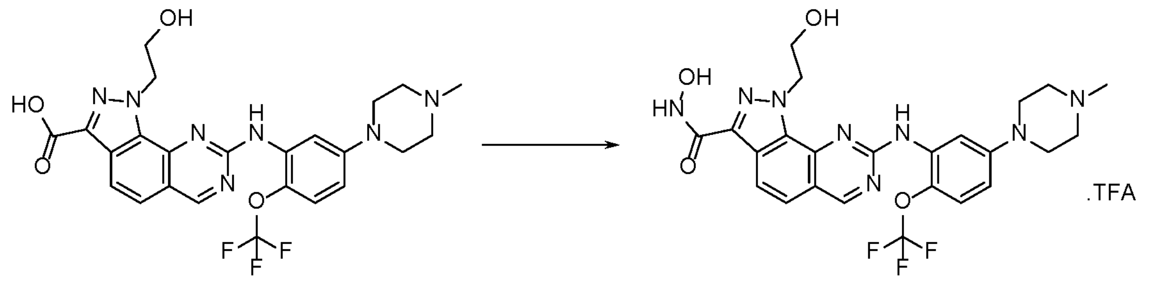

19-1 19

Intermediate **19-1** (37.0 mg, 69.6 μmol), HATU (29.1 mg, 76.6 μmol) and DIEA (36.0 mg, 278 μmol) were dissolved in DMF (2 mL), and hydroxylamine hydrochloride (9.68 mg, 139 μmol) was added. The mixture was reacted at room temperature for 2 h. The reaction liquid was concentrated under reduced pressure, and the residue was purified by preparative liquid chromatogram (C18 150*30 mm*5 μm; mobile phase: [water (TFA)-acetonitrile]; B%: 5%-45%, 10 min) to obtain the compound **19** (17 mg, 37% yield, brown solid, trifluoroacetate salt). MS (ESI) m/z = 547.2 [M+H]$^+$. $^1$H NMR (DMSO-$d_6$) δ 10.89 (s, 1H), 9.86 - 9.65 (m, 1H), 9.00 (s, 1H), 8.34 (s, 1H), 7.35 - 7.26 (m, 2H), 6.86 (dd, *J* = 2.9, 9.0 Hz, 1H), 4.62 (br t, *J* = 5.3 Hz, 2H), 3.84 (br d, *J* = 12.5 Hz, 2H), 3.64 (br t, *J* = 5.4 Hz, 3H), 3.20 - 3.10 (m, 2H), 2.94 (br t, *J* = 7.8 Hz, 4H), 2.86 (s, 3H), 2.83 - 2.77 (m, 2H), 2.55 (t, *J* = 5.5 Hz, 2H).

**Example 17: Synthesis of compound 20**

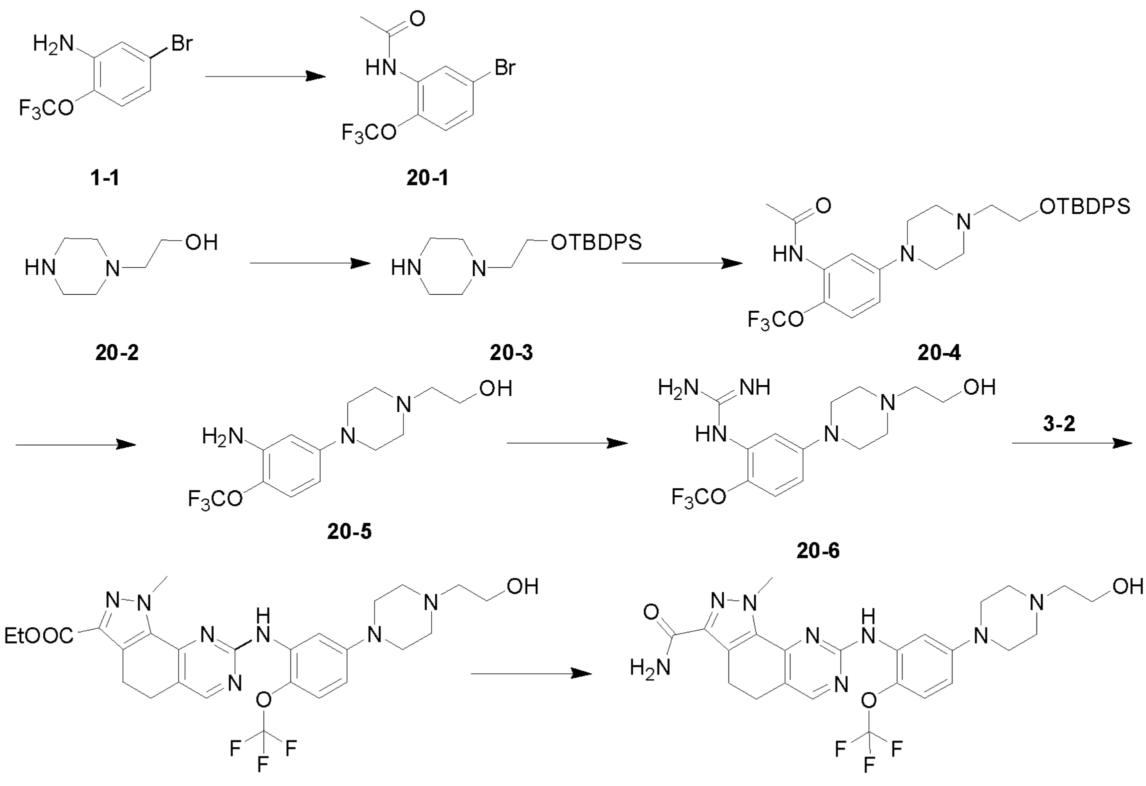


1-1 20-1

20-2 20-3 20-4

20-5 20-6

20-7 20

17.1 Synthesis of intermediate **20-1**

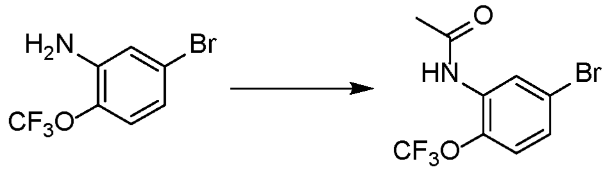

1-1 20-1

Intermediate **1-1** (10 g, 39.1 mmol) was dissolved in acetic anhydride (9.97 g, 97.6 mmol) and the mixture was reacted at room temperature for 12 h. The reaction liquid was concentrated under reduced pressure. The residue was subjected to silica gel column chromatography (methanol/dichloromethane = 0%-10%) to obtain intermediate **20-1** (10.5 g, yield 89.3%, white solid). MS (ESI) m/z = 297.9 $[M+H]^+$.

17.2 Synthesis of intermediate **20-3**

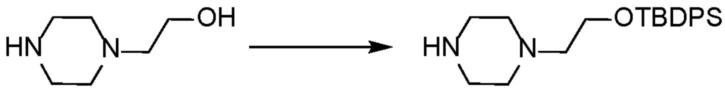

20-2 20-3

Intermediate **20-2** (2.50 g, 19.2 mmol) was dissolved in dichloromethane (50 mL), and TBDPSCl (6.33 g, 23.0 mmol), DMAP (0.23 g, 1.92 mmol) and imidazole ( 1.96 g, 28.8 mmol) were added. The mixture was reacted at room temperature for 12 h. The reaction liquid was concentrated under reduced pressure, and the residue was subjected to silica gel column chromatography (methanol/dichloromethane = 0%-10%) to obtain intermediate **20-3** (6.5 g, yield 91%, yellow solid). MS (ESI) m/z = 369.1 $[M+H]^+$.

17.3 Synthesis of intermediate **20-4**

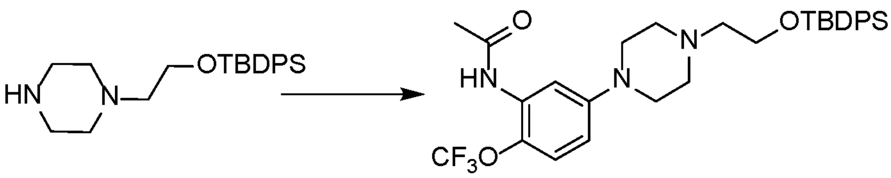

20-3 20-4

Intermediate **20-3** (3.56 g, 9.66 mmol) was dissolved in tetrahydrofuran (50 mL), LiHMDS (1 M, 18 mL) was added at 0°C, and the mixture was stirred at 0°C for 1 h. DavePhos (0.317 g, 0.805 mmol), $Pd_2(dba)_3$ (368.68 mg, 0.403 mmol) and intermediate **20-1** (2.4 g , 8.05 mmol) were added. The mixture was reacted at 80°C for 12 h. The reaction liquid was cooled to room temperature, water (100 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-10%) to obtain intermediate **20-4** (2.2 g, yield 46.3%, yellow oil). MS (ESI) m/z = 586.8 $[M+H]^+$.

17.4 Synthesis of intermediate **20-5**

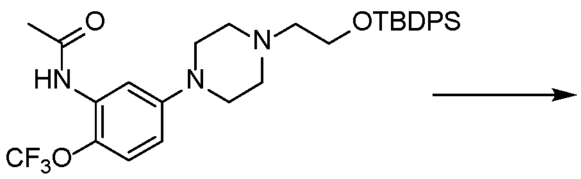

20-4 20-5

Intermediate **20-4** (2.4 g, 4.10 mmol) was dissolved in ethanol (20 mL) and hydrochloric acid (12 M, 112 mL) was added. The mixture was reacted at 80°C for 12 h. The reaction liquid was cooled to room temperature and concentrated under reduced pressure. The intermediate **20-5** (1.8 g, crude product) was obtained and used directly in the next step. MS (ESI) m/z = 306.1 $[M+H]^+$.

17.5 Synthesis of intermediate **20-6**

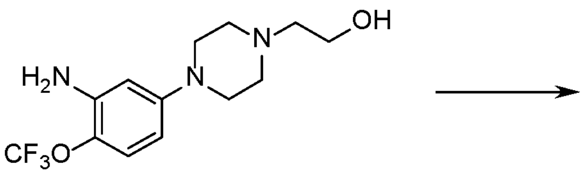

20-5 20-6

Intermediate **20-5** (1.8 g, 5.90 mmol) was dissolved in hydrochloric acid (12 M, 107 mL) and water (107 mL), and cyanamide (3.72 g, 88.44 mmol) was added. The mixture was reacted at 65°C for 48 h. The reaction liquid was cooled to room temperature, sodium hydroxide solution (1 M) was added to adjust pH = 13, and ethyl acetate (100 mL × 3) was added for extraction. The organic phases were combined, dried over anhydrous sodium sulfate, filtered, and the organic phase was concentrated under reduced pressure. The intermediate **20-6** (1.2 g, crude product) was obtained and used directly in the next step. MS (ESI) m/z = 348.1 $[M+H]^+$.

17.6 Synthesis of intermediate **20-7**

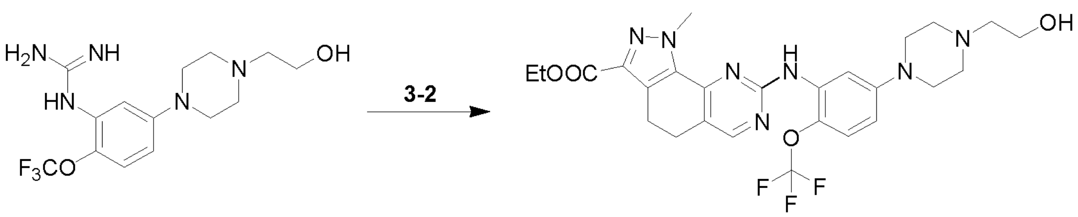


20-6 20-7

Intermediate **20-6** (1.2 g, 3.45 mmol) and intermediate **3-2** (0.80 g, 2.88 mmol) were dissolved in DMF (10 mL), and the mixture was reacted at 110°C for 16 h. The reaction liquid was quenched with water (50 mL), and extracted with ethyl acetate (50 mL × 3). The organic phases were combined, dried over anhydrous sodium sulfate, filtered, and the organic phase was concentrated under reduced pressure. The residue was subjected to silica gel column chromatography (methanol/dichloromethane = 0%-10%) to obtain intermediate **20-7** (0.21 g, yield 13.2%, yellow oil). MS (ESI) m/z = 562.2 $[M+H]^+$.

17.7 Synthesis of compound **20**

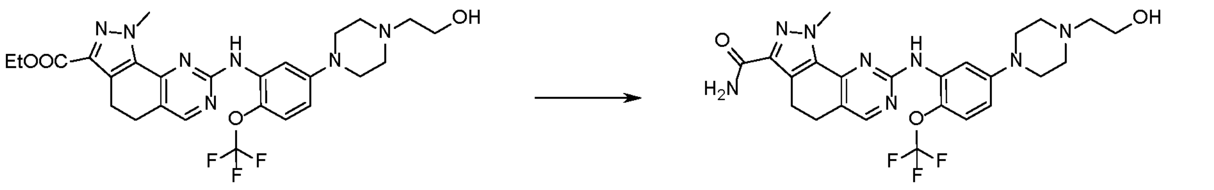

20-7 20

Intermediate **20-7** (150 mg, 267 μmol) was dissolved in a solution of ammonia in methanol (75 mL). The mixture was reacted for 48 h under sealed conditions at 85°C. The reaction liquid was concentrated under reduced pressure, and the residue was subjected to column chromatography (C18 75*30 mm*3 μm; mobile phase: [$H_2O$( $NH_4HCO_3$)-ACN]; B%: 27%-62%, 12 min). Compound **20** (21 mg, yield 15%, yellow solid) was obtained. MS (ESI) m/z =533.3 $[M+H]^+$. $^1H$ NMR (400 MHz, DMSO-*d6*) δ 8.86 - 8.94 (m, 1 H) 8.31 - 8.39 (m, 1 H) 7.45 (br s, 1 H) 7.17 - 7.31 (m, 3 H) 6.71 - 6.77 (m, 1 H) 4.37 - 4.47 (m, 1 H) 4.10 - 4.18 (m, 3 H) 3.50 - 3.56 (m, 2 H) 3.08 - 3.16 (m, 4 H) 2.91 - 3.00 (m, 2 H) 2.77 - 2.82 (m, 2 H) 2.51 - 2.56 (m, 4 H) 2.37 - 2.45 (m, 2 H).

**Example 18: Synthesis of compound 21**

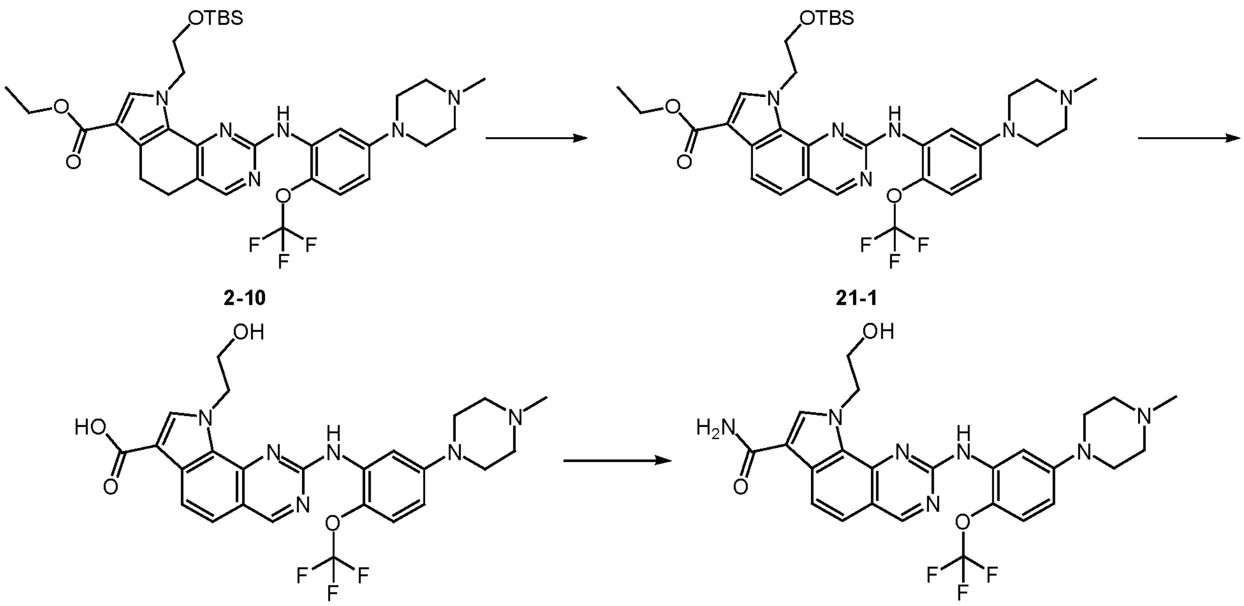

2-10 21-1

21-2 21

18.1 Synthesis of intermediate **21-1**

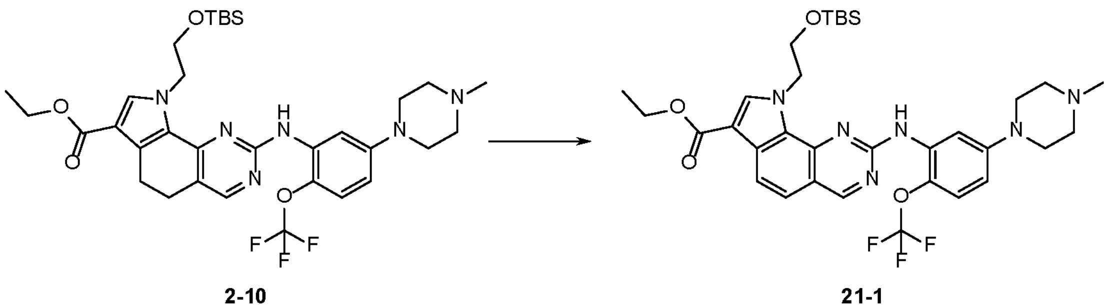

2-10 21-1

Intermediate **2-10** (100 mg, 148 μmol) and DDQ (168 mg, 741 μmol) were dissolved in dioxane (20 mL) and the mixture was reacted at 100°C for 12 h. Saturated $NaHCO_3$(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatogram (Waters Torus 2-PIC 150* 19 mm*5 μm; mobile phase: [heptane-ethanol (0.1% $NH_3H_2O$)]; B%: 0 %-20%, 15 min). The intermediate **21-1** (28 mg, yield 28.5%, yellow solid) was obtained. MS (ESI) m/z =673.3 $[M+H]^+$.

18.2 Synthesis of intermediate **21-2**

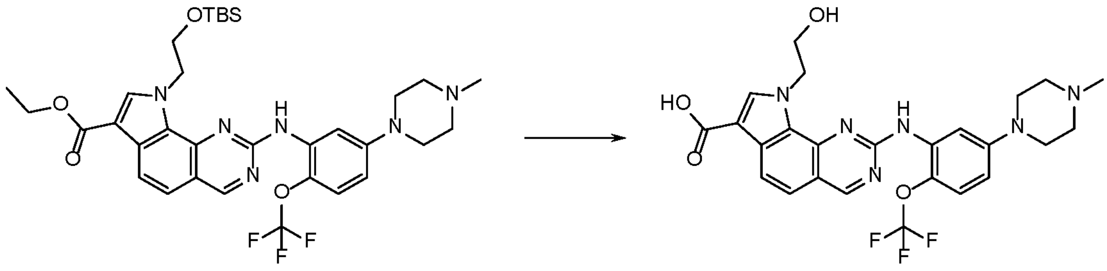

**21-1** **21-2**

Intermediate **21-1** (40.0 mg, 59.4 μmol) was dissolved in tetrahydrofuran (2 mL) and methanol (2 mL), and a solution of lithium hydroxide hydrate (24.9 mg, 594 μmol) in water (2 mL) was added. The mixture was reacted at room temperature for 24 h, and then reacted at 50°C for 24 h. The reaction liquid was concentrated under reduced pressure, the residue was slurried with petroleum ether and filtered, and the filter cake was collected and dried. Intermediate **21-2** (35 mg, crude product) was obtained. MS (ESI) m/z = 531.2 $[M+H]^+$.

18.3 Synthesis of compound **21**

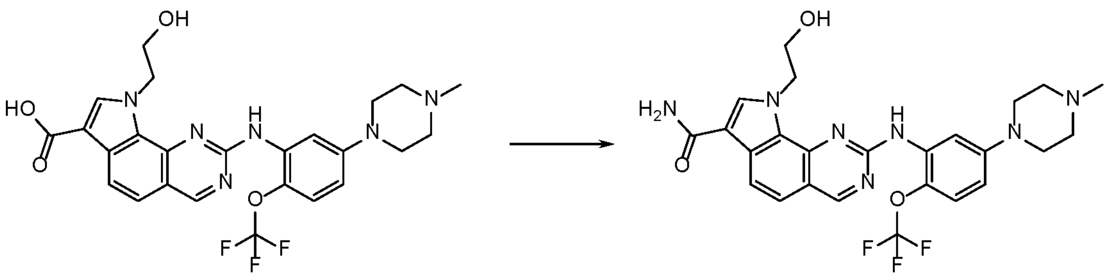

**21-2** **21**

Intermediate **21-2** (31.0 mg, 58.4 μmol), HATU (44.4 mg, 117 μmol) and DIEA (75.5 mg, 584 μmol) were dissolved in DMF (2 mL), and ammonium chloride (31.2 mg, 584 μmol) was added. The mixture was reacted at room temperature for 16 h. The reaction liquid was concentrated under reduced pressure, and the residue was purified by preparative liquid chromatogram (C18 150*30 mm*5 μm; mobile phase: [water ($NH_4HCO_3$)-acetonitrile]; B%: 27%- 62%, 14 min) to obtain compound **21** (10 mg, yield 32%, white solid). MS (ESI) m/z = 530.2 $[M+H]^+$. $^1H$ NMR (DMSO-$d_6$) δ 9.18 (s, 1H), 9.13 (s, 1H), 8.16 (d, *J* = 8.4 Hz, 1H), 8.06 (s, 1H), 7.50 - 7.48 (m, 2H), 7.26 (d, *J* = 8.8 Hz, 1H), 7.17 (d, *J* = 2.8 Hz, 1H), 6.87 - 6.84 (m, 2H), 4.66 (t, *J* = 5.2 Hz, 1H), 4.60 - 4.58 (m, 2H), 3.42 - 3.41 (m, 2H), 3.19 - 3.17 (m, 4H), 2.50 - 2.48 (m, 4H), 2.25 (s, 3H).

**Example 19: Synthesis of compound 22**

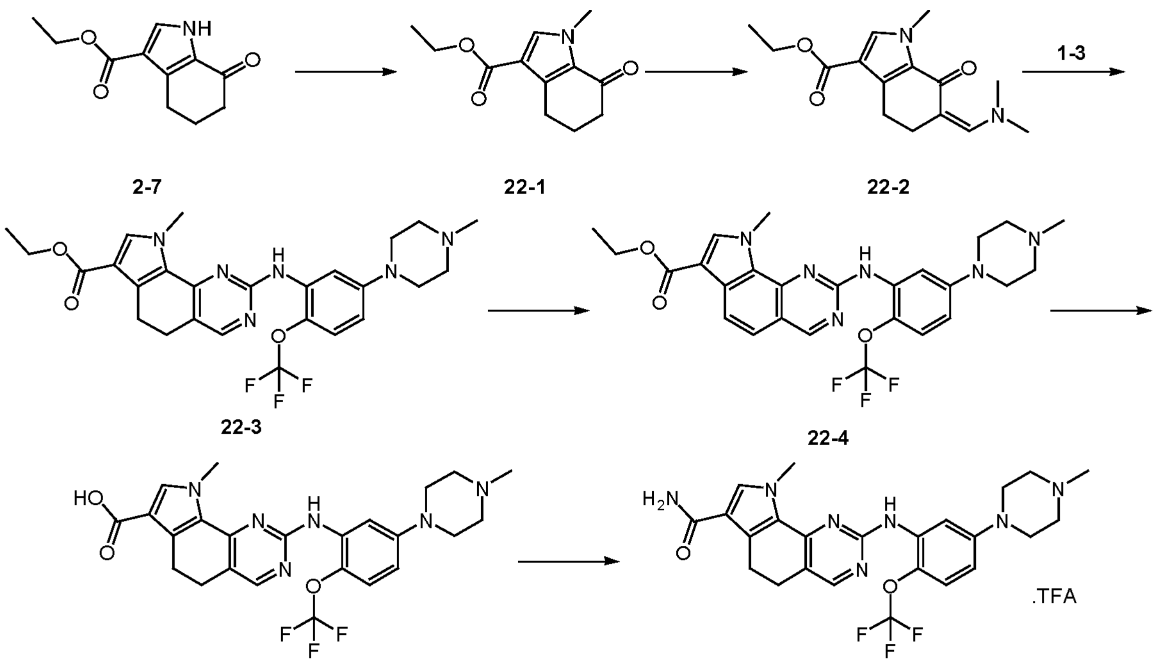


2-7 22-1 22-2

22-3 22-4

.TFA

22-5 22

19.1 Synthesis of intermediate **22-1**

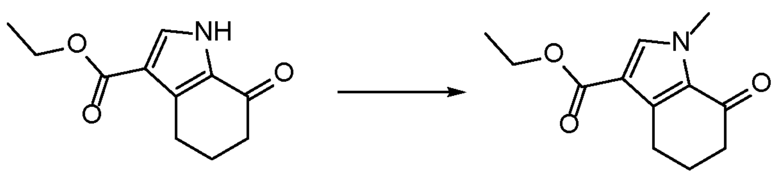

**2-7** **22-1**

Intermediate **2-7** (0.44 g, 2.12 mmol) was dissolved in DMF (5 mL), $K_2CO_3$ (0.67 g, 4.83 mmol) and iodomethane (0.68 g, 4.83 mmol) were added and the mixture was reacted at room temperature for 4 h. Water (50 mL) was added to quench the reaction, and dichloromethane (50 mL × 3) was added for extraction. The organic phases were combined, dried over anhydrous sodium sulfate, filtered, and the organic phase was concentrated under reduced pressure. The intermediate **22-1** (0.57 g, crude product) was obtained and used directly in the next step. MS (ESI) m/z = 222.2 $[M+H]^+$.

19.2 Synthesis of intermediate **22-2**

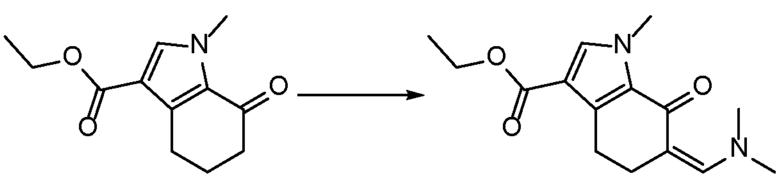

**22-1** **22-2**

Intermediate **22-1** (0.13 g, 0.588 mmol) was dissolved in DMF (5 mL), N,N-dimethylformamide dimethyl acetal (1.02 g, 8.58 mmol) was added, and the mixture was reacted under microwave at 145°C for 6 h. The mixture was cooled

to room temperature, water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The intermediate **22-2** (0.11 g, crude product) was obtained, which was used directly in the next step.

19.3 Synthesis of intermediate **22-3**

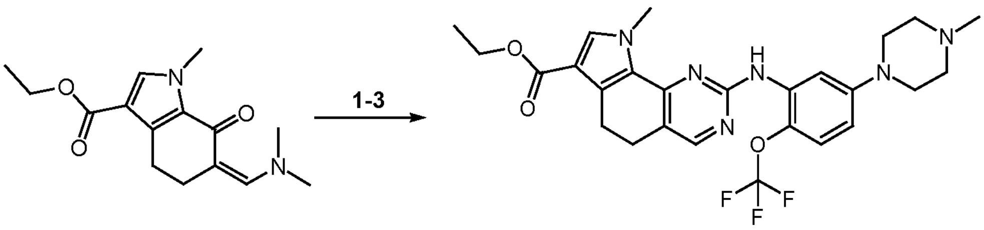


22-2 22-3

Intermediate **22-2** (0.11 g, 0.398 mmol) and intermediate **1-3** (0.19 g, 0.597 mmol) was dissolved in DMF (5 mL). The mixture was reacted at 110°C for 16 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (methanol/dichloromethane = 0%~8%) to obtain intermediate **22-3** (0.046 g, yield 21.6%, brown solid). MS (ESI) m/z = 531.2 $[M+H]^+$.

19.4 Synthesis of intermediate **22-4**

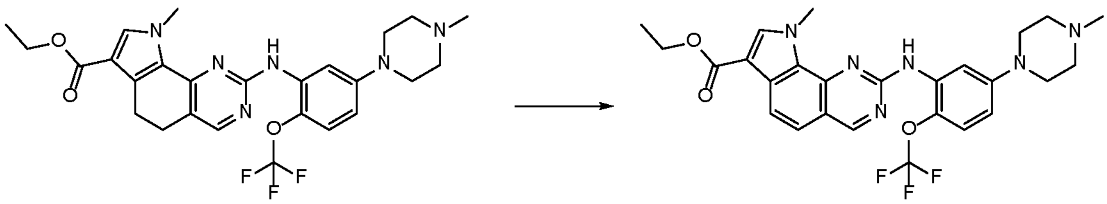

22-3 22-4

Intermediate **22-3** (60 mg, 113 μmol) and DDQ (128 mg, 565 μmol) were dissolved in dioxane (12 mL) and the mixture was reacted at 100°C for 2 h. Saturated $NaHCO_3$(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatogram (C18 150* 19 mm*5 μm; mobile phase: [heptane-ethanol (0.1% $NH_3H_2O$)]; B%: 0 %-20%, 15 min). The intermediate **22-4** (22 mg, yield 36.6%, yellow solid) was obtained. MS (ESI) m/z =529.2 $[M+H]^+$.

19.5 Synthesis of intermediate **22-5**

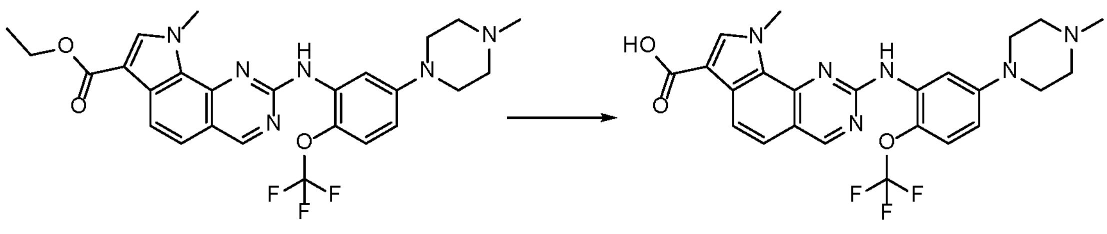

22-4 22-5

Intermediate **22-4** (30.0 mg, 56.7 μmol) was dissolved in tetrahydrofuran (2 mL) and methanol (2 mL), and a solution of lithium hydroxide hydrate (23.8 mg, 567 μmol) in water(2 mL) was added. The mixture was reacted at room temperature for 24 h, then reacted at 50°C for 24 h. The reaction liquid was concentrated under reduced pressure to obtain intermediate **22-5** (30 mg, crude product), which was directly used in the next step. MS (ESI) m/z = 501.2 $[M+H]^+$.

19.6 Synthesis of compound 22

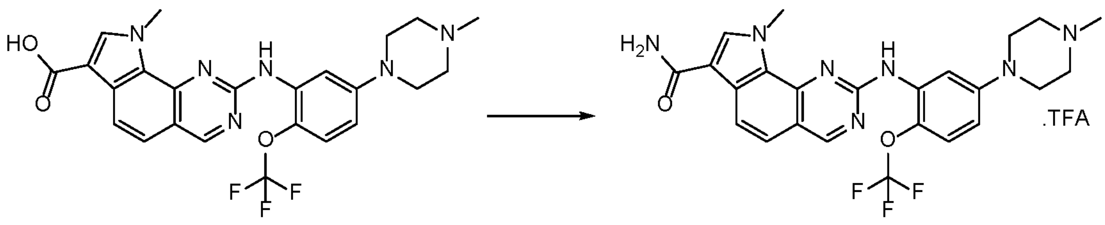

22-5 22

Intermediate **22-5** (28.0 mg, 55.9 μmol), HATU (31.9 mg, 83.9 μmol) and DIEA (72.3 mg, 559 μmol) were dissolved in DMF (2 mL), and ammonium chloride (29.9 mg, 559 μmol) was added. The mixture was reacted at room temperature for 16 h. The reaction liquid was concentrated under reduced pressure, and the residue was purified by preparative liquid chromatogram (C18 150*30 mm*5 μm; mobile phase: [water (TFA)-acetonitrile]; B%: 20%-55%, 25 min) to obtain the compound **22** (5.00 mg, yield 14.6%, trifluoroacetate, yellow solid). MS (ESI) m/z = 500.2 $[M+H]^+$. $^1H$ NMR (400 MHz $CD_3OD$) δ 9.32 (s, 1H), 8.32 (d, *J* = 8.8 Hz, 1H), 8.13 (d, *J* = 5.2 Hz, 1H), 7.65 (d, *J* = 8.8 Hz, 1H), 7.59 (d, *J* = 3.2 Hz, 1H), 7.40 (d, *J*= 9.2 Hz, 1H), 7.08 - 7.05 (m, 1H), 4.24 (s, 3H), 3.93 (d, *J*= 13.2 Hz, 2H), 3.64 (d, *J*= 11.6 Hz, 2H), 3.35 - 3.25 (m, 2H), 3.24 - 3.17 (m, 2H), 2.99 (s, 3H).

**Example 20: Synthesis of compound 23**

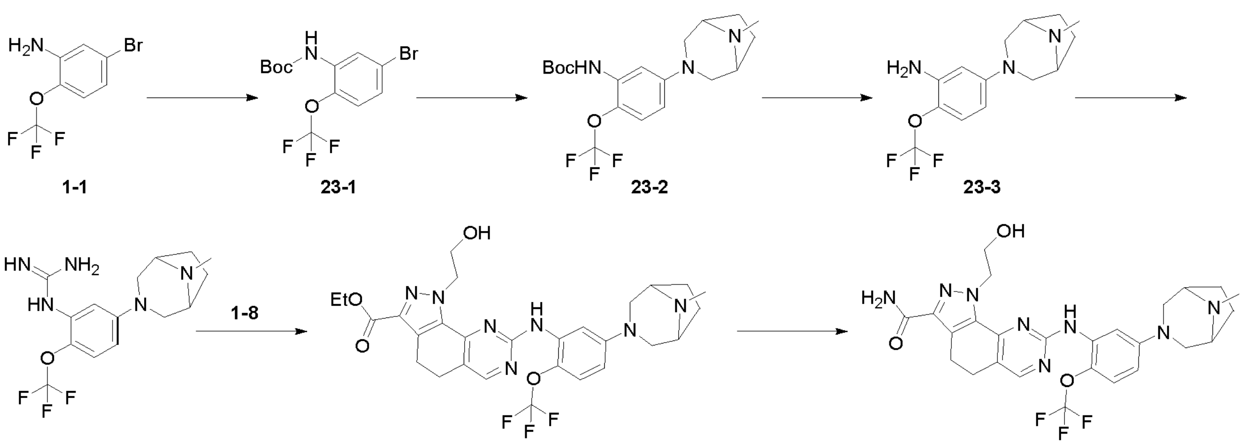


1-1 23-1 23-2 23-3

23-4 23-5 23

20.1 Synthesis of intermediate **23-1**

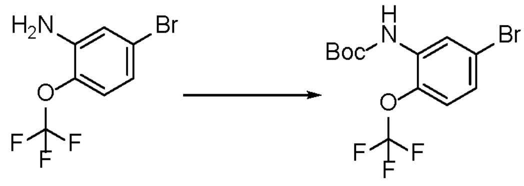

**1-1** **23-1**

Intermediate **1-1** (2.0 g, 7.81 mmol) was dissolved in tetrahydrofuran (50 mL), KHMDS (1.0 M solution in tetrahydrofuran , 15.6 mL, 15.6 mmol) was added at 0°C under nitrogen protection, and the mixture was reacted at 0°C for 0.5 h. Then BoczO (1.7 g, 7.81 mol) was added at 0°C under nitrogen protection, and the reaction was carried out at room temperature for 2 h. Water (20 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 10%-20%) to obtain intermediate **23-1** (2.2 g, yield 79%, yellow oil). MS (ESI) m/z = 356.0 $[M+H]^+$.

20.2 Synthesis of intermediate **23-2**

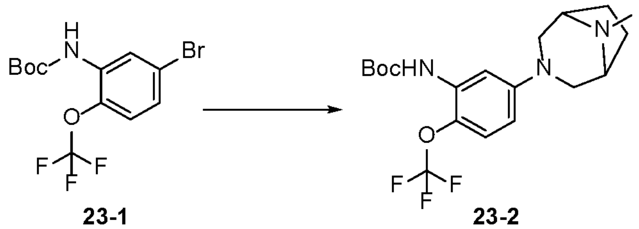

**23-1** **23-2**

The intermediate **23-1** (2.2 g, 6.18 mmol), $Cs_2CO_3$ (9.07 g, 27.8 mmol), $Pd_2(dba)_3$ (286.2 mg, 0.6 mmol) and X-Phos (274.5 mg, 0.3 mmol) were dissolved in dioxane (100 mL), and 8-methyl-3,8-diaza-bicyclo[3.2.1]octane hydrochloride (1.23 g, 6.18 mmol) was added under nitrogen protection. The mixture was reacted at 100°C overnight. Water (200 mL) was added to quench the reaction, and ethyl acetate (100 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 30%-50%) to obtain intermediate **23-2** (1.87 g, yield 75%, white solid). MS (ESI) m/z = 402.1 $[M+H]^+$.

20.3 Synthesis of intermediate **23-3**

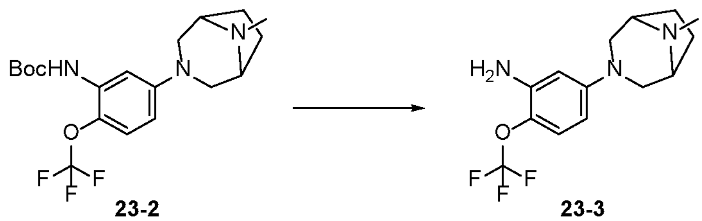

**23-2** **23-3**

Intermediate **23-2** (1.87 g, 4.66 mmol) was dissolved in hydrochloric acid/dioxane (20 mL) and the mixture was reacted at room temperature for 2 h. The reaction liquid was concentrated under reduced pressure. The residue was subjected to silica gel column chromatography (methanol/dichloromethane = 5%-10%) to obtain intermediate **23-3** (0.73 g, yield 51.7%, yellow solid). MS (ESI) m/z = 301.8$[M+H]^+$.

20.4 Synthesis of intermediate **23-4**

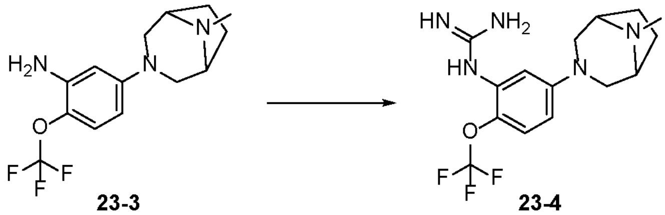

23-3 23-4

Intermediate **23-3** (500 mg, 1.66 mmol), cyanamide (175 mg, 4.17 mmol) and TMSCl (254 mg, 3.54 mmol) were dissolved in acetonitrile (15 mL). The mixture was reacted under microwave at 140°C for 1.5 h. The reaction liquid was concentrated under reduced pressure, and the residue was subjected to silica gel column chromatography (methanol/dichloromethane = 5%-10%) to obtain intermediate **23-4** (530 mg, yield 94%, yellow solid). MS (ESI) m/z = 344.0[M+H]$^+$.

20.5 Synthesis of intermediate **23-5**

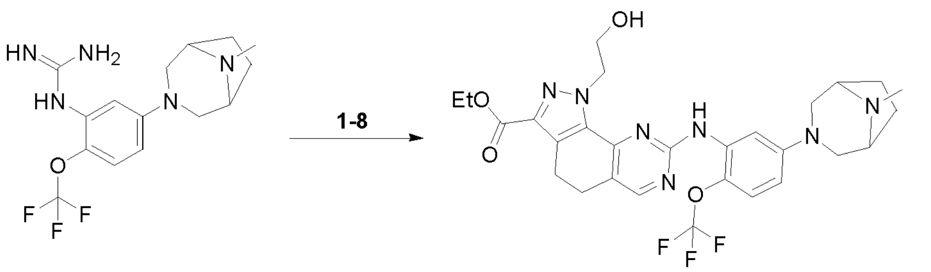


23-4 23-5

Intermediate **1-8** (475 mg, 1.56 mmol) and intermediate **23-4** (530 mg, 1.56 mmol) were dissolved in DMF (10 mL), and the mixture was reacted at 110°C for 16 h. The mixture was diluted with ethyl acetate (100 mL), and extracted with water (50 mL × 3). The organic phases were collected, dried over anhydrous sodium sulfate, filtered, and the organic phase was concentrated under reduced pressure. The residue was subjected to silica gel column chromatography (methanol/dichloromethane = 10%-13%) to obtain intermediate **23-5** (240 mg, yield 26.2%, white solid). MS (ESI) m/z = 588.0 [M+H]$^+$.

20.6 Synthesis of compound **23**

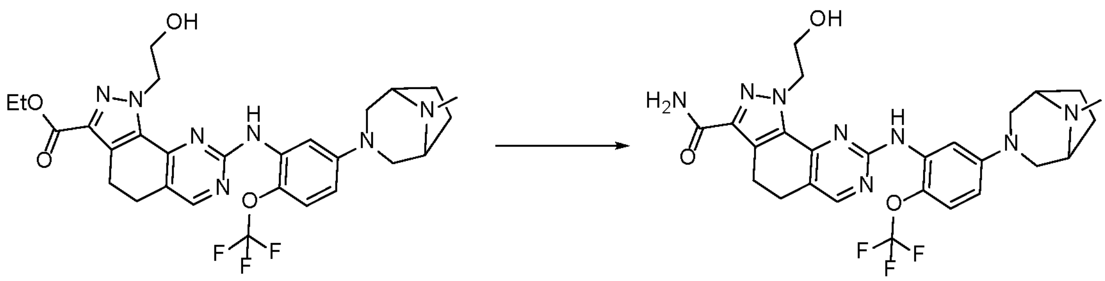

23-5 23

Intermediate **23-5** (200 mg, 0.340 mmol) was dissolved in $NH_3$/MeOH (10.0 mL). The mixture was reacted under microwave at 110°C for 2 h. The reaction liquid was concentrated under reduced pressure, and the residue was

purified by preparative liquid chromatogram (C18 30*150 mm*7 μm; mobile phase: [water-acetonitrile]; B%: 5%-30%, 40 min). Compound **23** (56.7 mg, yield 37.5%, yellow solid) was obtained. MS (ESI) m/z =559.0 $[M+H]^+$. $^1H$ NMR (400MHz, DMSO-*d6*) δ 8.82 (s, 1H), 8.32 (s, 1H), 7.43 (s, 1H), 7.24 (s, 1H), 7.17 (dd, *J* = 1.2 Hz, 9.2 Hz, 1H), 7.06 (d, *J* = 2.8 Hz, 1H), 6.49 (dd, *J* = 3.2 Hz, 9.2 Hz, 1H), 4.62 - 4.58 (m, 3H), 3.61 (d, *J* = 5.2 Hz, 2H), 3.31 (s, 2H), 3.19 (s, 2H), 2.97 (t, *J* = 7.6 Hz, 2H), 2.85 - 2.77 (m, 4H), 2.22 (s, 3H), 1.95 - 1.93 (m, 2H), 1.61 (d, *J* = 7.6 Hz, 2H).

**Example 21: Synthesis of compound 24**

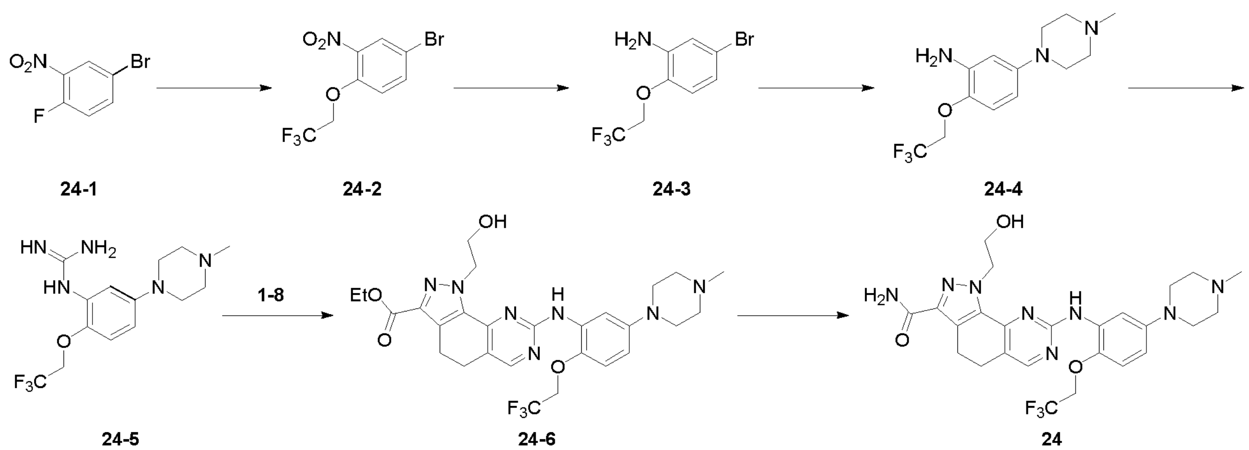


24-1 24-2 24-3 24-4

24-5 24-6 24

21.1 Synthesis of intermediate **24-2**

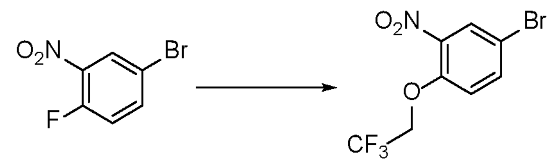

**24-1** **24-2**

Intermediate **24-1** (7.0 g, 31.8 mmol), trifluoroethanol (31.8 g, 318 mmol) and cesium carbonate (25.9 g, 79.5 mmol) were reacted at 70°C for 4 h. The reaction liquid was filtered, and the filtrate was dissolved in ethyl acetate (200 mL), and the obtained product was washed with water (200 mL). The organic phases were collected, dried over anhydrous sodium sulfate, filtered, and the organic phase was concentrated under reduced pressure. The intermediate **24-2** (7.8 g, yield 80%, yellow oil) was obtained. MS (ESI) m/z = 299.9 $[M+H]^+$.

21.2 Synthesis of intermediate **24-3**

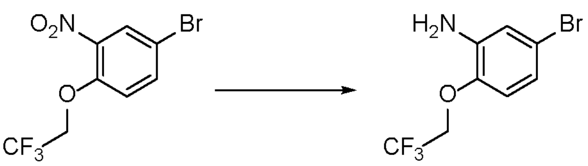

**24-2** **24-3**

Intermediate **24-2** (5.1 g, 17.0 mmol) was dissolved in ethanol/acetic acid (200 mL/200 mL), iron powder (9.49

g, 170 mmol) was added, and the mixture was reacted at 80°C for 2 h. The reaction liquid was filtered, and the filtrate was concentrated under reduced pressure. The residue was dissolved in ethyl acetate (200 mL), and the obtained product was washed with saturated sodium bicarbonate (200 mL) and water (200 mL). The organic phases were collected, dried over anhydrous sodium sulfate, filtered, and the organic phase was concentrated under reduced pressure. The intermediate **24-3** (3.2 g, yield 68.5%, yellow solid) was obtained. MS (ESI) m/z = 270.0 $[M+H]^+$.

21.3 Synthesis of intermediate **24-4**

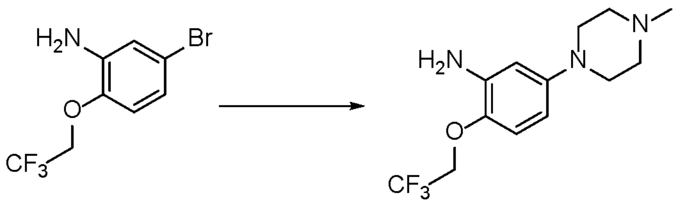

**24-3** **24-4**

Intermediate **24-3** (3.0 g, 11.1 mmol), $K_3PO_4$ (4.71 g, 22.2 mmol), BFMO (0.29 g, 0.6 mmol), CuI (0.21 g, 1.1 mmol), BFMO (0.28 g, 1.1 mmol) and N-methylpiperazine (3.34 g, 33.3 mmol) were dissolved in ethanol (50 mL). The mixture was reacted at 100°C for 24 h under nitrogen protection. The reaction liquid was cooled to room temperature, dissolved in dichloromethane (50 mL), filtered, and the organic phase was collected. The organic phase was dried over anhydrous sodium sulfate, filtered, and the organic phase was concentrated under reduced pressure. The residue was subjected to silica gel column chromatography (ethyl acetate/petroleum ether = 10%-50%) to obtain intermediate **24-4** (1.2 g, yield 36.9%, white solid). MS (ESI) m/z = 290.2 $[M+H]^+$.

21.4 Synthesis of intermediate **24-5**

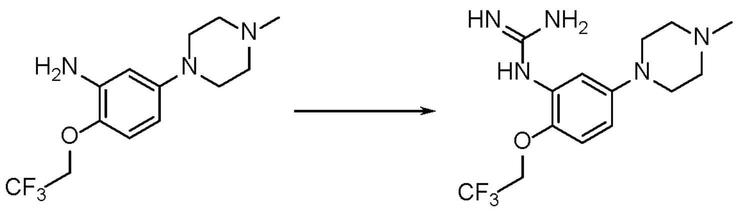

**24-4** **24-5**

Intermediate **24-4** (1.1 g, 3.8 mmol), cyanamide (0.32 g, 7.6 mmol) and TMSCl (0.62 g, 5.7 mmol) were dissolved in acetonitrile (15 mL). The mixture was reacted under microwave at 140°C for 2 h. The reaction liquid was concentrated under reduced pressure, and the residue was subjected to column chromatography (C18, $ACN/H_2O$ = 3/97 - 25/75) to obtain intermediate **24-5** (1.0 g, yield 78.9%, yellow solid). MS (ESI) m/z = 332.0$[M+H]^+$.

21.5 Synthesis of intermediate **24-6**

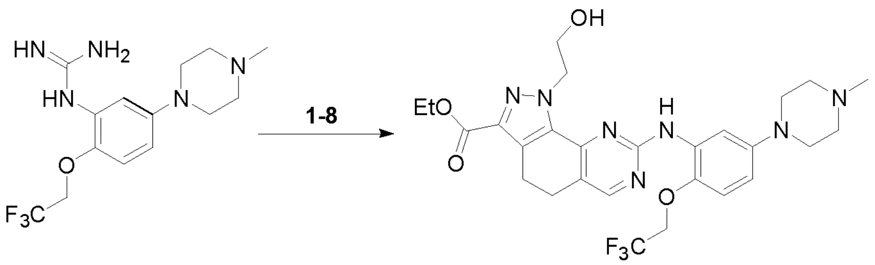


24-5 24-6

Intermediate **1-8** (1.06 g, 3.44 mmol) and intermediate **24-5** (0.95 g, 2.86 mmol) were dissolved in DMF (60 mL) and the mixture was reacted at 110°C for 3 h. The reaction liquid was concentrated under reduced pressure, and the residue was subjected to column chromatography (C18, ACN/$H_2O$ = 5/95 - 80/20) to obtain intermediate **24-6** (0.40 g, yield 24.5%, yellow solid). MS (ESI) m/z = 576.0$[M+H]^+$.

21.6 Synthesis of compound **24**

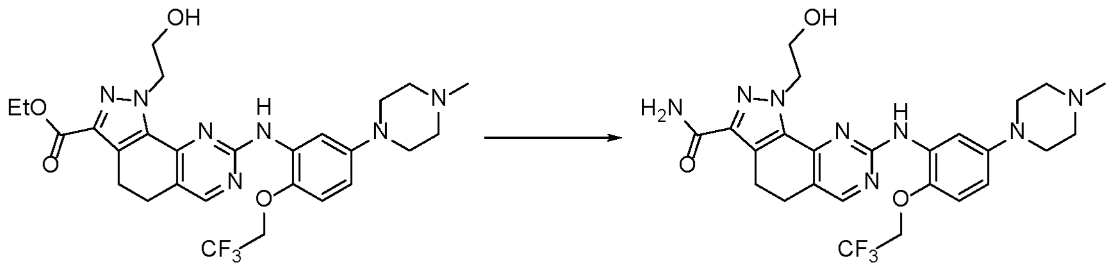

24-6 24

Intermediate **24-6** (300 mg, 0.52 mmol) was dissolved in $NH_3$/MeOH (10.0 mL). The mixture was reacted for 17 h under sealed conditions at 120°C. The reaction liquid was concentrated under reduced pressure, and the residue was purified by preparative liquid chromatogram (C18 30* 150 mm*7 μm; mobile phase: [water-acetonitrile]; B%: 5%-30%, 40 min). Compound **24** (105 mg, yield 36%, yellow solid) was obtained. MS (ESI) m/z =547.1 $[M+H]^+$. $^1$H NMR (400 MHz, DMSO-*d6*) δ 8.37 (s, 1H), 8.18 (s, 1H), 7.50-7.47 (m, 2H), 7.27 (s, 1H), 7.06 (d, *J*= 8.8 Hz, 1H), 6.66 (d, *J*= 8.8, 2.8 Hz, 1H), 4.70-4.63 (m, 5H), 3.72-3.70 (m, 2H), 3.06-3.04 (m, 4H), 2.97 (t, *J*= 7.6 Hz, 2H), 2.79 (t, *J*= 7.6 Hz, 2H), 2.50-2.44 (m, 4H), 2.22 (s, 3H).

**Example 22: Synthesis of compound 25**

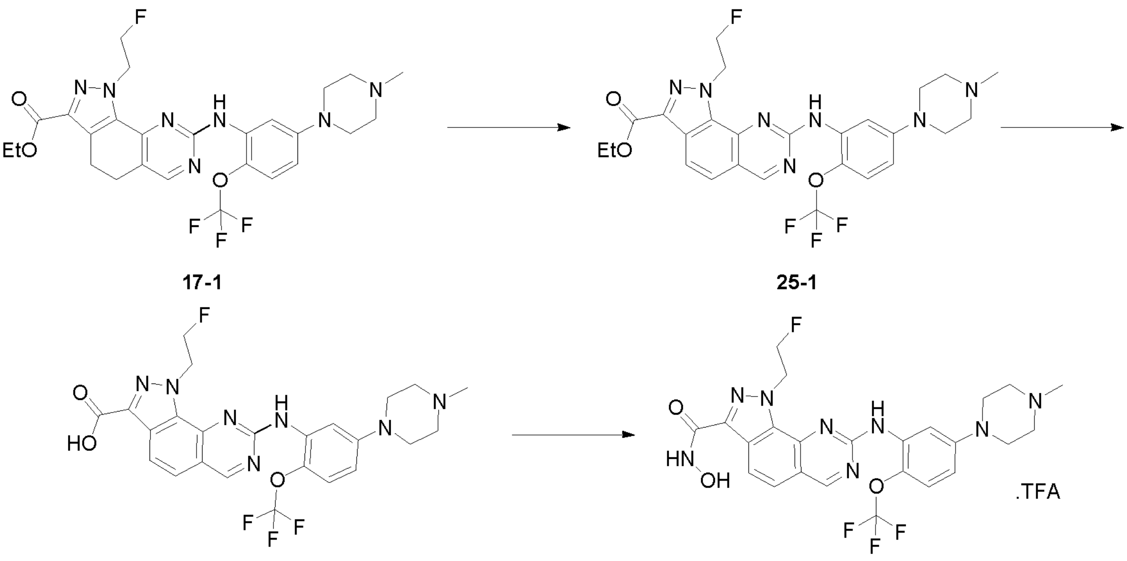

17-1 25-1

25-2 25

22.1 Synthesis of intermediate **25-1**

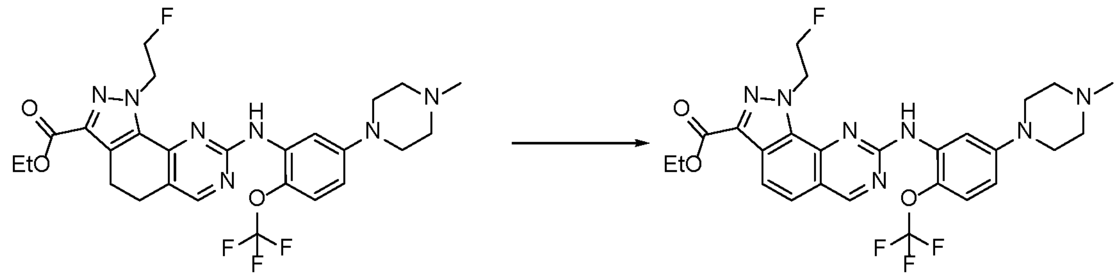

17-1 25-1

Intermediate **17-1** (160 mg, 0.280 mmol) and DDQ (319 mg, 1.41 mmol) were dissolved in dioxane (20 mL) and the mixture was reacted at 100°C for 2 h. Saturated NaHCOs(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatogram (C18 150*40 mm* 10 μm; mobile phase: [water ($NH_4HCO_3$)-ACN -acetonitrile]; B%: 45%-85%, 10 min). Intermediate **25-1** (57 mg, yield 35.7%, brown solid) was obtained. MS (ESI) m/z =562.2 $[M+H]^+$.

22.2 Synthesis of intermediate **25-2**

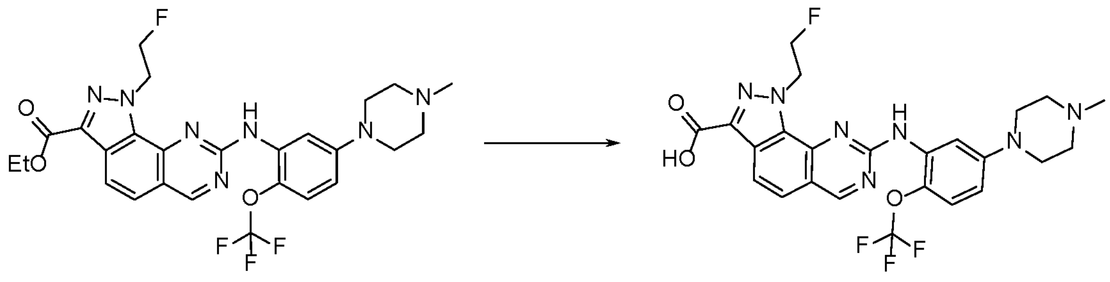

25-1 25-2

**Intermediate25-1** (45.0 mg, 82.2 μmol) was dissolved in methanol (2 mL) and lithium hydroxide aqueous solution (1 M, 411 μL) was added. The mixture was reacted at room temperature for 6 h. The pH was adjusted to 5-7 with hydrochloric acid at 0°C, the residue was purified by preparative liquid chromatogram (C18 150*30 mm*5 μm; mobile phase: [water (TFA)-acetonitrile]; B%: 10%- 50%, 10 min) to obtain intermediate **25-2** (14.8 mg, yield 30.1%, brown solid). MS (ESI) m/z = 534.2 $[M+H]^+$.

22.3 Synthesis of compound **25**

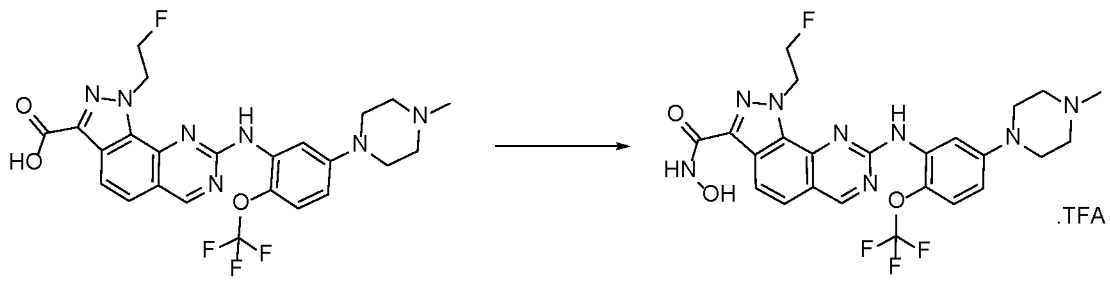


25-2 25

Intermediate **25-2** (20.9 mg, 39.3 μmol), HATU (16.4 mg, 43.2 μmol) and DIEA (10.2 mg, 78.67 μmol) were dissolved in DMF (2 mL), and hydroxylamine hydrochloride (10.2 mg, 78.67 μmol) was added. The mixture was reacted at room temperature for 2 h. The reaction liquid was concentrated under reduced pressure, and the residue was purified by preparative liquid chromatogram (C18 150*30 mm*5 μm; mobile phase: [water (TFA)-acetonitrile]; B%: 20%-50%, 25 min) to obtain the compound **25** (2.5 mg, yield 9.6%, brown solid). MS (ESI) m/z = 549.2 $[M+H]^+$. $^1H$ NMR (400 MHz, MeOD) δ 9.22 (s, 1 H), 8.05 (d, *J*= 8.76 Hz, 1 H), 7.53 - 7.61 (m, 2 H), 7.36 (d, *J* = 8.88 Hz, 1 H), 6.97 (dd, *J* = 9.07, 2.81 Hz, 1 H), 5.21 - 5.31 (m, 2 H), 4.81 (d, *J* = 4.88 Hz, 1 H), 4.69 (t, *J* = 4.88 Hz, 1 H), 3.93 (d, *J* = 13.51 Hz, 2 H), 3.64 (d, *J* = 12.01 Hz, 2 H), 3.26 - 3.30 (m, 2 H), 3.05 - 3.17 (m, 2 H), 2.99 (s, 3 H).

**Example 23: Synthesis of compound 16**

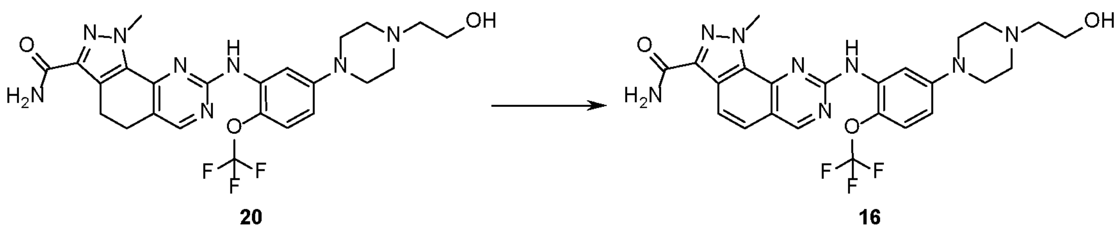

20 16

Compound **20** (149 mg, 0.28 mmol) and DDQ (319 mg, 1.41 mmol) were dissolved in dioxane (20 mL) and the mixture was reacted at 100°C for 2 h. Saturated $NaHCO_3$(20 mL) was added to quench the reaction, and ethyl acetate

(20 mL × 3) was added for extraction. The organic phases were combined, dried over anhydrous sodium sulfate, filtered, and the organic phase was concentrated under reduced pressure. The residue was purified by preparative liquid chromatogram (C18 150*40 mm* 10 μm; mobile phase: [water ($NH_4HCO_3$)-ACN -acetonitrile]; B%: 45%-85%, 10 min). Compound 16 (14.8 mg, yield 10%, brown solid) was obtained. MS (ESI) m/z =531.2 $[M+H]^+$.

**Example 24: Synthesis of compound 26**

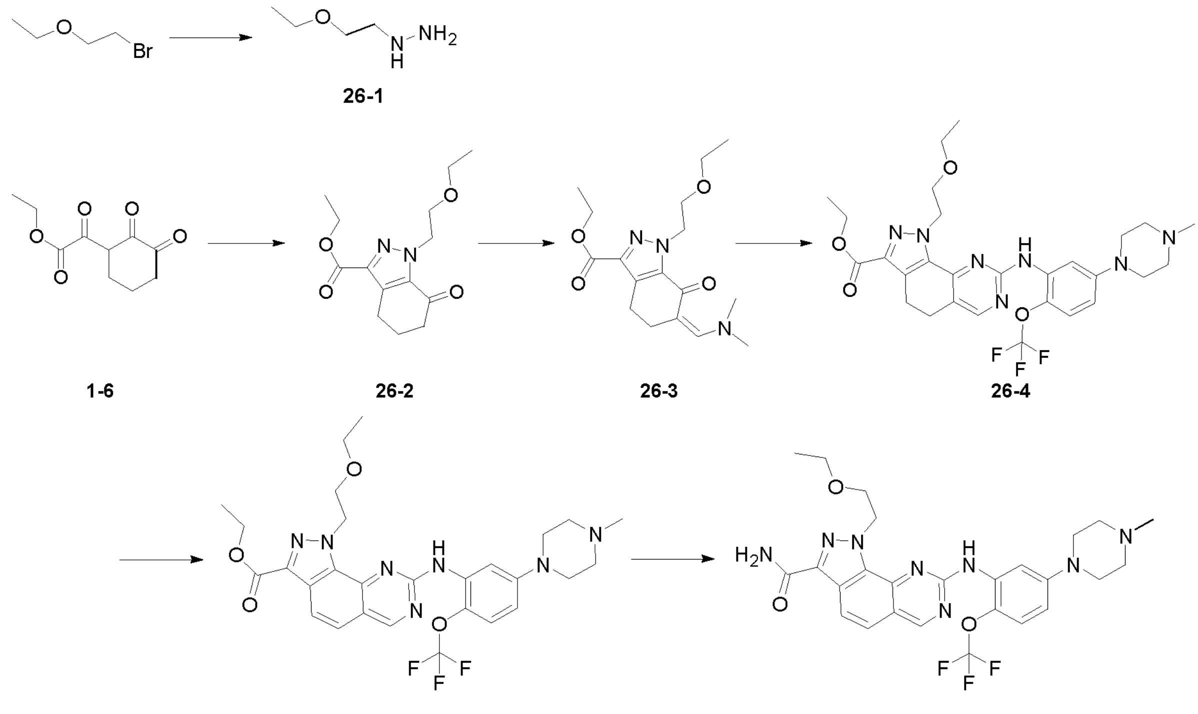

26-1

1-6 26-2 26-3 26-4

26-5 26

24.1 Synthesis of intermediate **26-1**

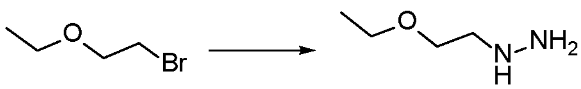

**26-1**

Compounds 2-bromoethyl ethyl ether (1.00 g, 6.54 mmol) and tert-butyl 2-(propane-2-ylidene)hydrazinecarboxylate(1.13 g, 6.54 mmol) were dissolved in acetonitrile (40 mL), and NaH (324 mg, 13.5 mmol) was added. The mixture was reacted at 80°C for 12 h and detection showed that the reaction was completed. Water was added to quench the reaction, ethyl acetate (40 mL × 3) was added for extraction, the obtained product was dried over anhydrous sodium sulfate, and filtered, the organic phase was collected, and concentrated under reduced pressure. The residue was dissolved in ethyl acetate hydrochloride (4 M, 10 mL) and the mixture was reacted at room temperature overnight. The reaction liquid was concentrated, the residue was dissolved in ethanol (20 mL), concentrated hydrochloric acid (5 mL) was added, and the mixture was stirred overnight. The reaction liquid was concentrated under reduced pressure to obtain intermediate **26-1** (0.95 g, crude product), which was directly used in the next step.

24.2 Synthesis of intermediate **26-2**

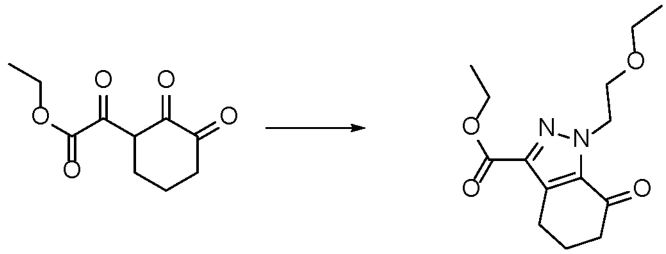

1-6 26-2

Intermediate **1-6** (850 mg, 3.54 mmol) was dissolved in acetic acid (10 mL), compound **26-1** (737 mg, 7.08 mmol) was added, and the mixture was reacted at room temperature for 15 h. Water (20 mL) was added to quench the reaction, saturated $Na_2CO_3$ (100 mL) was added, and dichloromethane (80 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-5%) to obtain intermediate **26-2** (473 mg, yield 47.8%, yellow solid). MS (ESI) m/z = 281.2 [M+H]$^+$.

24.3 Synthesis of intermediate **26-3**

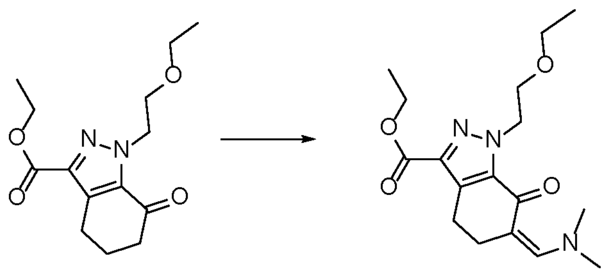

26-2 26-3

Intermediate **26-2** (600 mg, 2.14 mmol) was dissolved in DMF-DMA (10 mL) and the mixture was reacted at 110°C for 12 h. The reaction liquid was concentrated under reduced pressure to obtain intermediate **26-3** (650 mg, crude product), which was directly used in the next step.

24.4 Synthesis of intermediate **26-4**

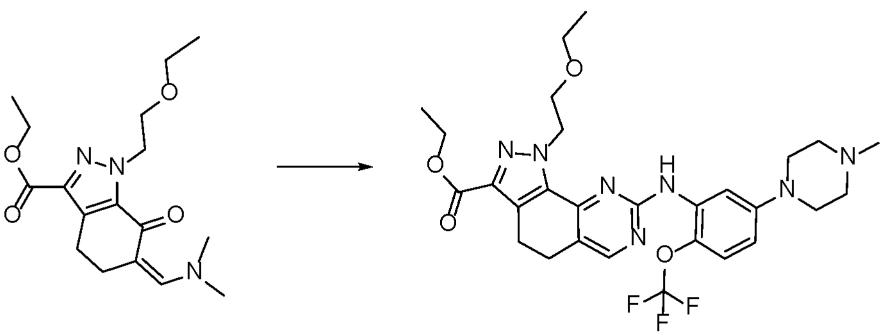

26-3 26-4

Intermediate **26-3** (600 mg, 1.79 mmol) and intermediate 1-3 (851 mg, 2.68 mmol) were dissolved in DMF (20 mL) and the mixture was reacted at 110°C for 12 h. The reaction liquid was concentrated under reduced pressure, and

the residue was subjected to silica gel column chromatography (ethyl acetate/petroleum ether = 0%-5%) to obtain intermediate **26-4** (228 mg, yield 21.6%, yellow solid). MS (ESI) m/z = 590.3 $[M+H]^+$.

24.5 Synthesis of intermediate **26-5**

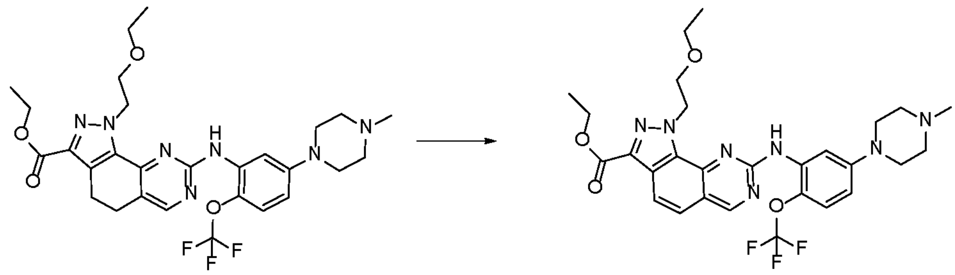

26-4 26-5

Intermediate **26-4** (44.0 mg, 74.6 μmol) and DDQ (88.0 mg, 387 μmol) were dissolved in dioxane (20 mL) and the mixture was reacted at 100°C for 12 h. Saturated $NaHCO_3$(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatography (C18 150*40 mm* 10 μm; mobile phase: [water (TFA)-acetonitrile]; B%: 44%-84%, 30 min). Intermediate **26-5** (16 mg, yield 36.5%, brown solid) was obtained. MS (ESI) m/z =588.4 $[M+H]^+$.

24.6 Synthesis of compound 26

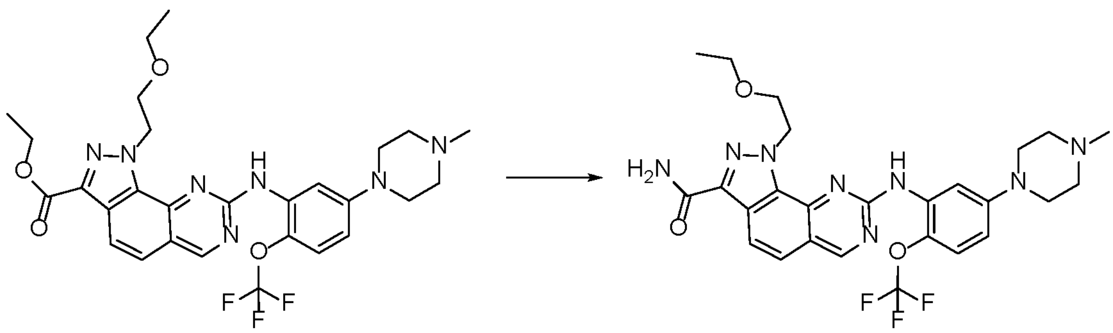

**26-5** **26**

Intermediate **26-5** (16 mg, 27.2 μmol) was dissolved in NMP (6 mL), and $NH_3$/MeOH (50.0 mL) was added. The mixture was reacted for 48 h under sealed conditions at 80.0°C. The reaction liquid was concentrated under reduced pressure, and the residue was purified by preparative liquid chromatogram (C18 30* 150 mm*7 μm; mobile phase: [water (TFA)-acetonitrile]; B%: 10%-80%, 40 min). Compound **26** (56.7 mg, yield 37.5%, yellow solid) was obtained. MS (ESI) m/z =531.2 $[M+H]^+$. $^1H$ NMR(400 MHz, DMSO-*d6*) δ 9.59 (s, 1 H), 9.38 (s, 1 H), 8.05 - 8.07 (d, *J*= 8.00 Hz, 1 H), 7.82 (s, 1 H), 7.63 - 7.66 (d, *J*= 12.0 Hz, 1 H), 7.53 (s, 1 H), 7.28 - 7.36 (m, 2 H), 6.93 - 6.95 (m, 1 H), 5.03 - 5.06 (t, *J*= 8.00 Hz, 2 H), 3.67 - 3.70 (t, *J*= 8.00 Hz, 2 H), 3.17 - 3.25 (m, 6 H), 2.56 - 2.57 (m, 3 H), 2.28 (s, 3 H), 1.28 - 1.37 (m, 1 H), 0.88 - 0.91 (m, 3 H).

**Example 25: Synthesis of compound 27**

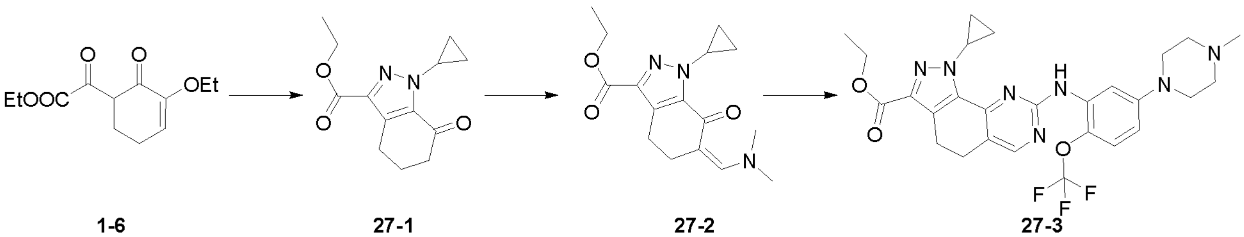

1-6 27-1 27-2 27-3

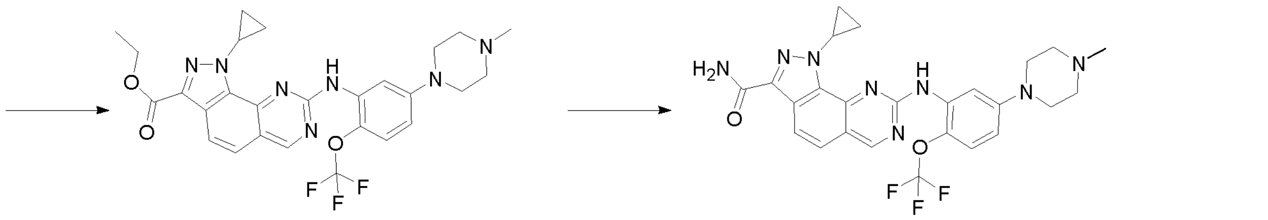

27-4 27-6

25.1 Synthesis of intermediate **27-1**

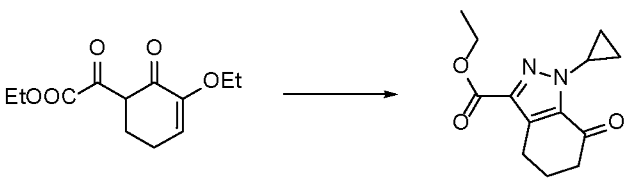

1-6 27-1

Intermediate **1-6** (200 mg, 832 μmol) was dissolved in acetic acid (10 mL), compound cyclopropylhydrazine (71.9 mg, 998 μmol) was added, and the mixture was reacted at room temperature for 15 h. Water (20 mL) was added to quench the reaction, saturated $Na_2CO_3$ (100 mL) was added, and dichloromethane (80 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-10%) to obtain intermediate **27-1** (90 mg, yield 43.6%, yellow solid). MS (ESI) m/z = 249.1 $[M+H]^+$.

25.2 Synthesis of intermediate **27-2**

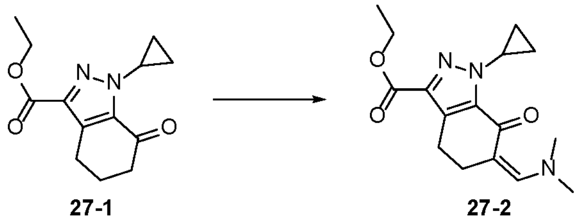

27-1 27-2

Intermediate **27-1** (500 mg, 2.01 mmol) was dissolved in DMF-DMA (10 mL) and the mixture was reacted at 110°C for 12 h. The reaction liquid was concentrated under reduced pressure, and the residue was dissolved in methyl tertiary ether and the mixture was stirred for 3 h. The resulting product was filtered, the filter cake was collected, and dried to obtain intermediate **27-2** (500 mg, crude product), which was directly used in the next step.

25.3 Synthesis of intermediate **27-3**

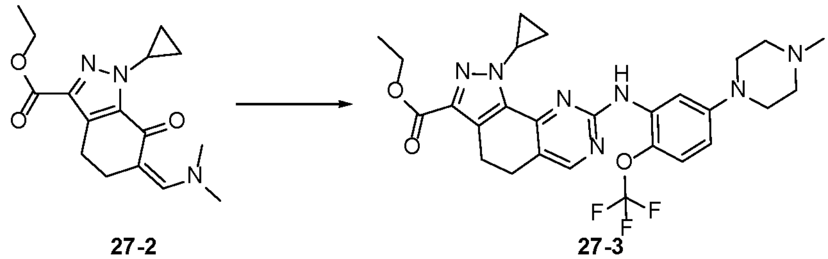

**27-2** **27-3**

Intermediate **27-2** (500 mg, 1.65 mmol) and intermediate **1-3** (630 mg, 1.98 mmol) were dissolved in DMF (20 mL) and the mixture was reacted at 110°C for 12 h. The reaction liquid was concentrated under reduced pressure, and the residue was subjected to silica gel column chromatography (ethyl acetate/petroleum ether = 0%-5%) to obtain intermediate **27-3** (100 mg, yield 10.9%, yellow solid). MS (ESI) m/z = 558.2 $[M+H]^+$.

25.4 Synthesis of intermediate **27-4**

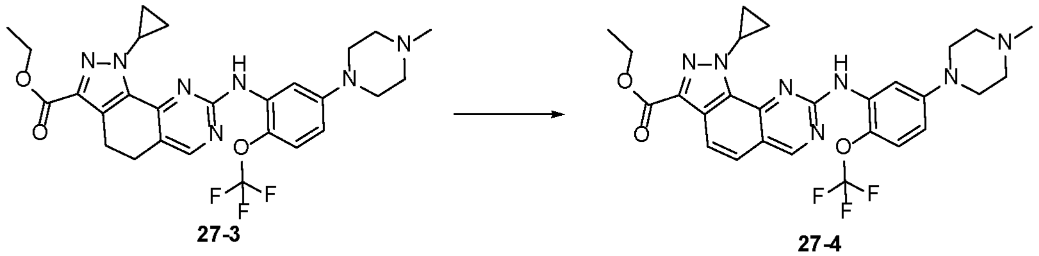

**27-3** **27-4**

Intermediate **27-3** (50.0 mg, 89.7 μmol) and DDQ (101 mg, 444 μmol) were dissolved in dioxane (20 mL) and the mixture was reacted at 100°C for 12 h. Saturated $NaHCO_3$(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatogram (C18 150*40 mm* 10 μm; mobile phase: [water (TFA)-acetonitrile]; B%: 51%-81%, 10 min). Intermediate **27-4** (30 mg, yield 20.1%, brown solid) was obtained. MS (ESI) m/z =556.3 $[M+H]^+$.

25.5 Synthesis of compound **27**

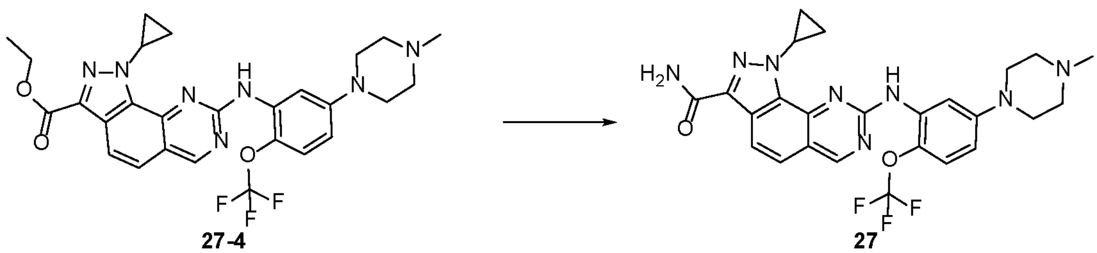

**27-4** **27**

Intermediate 27-**4** (30.0 mg, 54.0 μmol) was dissolved in NMP (6 mL), and $NH_3$/MeOH (50.0 mL) was added. The mixture was reacted for 48 h under sealed conditions at 80.0°C. The reaction liquid was concentrated under reduced pressure, and the residue was purified by preparative liquid chromatogram (C18 30* 150 mm*7 μm; mobile phase: [water (TFA)-acetonitrile]; B%: 40%-80%, 14 min). Compound 27 (17.0 mg, yield 59.8%, yellow solid) was obtained. MS (ESI) m/z = 527.2 $[M+H]^+$. $^1H$ NMR (400 MHz, DMSO-*d6*) δ 9.50 (s, 1H), 9.32 (s, 1H), 7.96 (d, *J*= 8.40 Hz, 1H), 7.72 (s, 1H,)

7.58 (d, J= 8.40 Hz, 1H), 7.44 (s, 1H), 7.29 (d, J= 2.80 Hz, 2H), 6.83 (dd, J= 9.20, 2.88 Hz, 1H), 4.83 - 4.92 (m, 1H), 3.33 (s, 13H), 3.08 - 3.17 (m, 4H), 2.39 - 2.47 (m, 5H), 2.21 (s, 3H), 1.28 - 1.33 (m, 2H), 1.23 (br s, 1H), 0.82 - 0.89 (m, 1H), 0.81 - 0.90 (m, 1H).

**Example 26: Synthesis of compound 28**

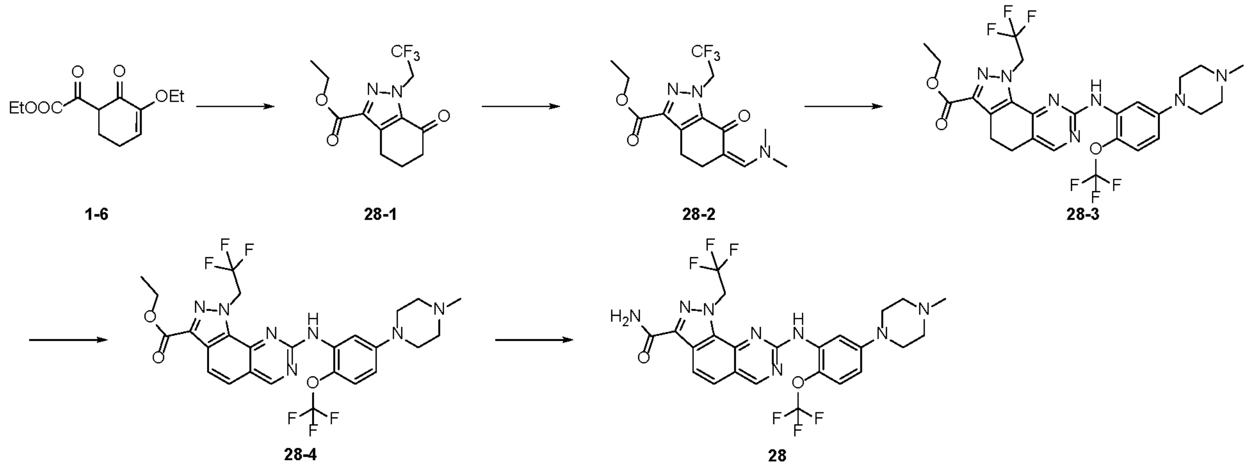

1-6 28-1 28-2 28-3

28-4 28

26.1 Synthesis of intermediate **28-1**

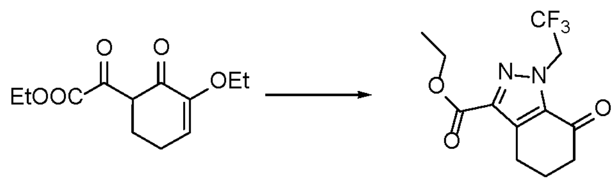

1-6 28-1

Intermediate **1-6** (2.00 g, 8.32 mmol) was dissolved in acetic acid (20 mL), compound trifluoroethylhydrazine (1.14 g, 9.99 mmol) was added, and the mixture was reacted at room temperature for 15 h. Water (20 mL) was added to quench the reaction, saturated $Na_2CO_3$ (200 mL) was added, and dichloromethane (80 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-10%) to obtain intermediate **28-1** (1.18 g, yield 49.1%, yellow solid). MS (ESI) m/z = 291.0 $[M+H]^+$.

26.2 Synthesis of intermediate **28-2**

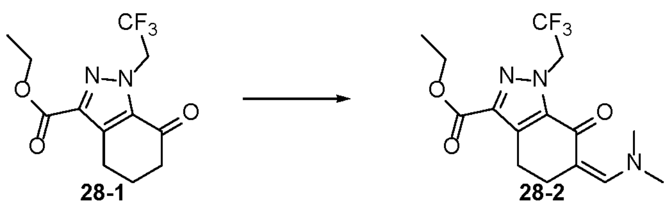

28-1 28-2

Intermediate **28-1** (1.23 g, 4.24 mmol) was dissolved in DMF-DMA (30 mL) and the mixture was reacted at 110°C for 12 h. The reaction liquid was concentrated under reduced pressure, and the residue was dissolved in methyl

tertiary ether and the mixture was stirred for 3 h. The resulting product was filtered, the filter cake was collected, and dried to obtain intermediate **28-2** (1.4 g, crude product), which was directly used in the next step.

26.3 Synthesis of intermediate **28-3**

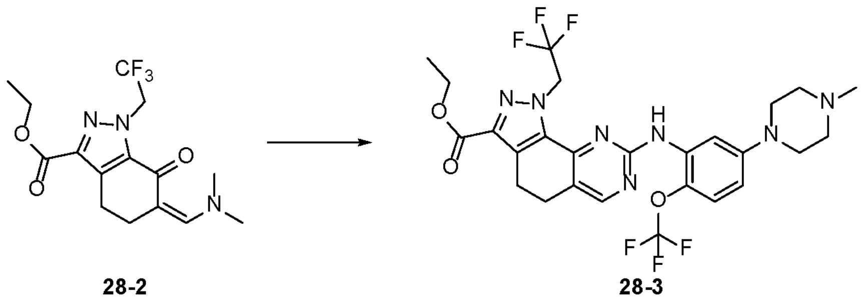

**28-2** **28-3**

Intermediate **28-2** (1.23 g, 3.56 mmol) and intermediate **1-3** (1.36 g, 4.28 mmol) were dissolved in DMF (50 mL) and the mixture was reacted at 110°C for 12 h. The reaction liquid was concentrated under reduced pressure, and the residue was subjected to silica gel column chromatography (ethyl acetate/petroleum ether = 0%-5%) to obtain intermediate **28-3** (0.476 g, yield 22.3%, yellow solid). MS (ESI) m/z = 600.6 $[M+H]^+$.

26.4 Synthesis of intermediate **28-4**

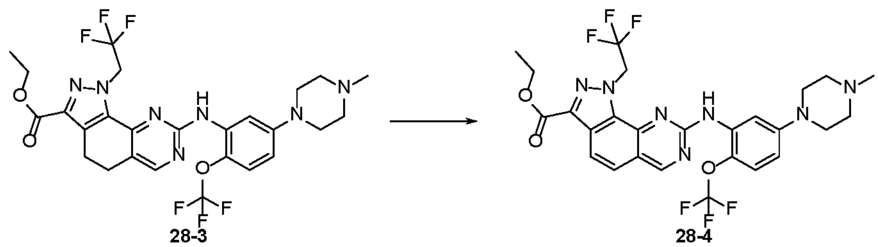

**28-3** **28-4**

Intermediate **28-3** (0.61 g, 1.02 mmol) and DDQ (1.14 g, 5.04 mmol) were dissolved in dioxane (20 mL) and the mixture was reacted at 100°C for 12 h. Saturated $NaHCO_3$(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatogram (C18 150*40 mm* 10 μm; mobile phase: [water (TFA)-acetonitrile]; B%: 50%-90%, 35 min). Intermediate **28-4** (0.078 g, yield 12.8%, brown solid) was obtained. MS (ESI) m/z = 598.2 $[M+H]^+$.

26.5 Synthesis of compound **28**

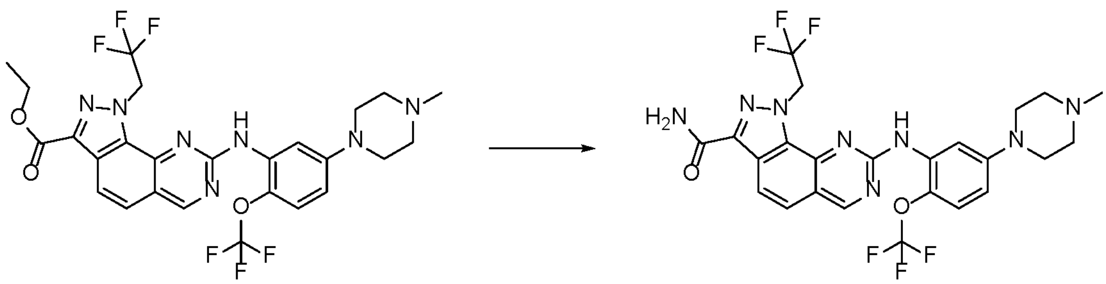

**28-4** **28**

Intermediate **28-4** (50.0 mg, 83.7 μmol) was dissolved in NMP (6 mL), and $NH_3$/MeOH (50.0 mL) was added. The mixture was reacted for 48 h under sealed conditions at 80.0°C. The reaction liquid was concentrated under reduced pressure, and the residue was purified by preparative liquid chromatogram (C18 30* 150 mm*7 μm; mobile phase: [water (TFA)-acetonitrile]; B%: 32%-72%, 36 min). Compound 28 (9.0 mg, yield 18.9%, yellow solid) was obtained. MS (ESI) m/z = 569.1 $[M+H]^+$. $^1H$ NMR (400 MHz, DMSO-*d6*) δ 9.66 (s, 1 H), 9.36 (s, 1H), 8.00 (d, *J*= 8.74 Hz, 1H), 7.55 - 7.84 (m, 3H), 7.25 (d, *J*= 9.00 Hz, 1H), 6.90 (dd, *J* = 9.14, 2.88 Hz, 1H), 5.80 (d, *J*= 8.50 Hz, 2H), 3.12 - 3.20 (m, 4H), 2.40 - 2.46 (m, 4H), 2.21 (s, 3H).

**Example 27: Synthesis of compound 29**

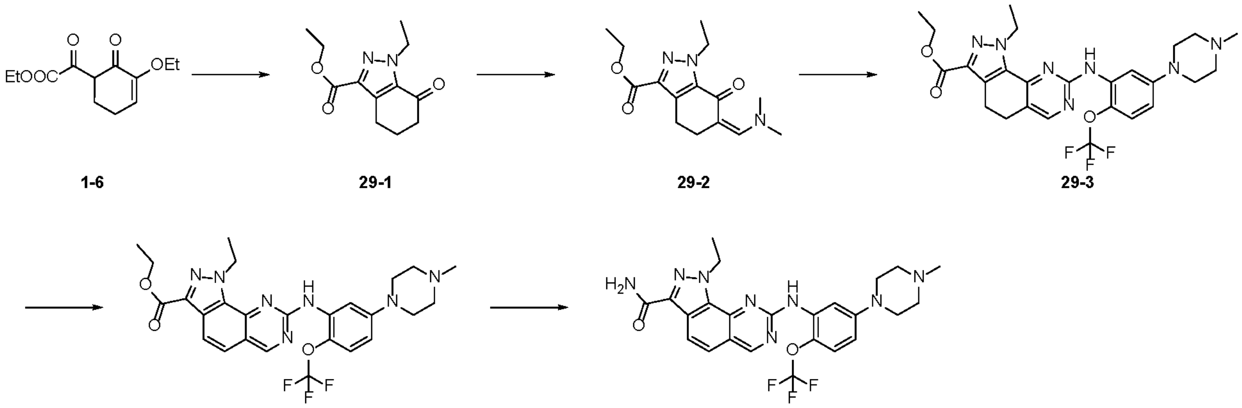

1-6 29-1 29-2 29-3

29-4 29

27.1 Synthesis of intermediate **29-1**

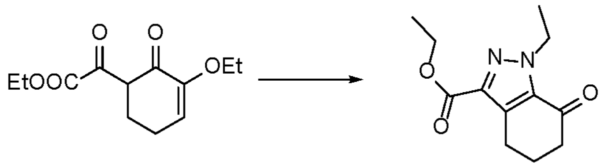

1-6 29-1

Intermediate **1-6** (1.60 g, 6.66 mmol) was dissolved in acetic acid (20 mL), compound ethylhydrazine (0.44 g, 7.33 mmol) was added, and the mixture was reacted at room temperature for 15 h. Water (20 mL) was added to quench the reaction, saturated $Na_2CO_3$ (200 mL) was added, and dichloromethane (80 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-10%) to obtain intermediate **29-1** (0.74 g, yield 47.1%, yellow solid). MS (ESI) m/z = 237.1 $[M+H]^+$.

27.2 Synthesis of intermediate **29-2**

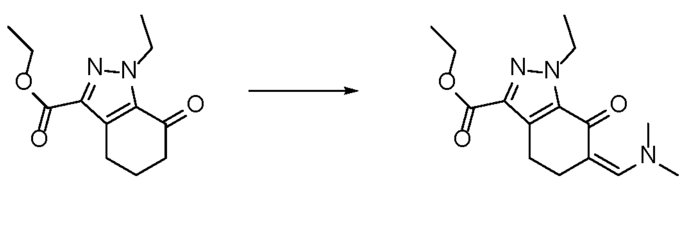

29-1 29-2

Intermediate **29-1** (1.00 g, 3.81 mmol) was dissolved in DMF-DMA (30 mL) and the mixture was reacted at 110°C for 12 h. The reaction liquid was concentrated under reduced pressure, and the residue was dissolved in methyl tertiary ether and the mixture was stirred for 3 h. The resulting product was filtered, the filter cake was collected, and dried to obtain intermediate **29-2** (1.0 g, crude product), which was directly used in the next step.

27.3 Synthesis of intermediate **29-3**

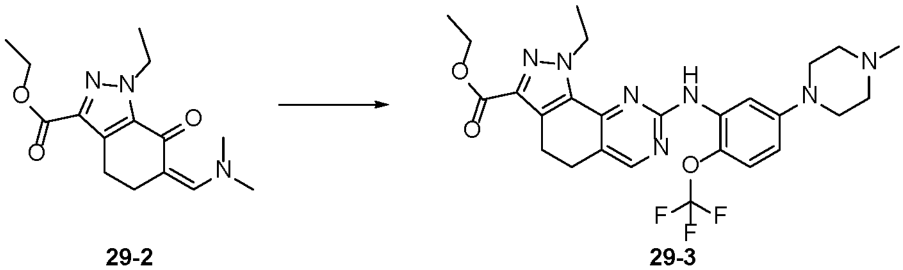

**29-2** **29-3**

Intermediate 29-2 (1.00 g, 3.43 mmol) and intermediate 1-3 (1.09 g, 3.43 mmol) were dissolved in DMF (50 mL) and the mixture was reacted at 110°C for 12 h. The reaction liquid was concentrated under reduced pressure, and the residue was subjected to silica gel column chromatography (methanol/dichloromethane = 0%-10%) to obtain intermediate **29-3** (0.70 g, yield 37.4%, yellow solid). MS (ESI) m/z = 546.2 $[M+H]^+$.

27.4 Synthesis of intermediate **29-4**

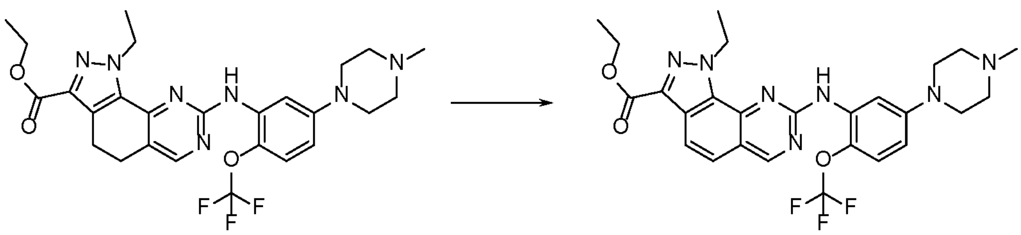

**29-3** **29-4**

Intermediate **29-3** (0.70 g, 1.28 mmol) and DDQ (1.02 g, 4.49 mmol) were dissolved in dioxane (20 mL) and the mixture was reacted at 100°C for 12 h. Saturated $NaHCO_3$(20 mL) was added to quench the reaction, and ethyl acetate (20 mL × 3) was added for extraction. The organic phases were combined, dried over anhydrous sodium sulfate, filtered, and the organic phase was concentrated under reduced pressure. The residue was purified by preparative liquid chromatogram (C18 150*40 mm* 10 μm; mobile phase: [water (TFA)-acetonitrile]; B%: 45%-85%, 10 min). The intermediate **29-4** (0.3 g, yield 43%, yellow solid) was obtained. MS (ESI) m/z = 544.3 $[M+H]^+$.

27.5 Synthesis of compound **29**

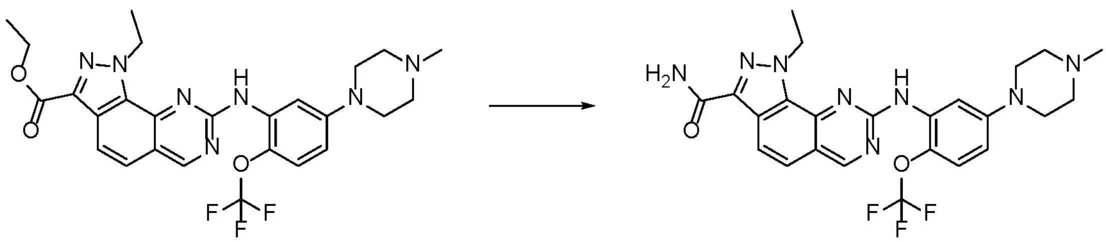

**29-4** **29**

Intermediate **29-4** (300 mg, 552 μmol) was dissolved in NMP (6 mL), and $NH_3$/MeOH (50.0 mL) was added. The mixture was reacted for 48 h under sealed conditions at 80.0°C. The reaction liquid was concentrated under reduced pressure, and the residue was purified by preparative liquid chromatogram (C18 30* 150 mm*7 μm; mobile phase: [water (TFA)-acetonitrile]; B%: 32%-72%, 35 min). Compound 29 (72 mg, yield 25.3%, yellow solid) was obtained. MS (ESI) m/z = 515.2 $[M+H]^+$. $^1H$ NMR (400 MHz, DMSO-*d6*) δ 9.52 (br s, 1 H), 9.31 (s, 1 H), 7.98 (d, *J*= 8.80 Hz, 1 H), 7.76 (s, 1 H), 7.57 (d, *J*= 8.80 Hz, 1 H), 7.45 (s, 1 H), 7.27 (d, *J*= 8.00 Hz, 1 H), 7.21 (d, *J*= 2.80 Hz, 1 H), 6.88 (dd, *J* = 9.20, 2.80 Hz, 1 H), 4.82 (q, *J*= 7.20 Hz, 2 H), 3.08 - 3.25 (m, 4 H), 2.42 - 2.43 (m, 4 H), 2.21 (s, 3 H), 1.23 (t, *J*= 7.20 Hz, 3 H).

**Example 28: Synthesis of compound 30**

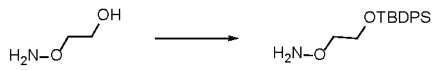

30-1

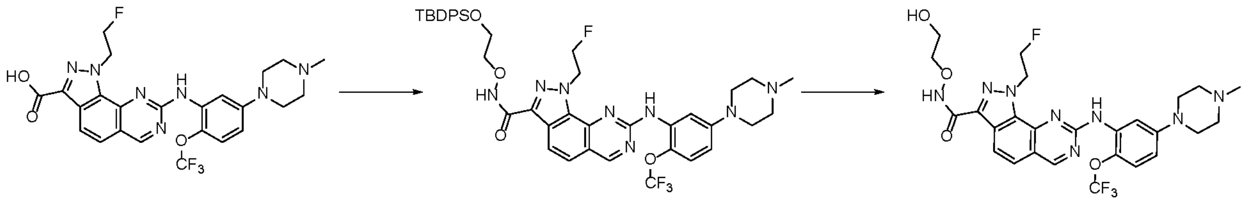

25-2 30-2 30

28.1 Synthesis of intermediate **30-1**

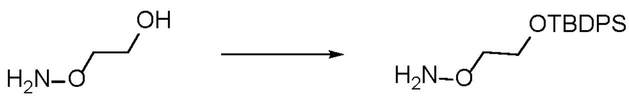

**30-1**

2-aminooxyethanol (1.00 g, 12.9 mmol) was dissolved in dichloromethane (20 mL), DMAP (0.158 g, 1.30 mmol), imidazole (1.32 g, 19.5 mmol) and TBDPSCl (4.28 g, 15.6 mmol) were added, and the mixture was reacted room temperature for 12 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-15%) to obtain intermediate **30-1** (1.59 g, yield 39%, oil). MS(ESI) m/z = 316.1$[M+H]^+$.

28.2 Synthesis of intermediate **30-2**

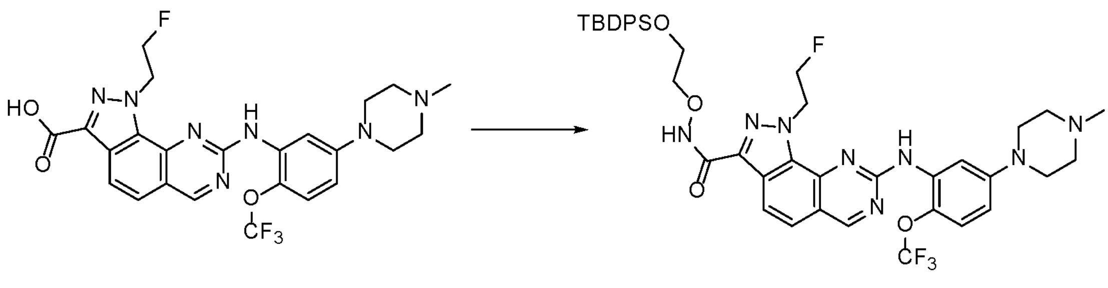

**25-2** **30-2**

Compound **25-2** (50.0 mg, 93.7 μmol) was dissolved in DMF (3.00 mL), and HATU (39.2 mg, 103 μmol) was

added. The obtained product was stirred at room temperature for 10 min, then DIEA (24.2 mg, 187 μmol) and intermediate **30-1** (59.1 mg, 187 μmol) were added. The mixture was reacted at room temperature for 1 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The intermediate **30-2** (60.0 mg, crude product) was obtained and used directly in the next step. MS (ESI) m/z = 831.5 [M+H]+.

28.3 Synthesis of compound **30**

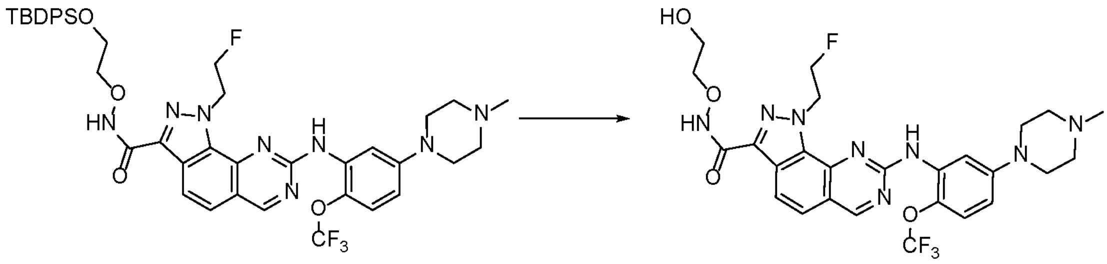

**30-2** **30**

Intermediate **30-2** (50.0 mg, 60.2 μmol) was dissolved in DCM (5 mL), triethylamine trihydrofluoride (19.4 mg, 120 μmol) was added, and the mixture was reacted at room temperature for 5 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was purified by preparative liquid chromatogram (C18 30* 150 mm*7 μm; mobile phase: [water (TFA)-acetonitrile]; B%: 13%-60%, 14 min). Compound **30** (60 mg, yield 76.0%, yellow solid) was obtained. MS (ESI) m/z = 593.0 [M+H]+. $^1$H NMR (400 MHz, DMSO-*d6*) δ 9.59 (s, 1H) 9.34 (s, 1H) 7.93 (d, *J*= 8.80 Hz, 1H) 7.63 (d, *J*= 8.80 Hz, 1H) 7.26 - 7.35 (m, 1H) 7.19 (d, *J*= 2.80 Hz, 1H) 6.89 (dd, *J*= 9.20,8.80 Hz, 1H) 5.06 - 5.22 (m, 2H) 4.69 - 4.81 (m, 2H) 4.61 (s, 1H) 3.95 (t, *J*= 4.80 Hz, 2H) 3.62 (m, *J* = 4.80 Hz, 2H) 3.14 - 3.22 (m, 4H) 2.40 - 2.45 (m, 4H) 2.21 (s, 3H).

**Example 29: Synthesis of compound 31**

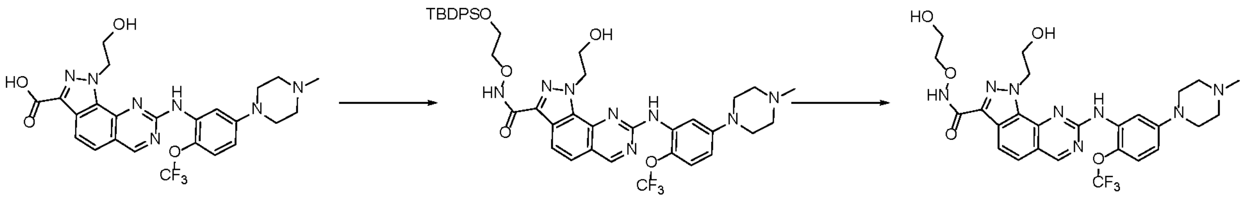

**19-1** **31-1** **31**

29.1 Synthesis of intermediate **31-1**

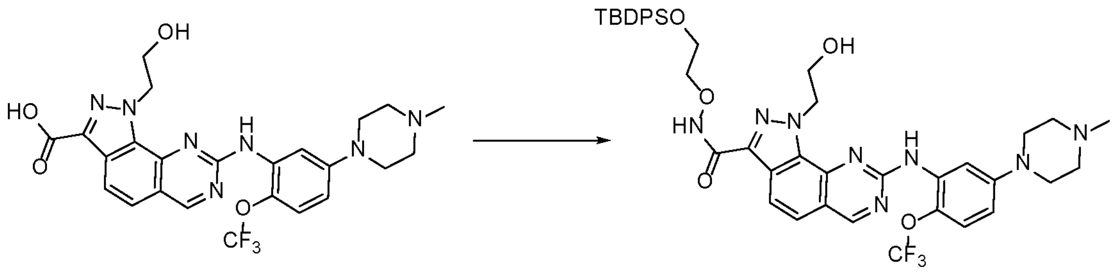

**19-1** **31-1**

Compound **19-1** (50.0 mg, 94.1 μmol) was dissolved in DMF (3.00 mL), and HATU (39.4 mg, 103 μmol) was added. The obtained product was stirred at room temperature for 10 min, then DIEA (24.3 mg, 188 μmol) and intermediate **30-1** (59.4 mg, 188 μmol) were added. The mixture was reacted at room temperature for 12 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The intermediate **31-1** (60.0 mg, crude product) was obtained and used directly in the next step. MS (ESI) m/z = 829.5 [M+H]+.

29.2 Synthesis of compound **31**

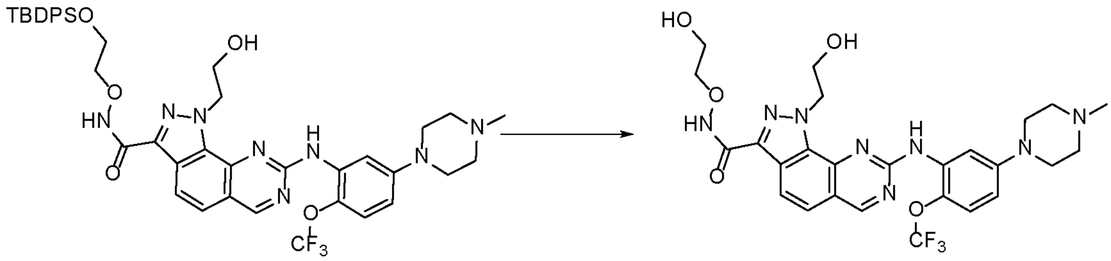

**31-1** **31**

Intermediate **31-1** (50.0 mg, 60.3 μmol) was dissolved in DCM (5 mL), triethylamine trihydrofluoride (48.6 mg, 301 μmol) was added, and the mixture was reacted at room temperature for 5 h. Water (50 mL) was added to quench the reaction, and ethyl acetate (50 mL × 3) was added for extraction. The organic phases were combined, dried over anhydrous sodium sulfate, filtered, and the organic phase was concentrated under reduced pressure. The residue was purified by preparative liquid chromatogram (C18 30* 150 mm*7 μm; mobile phase: [water (TFA)-acetonitrile]; B%: 25%-85%, 14 min). Compound **31** (22.0 mg, yield 30.6%, yellow solid) was obtained. MS (ESI) m/z = 591.3 [M+H]+. $^1$H NMR (400 MHz, DMSO-*d6*) δ 9.50 (s, 1H) 9.32 (s, 1H) 7.90 - 8.00 (m, 1H) 7.61 (d, *J*= 8.40 Hz, 1H) 7.19 - 7.31 (m, 2H) 6.88 (dd, *J*= 9.07, 3.20 Hz, 1H) 4.88 (m, 2H) 4.76 - 4.84 (m, 1H) 4.57 - 4.63 (m, 1H) 3.88 - 3.96 (m, 2H) 3.55 - 3.72 (m, 5H) 3.15 - 3.21 (m, 4H) 2.42 - 2.46 (m, 4H) 2.18 - 2.22 (m, 1H) 2.22 (s, 3H).

**Example 30: Synthesis of compound 32**

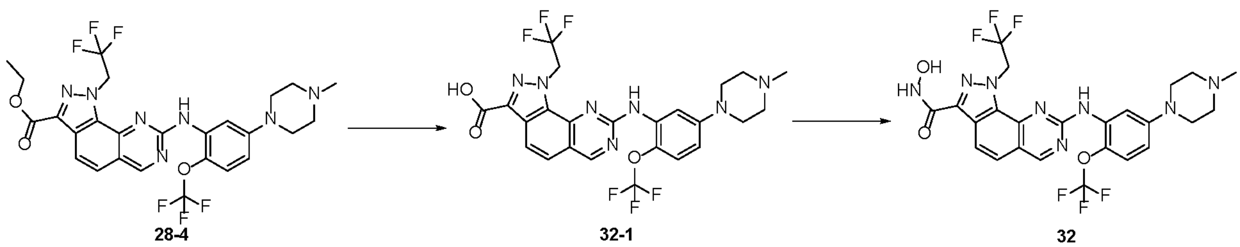

**28-4** **32-1** **32**

30.1 Synthesis of intermediate **32-1**

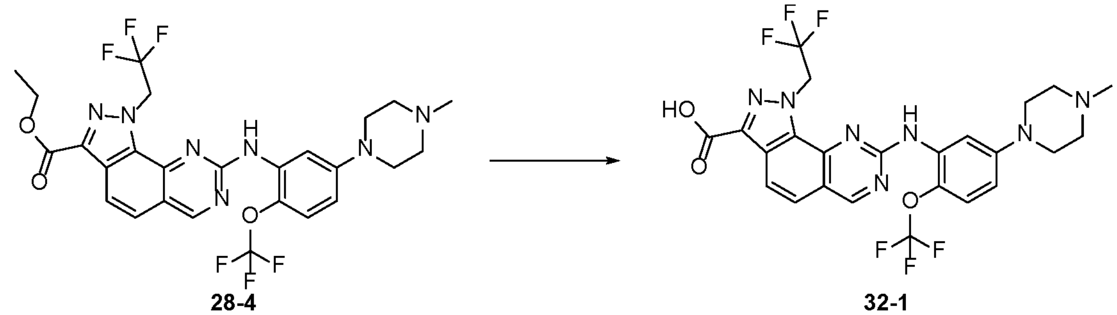

28-4 32-1

Intermediate **28-4** (50.0 mg, 83.7 μmol) was dissolved in methanol (5 mL), and lithium hydroxide aqueous solution (1.00 M, 418 μL) was added. The mixture was reacted at room temperature for 2 h. The reaction liquid was adjusted to acidic pH with hydrochloric acid, and concentrated under reduced pressure, and the residue was purified by preparative liquid chromatogram (C18 30* 150 mm*7 μm; mobile phase: [water (TFA)-acetonitrile]; B%: 20%-50%, 10 min). The intermediate **32-1** (40.0 mg, yield 83.8%, yellow solid) was obtained. MS (ESI) m/z = 570.1 $[M+H]^+$.

30.2 Synthesis of compound **32**

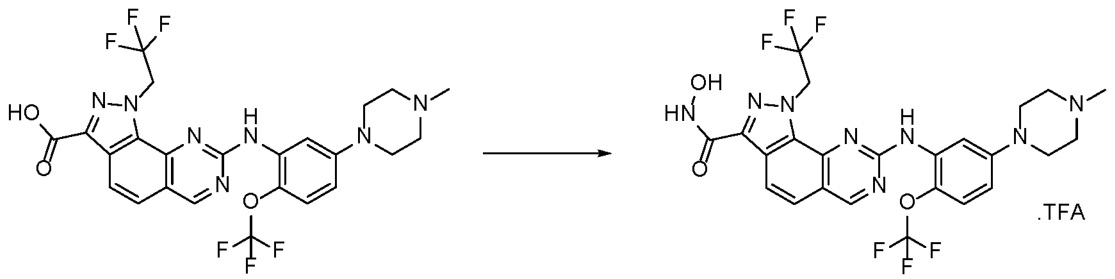

32-1 32

Intermediate **32-1** (40.0 mg, 70.2 μmol), HATU (29.4 mg, 77.1 μmol), DIEA (18.2 mg, 140 μmol), and $NH_2OH$ (9.76 mg , 140 μmol) were dissolved in DMF (2 mL) and the mixture was reacted at room temperature for 2 h. The reaction liquid was concentrated under reduced pressure, and the residue was purified by preparative liquid chromatogram (C18 30*150 mm*7 μm; mobile phase: [water (TFA)-acetonitrile]; B%: 10%-50%, 12 min). Compound **32** (5.0 mg, yield 12.2%, brown solid) was obtained. MS (ESI) m/z = 585.3 $[M+H]^+$.

**Example 31: Synthesis of compound 33**

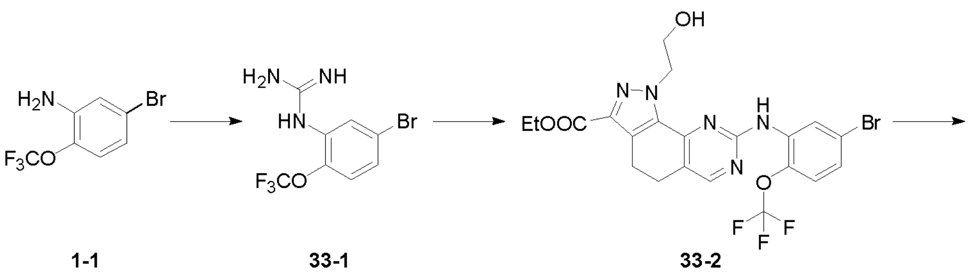

1-1 33-1 33-2

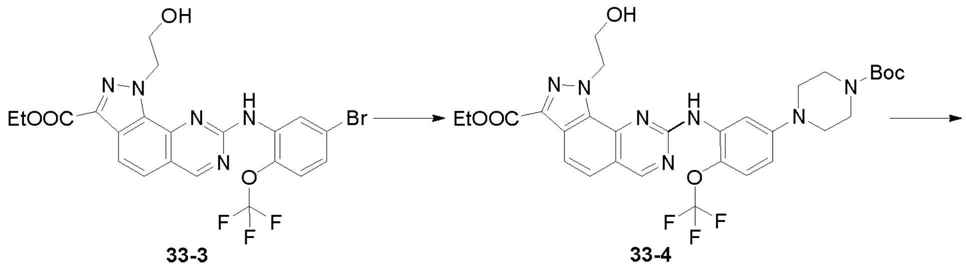

33-3 33-4

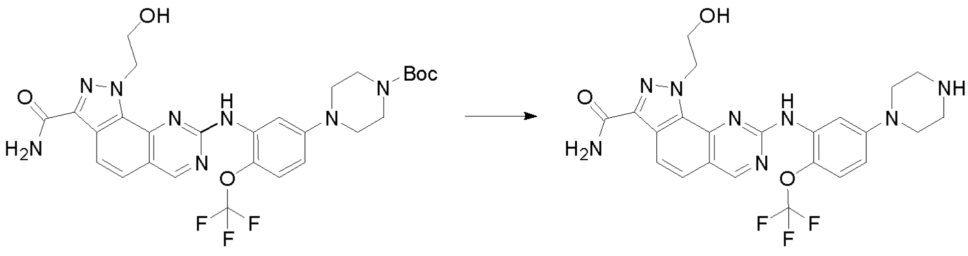

33-5 33

31.1 Synthesis of intermediate **33-1**

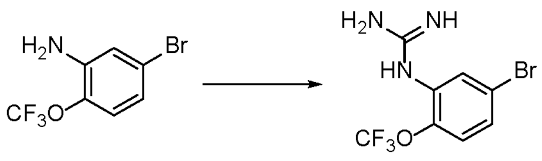

1-1 33-1

Compound **1-1** (13.0 g, 50.8 mmol) was dissolved in water (130 mL), and concentrated hydrochloric acid (12 mL) and cyanamide (10.6 g, 254 mmol) were added. The mixture was reacted at 60°C for 16 h. The mixture was adjusted to pH = 14 with 40% sodium hydroxide solution, and extracted with ethyl acetate (150 mL × 3). The organic phases were combined, dried over anhydrous sodium sulfate, filtered, and the organic phase was concentrated under reduced pressure. The intermediate **33-1** (10.9 g, yield 72.2%, brown solid) was obtained. MS (ESI) m/z = 297.9 $[M+H]^+$.

31.2 Synthesis of intermediate **33-2**

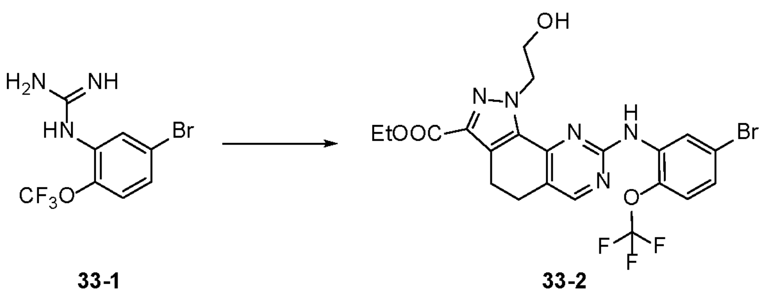

33-1 33-2

Intermediate **33-1** (5.00 g, 16.8 mmol) was dissolved in DMF (50 mL), intermediate **1-8** (5.16 g, 16.8 mmol) was added, and the mixture was reacted at 110°C for 16 h. Water (200 mL) was added to quench the reaction, and ethyl acetate (100 mL × 3) was added for extraction. The organic phases were combined, dried over anhydrous sodium sulfate, filtered, and the organic phase was concentrated under reduced pressure. The residue was subjected to silica gel column chromatography (ethyl acetate/petroleum ether = 0%-100%) to obtain intermediate **33-2** (2.96 g, yield 32.5%, yellow solid). MS (ESI) m/z = 542.3 $[M+H]^+$.

31.3 Synthesis of intermediate **33-3**

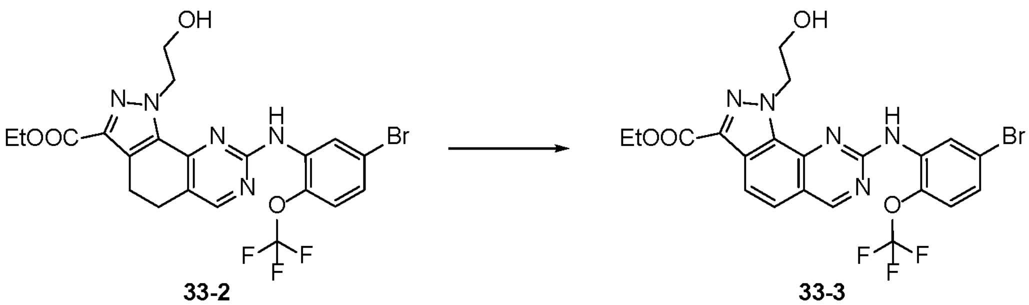

33-2 33-3

Intermediate **33-2** (2.55 g, 4.70 mmol) and DDQ (2.35 g, 10.34 mmol) were dissolved in dioxane (60 mL) and the mixture was reacted at 100°C for 9 h. The resulting product was filtered, the filtrate was collected, and concentrated under reduced pressure to obtain intermediate **33-3** (2 g, crude product), which was directly used in the next step. MS (ESI) m/z = 540.0 $[M+H]^+$.

31.4 Synthesis of intermediate **33-4**

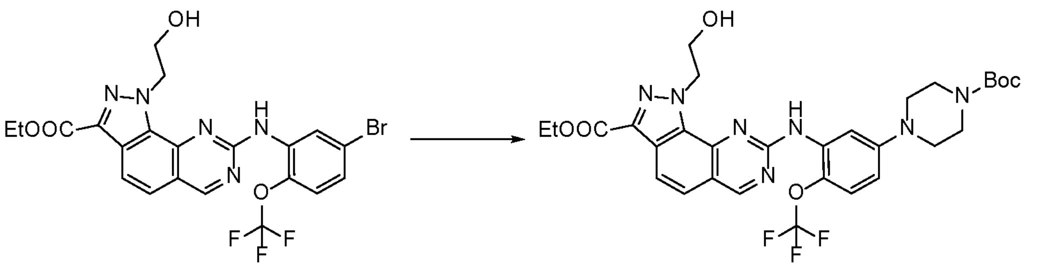

33-3 33-4

Intermediate **33-3** (1.20 g, 1.49 mmol, purity 67%) and acetate salt of tert-butyl piperazine-1-carboxylate (1.39 g, 7.44 mmol) were dissolved in dioxane ( 6 mL), XPhos (354 mg, 744 μmol), KOtBu (334 mg, 2.98 mmol) and $Pd_2(dba)_3$ (341 mg, 372 μmol) were added. The mixture was reacted at 75°C for 20 h. Water (100 mL) was added to quench the reaction, and ethyl acetate (100 mL × 3) was added for extraction. The organic phases were combined, dried over anhydrous sodium sulfate, filtered, and the organic phase was concentrated under reduced pressure. The residue was

purified by preparative liquid chromatogram (C18 30* 150 mm*7 μm; mobile phase: [water ($NH_3H_2O$+$NH_4HCO_3$)-acetonitrile]; B%: 30%-70%, 20 min). The intermediate 33-4 (0.12 g, yield 12.5%, brown solid) was obtained. MS (ESI) m/z = 646.2 $[M+H]^+$.

31.5 Synthesis of intermediate **33-5**

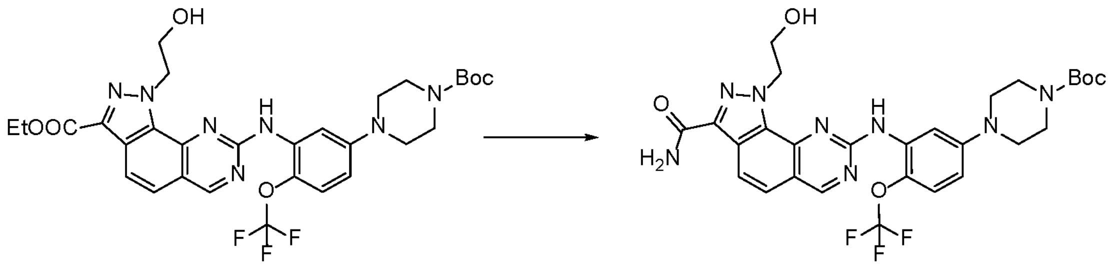

33-4 33-5

Intermediate **33-4** (90.0 mg, 139 μmol) was dissolved in $NH_3$/MeOH (10 mL, 7 M) and the mixture was reacted at 80°C for 48 h. After cooling to room temperature, the reaction liquid was concentrated under reduced pressure to obtain intermediate **33-5** (100 mg, crude product), which was directly used in the next step. MS (ESI) m/z = 617.4 $[M+H]^+$.

31.6 Synthesis of compound **33**

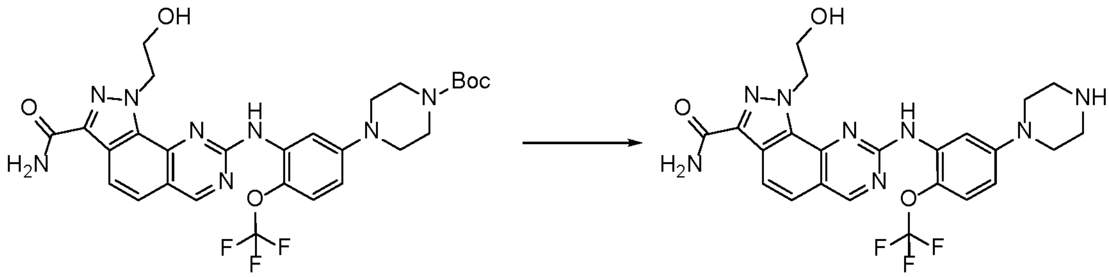

33-5 33

Intermediate **33-5** (90.0 mg, 124 μmol, 85% purity) was dissolved in dichloromethane (5 mL), TFA (212 mg, 1.86 mmol) was added, and the mixture was reacted at room temperature for 4 h. The reaction liquid was concentrated under reduced pressure, and the residue was purified by preparative liquid chromatogram (C18 30* 150 mm*7 μm; mobile phase: [water (TFA)-acetonitrile]; B%: 4%-44%, 36 min). The prepared freeze-dried product was dissolved in water and acetonitrile (volume ratio 1 : 1), a solid was precipitated, filtered by suction, and dried. Compound **33** (30.3 mg, yield 57.2%, yellow solid) was obtained. MS (ESI) m/z = 517.4 $[M+H]^+$. $^1$H NMR (400 MHz, DMSO-d6) δ 9.44 (s, 1 H), 9.31 (s, 1 H), 8.00 (d, *J* = 8.8 Hz, 1 H), 7.74 (br s, 1 H), 7.58 (d, *J* = 8.8 Hz, 1 H), 7.44 (br s, 1 H), 7.19 - 7.30 (m, 2 H), 6.85 (dd, *J* = 9.2, 2.8 Hz, 1 H), 4.89 (t, *J*= 4.8 Hz, 2 H), 4.59 (br s, 1 H), 3.64 - 3.73 (m, 2 H), 3.00 - 3.15 (m, 4 H), 2.76 - 2.90 (m, 4 H).

**Test example 1. Inhibitory activity on PLK1 kinase**

1. Test materials: PLK1 kinase (Carnabio #05-157)

Detection solution PerkinElmer #TRF0218-M
Detection instrument PerkinElmer EnVision

2. Test method:

a. Using the Echo machine, the compounds to be tested were diluted by 1 : 3 on a 384-well plate, and the highest concentration was 1 μM, with a total of 10 concentration points, and each concentration was tested in duplicate wells.
b. 5 μL/well of PLK1 kinase and peptide substrate mixture were added to the assay plate (containing controls). Each assay plate was set with GSK461364 as a positive control and DMSO solvent group as a negative control.
c. The assay plate was centrifuged at 1000 rpm for approximately 15 seconds and incubated at 23°C for 15 min.
d. The reaction was initiated by adding 5 μL/well of ATP solution;
e. The assay plate was centrifuged at 1000 rpm for about 15 seconds, a film was used to seal the assay plate which was incubated at 23°C for a period of time;
f. A detection solution was added to all wells of the assay plate;
g. The assay plate was centrifuged at 1000 rpm for approximately 15 seconds, a film was used to seal the assay plate which was incubated at 23°C for 60 min.
h. The assay plate was read on EnVision. The test results were analyzed using XLFIT5 software.

3. Test results: The $IC_{50}$ and $I_{max}$ (%) for the inhibitory activity of the compound of the present invention on PLK1 kinase are shown in Table 1. $I_{max}$ is the maximum effect (S/B 8.52, Z factor 0.94) caused by the compound at the test concentration.

Table 1 Inhibitory test results on PLK1 kinase

| Compound No. | $IC_{50}$ (nM) | $I_{max}$ (%) |
|---|---|---|
| GSK461364[1] | 0.84 | 101.08 |
| Onvansertib[2] | 1.08 | 100.02 |
| 1 | 1.81 | 99.35 |
| 2 | 0.99 | 99.81 |
| 3 | 0.714 | 99.57 |
| 4 | 1.64 | 99.51 |
| 5 | 73.29 | 76.81 |
| 7 | 0.79 | 100.68 |
| 8 | 14.7 | 95.96 |
| 9 | 327.07 | 72.68 |
| 10 | 1.16 | 99.92 |
| 11 | 1.80 | 99.65 |
| 12 | 2.76 | 99.53 |
| 13 | 2.49 | 99.41 |
| 16 | 4.77 | 98.76 |
| 17 | 0.814 | 99.97 |
| 18 | 0.661 | 100.19 |
| 19 | 1.06 | 99.97 |
| 20 | 1.28 | 99.29 |
| 21 | 0.756 | 99.15 |
| 22 | 1.51 | 99.99 |
| 23 | 3.66 | 98.18 |
| 24 | 1.21 | 99.48 |
| 25 | 1.12 | 100.23 |
| 27 | 0.459 | 100.24 |
| 28 | 0.670 | 100.08 |
| 29 | 0.805 | 100.08 |
| 30 | 0.776 | 99.97 |
| 31 | 1.55 | 99.54 |
| 32 | 0.463 | 99.87 |

1. The compound prepared and disclosed in Example 3 of CN 101300251 B;
2. The compound prepared and disclosed in Example 38 of CN 101563351 B.

Conclusion: The $IC_{50}$ of most compounds of the present invention in inhibiting PLK1 kinase is at the level of 1-2 nM, which is equivalent to the control drug onvansertib. The kinase activity of compounds 3, 7, 17, 18, 21, 27-30, and 32 is higher than that of the control drug.

**Test example 2. Inhibitory activity on HCT116 cell proliferation**

1. Test materials:

Cell lines: HCT116 cells
Cell culture medium: RPMI1640 + 10% FBS (GBICO)
Cell culture plate: 96-well plate (Shanghai Jingan Biotechnology Co., Ltd.)
Detection kit: ATPlite 1step Luminescence (PerkinElmer)
Detection instrument: BioTek multifunctional microplate reader

2. Test method: HCT116 cells in the logarithmic growth phase were seeded into a white-wall clear-bottom 96-well plate at a density of 720 cells per well, and cultured overnight in a 37°C, 5% $CO_2$ incubator. The next day, a compound to be tested was added to the cells, wherein the test compound had a concentration of 10 μM and was subjected to 3-fold gradient dilution to obtain 9 concentrations, and double replicate wells were set up for each concentration. The blank culture medium was used as a negative control, and the same concentration of Onvansertib was used as a positive control. After adding the drug, culturing was continued for 72 h in a 37°C, 5% $CO_2$ incubator. ATPlite 1step Luminescence reagent with the same volume as the cell fluid was added to the cells, incubated in the dark for 3 min at room temperature, and oscillated for 2 min with a micro-oscillator at 500 r, and the luminescent intensity was detected by a microplate reader to calculate the cell inhibition rate.

Cell inhibition rate (%) = $[100- (Lum_{\text{sample to be tested}}-Lum_{\text{culture solution}})/ (Lum_{\text{negative control}}-Lum_{\text{culture solution}}) \times 100]\%$.

GraphPad Prism 7.0 was used to process the data, the cell inhibition rate curve was obtained and $IC_{50}$ was calculated.

3. Test results:

The half inhibitory concentration $IC_{50}$ of the compound of the present invention on HCT116 cell proliferation is shown in Table 2.

Table 2 $IC_{50}$ of the compound of the present invention on HCT116 cells

| Compound No. | $IC_{50}$ (μM) |
|---|---|
| Onvansertib[1] | 0.042 |
| 1 | 0.051 |
| 2 | 0.027 |
| 3 | 0.093 |
| 4 | 0.332 |
| 5 | 0.924 |
| 6 | 2.724 |
| 8 | 3.401 |
| 9 | > 10* |
| 10 | 0.049 |
| 11 | 0.205 |
| 12 | 0.861 |
| 13 | 0.831 |
| 16 | 0.502 |
| 18 | 0.005 |
| 19 | 0.096 |
| 20 | 0.143 |
| 23 | 0.214 |
| 24 | 0.032 |

(continued)

| Compound No. | $IC_{50}$ (μM) |
|---|---|
| 29 | 0.077 |

1. The compound prepared and disclosed in Example 38 of CN 101563351 B.
* indicates that the inhibitory effect of the compound is less than 50% at 10 μM.

Conclusion: The half inhibitory concentration of compound 1 of the present invention on HCT116 cells is 51 nM, and the half inhibitory concentration of compound 10 on HCT116 cells is 49 nM, which are equivalent to the control Onvansertib. The half inhibitory concentration of compound 2 on HCT116 cells is 27 nM, the half inhibitory concentration of compound 24 on HCT116 cells is 32 nM, and the half inhibitory concentration of compound 18 on HCT116 cells is 5 nM. Compared to the control drug Onvansertib, the activity is enhanced.

**Test example 3. Evaluation of permeability of test substance and transporter substrate**

1. Test materials:

In this experiment, Caco-2 cells were seeded in Transwell-96 well plates at a seeding density of $1 \times 10^5$ cells/$cm^2$. The cells were cultured in a carbon dioxide incubator for 21-28 days before being used for transport experiments. During this period, the culture medium was changed every four to five days.

2. Test method:

This project uses Hank's balanced salt buffer (pH 7.40 ± 0.05) containing 10 mM HEPES as the transport buffer. The culture medium was removed from the culture plate and the cells were washed twice with preheated transport buffer (approximately 75 μL and 40 mL of transport buffer for each apical well and basolateral receiver plate respectively). The donor solution and receiver solution were added to the corresponding cell plate wells (75 and 250 μL for each apical and basolateral well respectively), and the bidirectional transport experiment was started (apical to basolateral (A-B) and basolateral to apical (B-A)).
The test concentration of the test substance was 5.00 μM, and two replicate wells were made for each dosing concentration. The test concentration of Digoxin was 10.0 μM, with dosing bidirectionally; the test concentrations of nadolol and metoprolol were both 2.00 μM, and they were dosed in one direction (A-B direction), and two replicate wells were made for each of the three control compounds. After adding the sample, the cell plate was incubated at 37 ± 1°C, 5% $CO_2$ and saturated humidity for 120 min.
The initial donor solution is the T0 sample. After adding the sample, it was mixed with the transport buffer and stop solution in a certain proportion. After incubation for 120 min, the final samples were collected from the donor side and receiver side, and were also mixed with transport buffer and stop solution in a certain proportion.
All samples were vortexed, and then centrifuged at 3220 × g for 20 min at 20°C. A proper volume of supernatant was transferred to a sample assay plate, and the sample was stored at 2-8°C if it was not analyzed immediately after the plate was sealed, and LC MS/MS method was used for analysis.
The Lucifer Yellow Rejection Assay was used to test the integrity of the Caco-2 cell layer.

3. Data analysis:

The following formulas were used to calculate the apparent permeability coefficient ($P_{app}$, cm/s), efflux ratio (ER), and solution recovery (%) (%Solution Recovery).

$$P_{app} = \frac{V_R}{Area \times Time} \times \frac{[drug]_{recevier}}{[drug]_{initial,\, donor}} = \frac{V_R}{Area \times Time} \times \frac{C_R}{C_0}$$

$$ER = \frac{P_{app}(B-A)}{P_{app}(A-B)}$$

$$\%Solution\ Recovery = \frac{C_R \times V_R + C_D \times V_D}{C_0 \times V_D} \times 100$$

$V_R$ is the volume of the receiver side solution (side A is 0.075 mL, side B is 0.25 mL); Area is the relative surface area of the cell monolayer (0.0804 $cm^2$); Time is the incubation time (7200 s); $C_0$ is the initial concentration (nM) of the test substance at the donor side or the peak area ratio of the control compound; $V_D$ is the volume of the donor side (side A is 0.075 mL, side B is 0.25 mL); $C_D$ and $C_R$ are the final concentration (nM) of the test substance at the donor side and the receiver side respectively or the peak area ratio of the control compound;
Lucifer yellow transmittance rate (%Lucifer Yellow) is calculated using the following formula:

$$\%\ Lucifer\ Yellow = \frac{V_{Basolateral} \times RFU_{Basolateral}}{V_{Apical} \times RFU_{Apical} + V_{Basolateral} \times RFU_{Basolateral}} \times 100$$

RFUApical and RFUBasolateral are the relative fluorescence intensity of lucifer yellow at the apical and basolateral sides respectively. VApical and VBasolateral are the sample loading volumes at the apical and basolateral sides respectively (0.075 and 0.25 mL respectively).

4. Classification criteria:

| Parameter | Classification | Standard |
|---|---|---|
| Permeability | Low | $P_{app}$ (A -B) ≤ 0.500 (× $10^{-6}$ cm/s) |
| | Medium | 0.500 < $P_{app}$ (A-B) < 2.50 (× $10^{-6}$ cm/s) |
| | High | $P_{app}$ (A-B) ≥ 2.50 (× $10^{-6}$ cm/s) |
| Efflux transporter substrate | Likely | $ER_a$ ≥ 2.00 |
| | Very unlikely or not | $ER_a$ < 2.00 |

5. Test results: The permeability results of the compounds of the present invention in Caco-2 cells are shown in Table 5.

Table 5. Permeability results in Caco-2 cells

| Compound No. | Average $P_{app}$ ($10^{-6}$ cm/s) | | Efflux | Classification | |
|---|---|---|---|---|---|
| | A-B | B-A | ratio | Permeability | Efflux transporter substrate |
| Onvansertib | 0.299 | 43.6 | 146 | Low | Likely |
| 1 | 0.965 | 20.8 | 21.5 | Medium | Likely |
| 2 | < 0.480 | 26.1 | > 54.4 | Low | Likely |
| 3 | 0.644 | 0.851 | 1.32 | Medium | Very unlikely or not |
| 4 | 1.16 | 25.5 | 22.1 | Medium | Likely |
| 7 | 0.275 | 0.542 | 1.97 | Low | Very unlikely or not |
| 8 | < 0.480 | 14.5 | > 30.3 | Low | Likely |
| 9 | < 0.254 | 6.43 | > 25.4 | Low | Likely |
| 10 | < 0.480 | 24.8 | > 51.7 | Low | Likely |
| 11 | 1.13 | 25.0 | 22.1 | Medium | Likely |
| 12 | < 0.264 | 15.0 | > 56.7 | Low | Likely |
| 13 | < 0.480 | 13.7 | > 28.6 | Low | Likely |
| 16 | 0.917 | 6.66 | 7.27 | Medium | Likely |
| 18 | 2.03 | 3.36 | 1.65 | Medium | Very unlikely or not |
| 20 | 1.30 | 13.4 | 10.4 | Medium | Likely |
| 23 | < 0.280 | 23.1 | > 82.6 | Low | Likely |

(continued)

| Compound No. | Average $P_{app}$ ($10^{-6}$ cm/s) | | Efflux | Classification | |
|---|---|---|---|---|---|
| | A-B | B-A | ratio | Permeability | Efflux transporter substrate |
| 24 | 0.106 | 29.5 | 279 | Low | Likely |
| Conclusion: The compounds of the present invention are low or moderately permeable in Caco-2 cells. | | | | | |

**Test example 4. Evaluation of metabolic stability of liver microsomes**

1. Reagents:

| Name | Content | Batch No. | Source |
|---|---|---|---|
| Tetrasodium salt of nicotinamide adenine dinucleotide phosphate (NADPH) | 98% | 50650124 | Rocheroche Diagnostics GmbH |
| Phosphate buffered saline | / | SLBX1022 | SIGMA |
| Magnesium chloride hexahydrate ($MgCl_2 \cdot 6H_2O$) | ≥ 99.0% | BCBW0417 | SIGMA-ALDRICH |
| Formic acid (FA) | ≥ 96.0% | SHBJ0951 | SIGMA-ALDRICH |
| Acetonitrile | / | JA104130 | Merck KGaA |
| Methanol | / | I10099407026 | Merck KGaA |
| Ultra-pure water | / | / | Prepared from MilliQ water machine |

2. Equipment

| Name | Model | Manufacturer |
|---|---|---|
| Chromatographic column Agilent XDB C18 | 2.1 × 150 mm, 3.5 micro | Agilent |
| Quadrupole high-resolution mass spectrometer | Q Exactive | Thermoscientific |
| Ultra high performance liquid chromatograph | HCLass | Waters |
| Electric heating constant temperature oscillating water tank | DKZ | Shanghai Yiheng Technology Co., Ltd. |
| High speed desktop centrifuge | XIR | Thermo |
| Centrifuge | Legend Micro 21R | Thermo |
| Ultrapure water instrument | Advantage A10 | Millipore |
| Electronic balance | XS105 | METTLER TOLEDO |

3. Test method

a. Preparation of phosphate buffer: The phosphate buffer powder was dissolved in 100 mL of pure water to prepare a phosphate buffer solution with a pH of 7.4 and a concentration of 100 mM, which was stored in a 4°C refrigerator for later use.
b. All samples as well as the reference testosterone were dissolved in DMSO to obtain a 10 mM stock solution. The 10 mM stock solution was diluted to 100 μM with acetonitrile to obtain a working solution for later use.
c. Preparation of liver microsome working solution: Each of the mouse, rat and human liver microsome stock solutions (at concentration of 20 mg/mL) was taken and diluted to 0.56 mg/mL with 100 mM phosphate buffer solution to obtain the liver microsome working solution.
d. Preparation of solution: An appropriate amount of MgCl2 was weighed and used to prepare a MgCl2 solution at a concentration of 60 mM using 100 mM phosphate buffer solution. An appropriate amount of NADPH was weighed and used to prepare a NADPH solution at a concentration of 20 mM using 100 mM phosphate buffer solution. An

equal volume of 60 mM $MgCl_2$ solution was added to prepare an NADPH working solution containing 10 mM NADPH and 30 mM $MgCl_2$. An acetonitrile solution containing 20 ng/mL tolbutamide was prepared as the stop solution.

e. Liver microsome incubation: 2 μL of each compound and the positive drug testosterone working solution were added to 178 μL of liver microsome working solution (0.56 mg/mL), mixed gently, and pre-incubated in a vibrating constant temperature water bath pot at 37°C for 10 min. After adding 20 μL of NADPH working solution, the mixture was placed in a vibrating constant temperature water bath pot at 37°C and timed incubation was started for 5, 10, 20, 30 and 60 min.

f. Stopping reaction: After the corresponding time of incubation, 400 μL of stop solution was added to stop the reaction, and then the sample was immediately shaken for 1 min. The sample was centrifuged at 13500 rpm for 10 min at 10°C. After centrifugation, the supernatant was removed, diluted with water according to the needs of each compound, and analyzed by LC-MS. For the 0-minute sample, the stop solution was first added and mixed evenly with the liver microsomes, and then the NADPH working solution was added. For the negative control sample, 20 μL of 30 mM MgCl2solution was added instead of the NADPH working solution.

4. Data analysis:

$T_{1/2}$ and $CL_{int(mic)}$ were calculated by the following formula.

$$C_t = C_0 * e^{-k_e \cdot t}$$

When $C_t = 1/2C_0$, $T_{1/2} = \ln 2/k_e = 0.693/k_e$

$$CL_{int(mic)} = 0.693/T_{1/2}/\text{liver microsomal protein concentration during incubation (mg/mL)}$$

$$CL_{int(liver)} = CL_{int(mic)} * \text{liver microsomal protein (mg)/liver weight (g)} * \text{liver weight to body weight ratio}$$

Liver microsomal protein (mg)/liver weight (g): the value is 45 in both animal and human species.

Liver weight to body weight ratio: the parameters for mice, rats and humans are 88, 40 and 20 g/kg respectively.

5. Test results: The metabolic stability results of the compounds of the present invention in mouse, rat and human liver microsomes are shown in Table 6.

Table 6 Metabolic stability results in mouse, rat and human liver microsomes

| Compound | Species | 60min remaining amount % | $T_{1/2}$ min | $CL_{int(mic)}$ [μL/min/mg protein | $CL_{int(liver)}$ mL/min/kg | Clearance rate |
|---|---|---|---|---|---|---|
| Onvansertib | Mouse | 21.5 | 27.2 | 51.0 | 202 | Medium |
| | Rat | 15.7 | 22.7 | 61.0 | 110 | Medium |
| | Human | 64.8 | 81.5 | 17.0 | 15.3 | Medium |
| 1 | Mouse | 19.2 | 26.7 | 52.0 | 206 | Medium |
| | Rat | 3.06 | 12.2 | 113 | 204 | High |
| | Human | 26.0 | 30.8 | 45.0 | 40.5 | Medium |
| 2 | Mouse | 26.7 | 31.4 | 44.2 | 175 | Medium |
| | Rat | 17.0 | 24.3 | 57.0 | 103 | Medium |
| | Human | 49.9 | 55.0 | 25.2 | 22.7 | Medium |
| 3 | Mouse | 4.32 | 13.4 | 103 | 409 | High |
| | Rat | 2.74 | 4.94 | 281 | 505 | High |
| | Human | 7.83 | 15.9 | 87.4 | 78.7 | High |
| 4 | Mouse | 1.33 | 4.47 | 310 | 1229 | High |
| | Rat | 2.76 | 11.8 | 117 | 211 | High |
| | Human | 23.7 | 28.3 | 49.0 | 44.1 | Medium |

(continued)

| Compound | Species | 60min remaining amount % | $T_{1/2}$ min | $CL_{int(mic)}$ [μL/min/mg protein | $CL_{int(liver)}$ mL/min/kg | Clearance rate |
|---|---|---|---|---|---|---|
| 7 | Mouse | 2.92 | 5.84 | 237 | 940 | High |
| | Rat | 2.06 | 2.30 | 603 | 1086 | High |
| | Human | 14.1 | 20.6 | 67.4 | 60.7 | High |
| 12 | Mouse | 26.9 | 33.6 | 41.2 | 163 | Medium |
| | Rat | 7.43 | 16.3 | 85.0 | 153 | High |
| | Human | 70.8 | 119 | 11.6 | 10.4 | Medium |
| 13 | Mouse | 38.6 | 44.7 | 31.0 | 123 | Medium |
| | Rat | 27.8 | 33.5 | 41.4 | 74.5 | Medium |
| | Human | 58.5 | 71.4 | 19.4 | 17.5 | Medium |
| 16 | Mouse | 24.6 | 29.9 | 46.4 | 184 | Medium |
| | Rat | 21.6 | 27.7 | 50.5 | 90.0 | Medium |
| | Human | 23.3 | 28.3 | 49.0 | 44.1 | Medium |
| 17 | Mouse | 2.46 | 11.7 | 119 | 470 | High |
| | Rat | 1.93 | 6.08 | 228 | 410 | High |
| | Human | 26.0 | 29.9 | 46.4 | 41.8 | Medium |
| 18 | Mouse | 4.32 | 13.4 | 103 | 409 | High |
| | Rat | 2.74 | 4.94 | 281 | 505 | High |
| | Human | 7.83 | 15.9 | 87.4 | 78.7 | High |
| 19 | Mouse | 22.0 | 28.5 | 48.6 | 192 | Medium |
| | Rat | 11.9 | 19.6 | 70.8 | 127 | Medium |
| | Human | 51.9 | 63.6 | 21.8 | 19.6 | Medium |
| 20 | Mouse | 15.9 | 23.1 | 600 | 238 | High |
| | Rat | 21.6 | 26.2 | 53.0 | 95.4 | Medium |
| | Human | 26.5 | 31.9 | 43.4 | 39.1 | Medium |
| 24 | Mouse | 58.2 | 73.7 | 18.8 | 74.4 | Medium |
| | Rat | 47.9 | 53.7 | 25.8 | 46.4 | Medium |
| | Human | 40.1 | 43.6 | 31.8 | 28.6 | Medium |

**Test example 5. In vivo pharmacodynamic study of compound 1 in mice**

1. Test materials:

Balb/C Nude mice, SPF grade, male, weighing 18-20 g, were purchased from Jiangsu Gempharmatech Co., Ltd.
HCT116 cells were purchased from ATCC.
Matrigel Matrix, Cat. No. 354248, was purchased from Becton, Dickinson and Company.
Kolliphor® HS 15, Cat. No. 42966, was purchased from Sigma-Aldrich.

2. Test method:

a. Establishment of tumor-bearing mouse model: HCT116 cells in the logarithmic growth phase were taken and cultured, the cells were collected and counted, and then subcutaneously inoculated into Balb/C Nude mice at 1 × 106/mouse. The tumor volume and body weight of the mice were measured regularly. 7 days after inoculation, mice with tumor volume of 150 mm3 were randomly divided into groups and treated with corresponding drugs.
b. Animal grouping and administration: All mice were randomly divided into groups: 6 in the negative control group

(sham group, 20% Solutol HS15), 6 in the onvansertib alone administration group (ONV-20 mg/kg group), and 6 in the compound 1 alone administration group (T1- 20 mg/kg group). The administration was performed once a day by gavage for 21 consecutive days, and the tumor volume and body weight of the mice were measured regularly.

3. Data processing:

All data were organized in EXCEL, and One-way ANOVA analysis and drawing were performed using GraphPad Prism 8.0 software. Statistical significance was achieved when p < 0.05.

$$\text{Tumor volume(V)} = 0.5 \times \text{Major axis} \times \text{Minor axis}^2$$

$$\text{Tumor inhibition rate(TGI, \%)} = \frac{\text{Average tumor volume in negative control group} - \text{Average tumor volume in drug treatment group}}{\text{Average tumor volume in negative control group}} \times 100\%$$

4. Test results:

As shown in Fig. 1, when mice are treated with drugs for 11 days, compared to the sham group, the tumor inhibition rate of the T1-20 mg/kg group reaches 82.0% ($p < 0.0001$), and the tumor inhibition rate in the ONV-20 mg/kg group is 54.9% ($p < 0.05$). After continuous administration for 16 days, compared to the sham group, the tumor inhibition rate of the T1-20 mg/kg group reaches 86.5% ($p < 0.001$), and the tumor inhibition rate in the ONV-20 mg/kg group is 39.6% ($p < 0.05$), and there is a significant difference for the data of the T1-20 mg/kg and ONV-20 mg/kg group ($p < 0.01$). After continuous administration for 21 days, compared to the sham group, the tumor inhibition rate of the T1-20 mg/kg group reaches 88.3% ($p < 0.0001$), and the tumor inhibition rate in the ONV-20 mg/kg group is 37.0% ($p < 0.05$), and there is a significant difference for the data of the T1-20 mg/kg and ONV-20 mg/kg group ($p < 0.01$).

As shown in Fig. 2, during the test period, there is no significant difference in the body weight of animals in each group at each measurement.

5. Test conclusion: Compound 1 of the present invention has a significant inhibitory effect on the volume growth of colorectal tumors, and its efficacy at the same dose is better than that of the control drug onvansertib.

**Test example 6. In vivo pharmacodynamic study of compound 18 in mice**

1. Experiment materials: Same as Test example 5.
2. Experimental method:

   a. Establishment of tumor-bearing mouse model: Same as Test example 5.
   b. Animal grouping and administration: All mice were randomly divided into groups: 7 in the negative control group (control group, 20% Solutol HS15), 7 in the onvansertib alone administration group (onv-60 mg/kg group), and 7 in the compound 18 alone administration group (T18- 10 mg/kg group). The administration was performed once a day by gavage for 7 consecutive days, and the tumor volume and body weight of the mice were measured regularly.

3. Data processing: Same as Test example 5.
4. Test results:
As shown in Fig. 3, when mice are treated with drugs for 7 days, compared to the control group, the T18-10 mg/kg group can significantly inhibit mouse tumor growth, with an inhibition rate of 70.9% ($p < 0.001$), the tumor inhibition rate in the onv-60 mg/kg group is 62.7% ($p < 0.01$).

As shown in Fig. 4, after continuous administration for 5 days, the body weight of the mice in the T18-10 mg/kg group decreased by 7.6% compared to that before the administration, and the body weight of the mice in the onv-60 mg/kg group decreased by 8.4% ($p < 0.05$) compared to that before the administration. After continuous administration for 7 days, the body weight of the mice in the T18-10 mg/kg group decreased by 16.1 % ($p < 0.01$) compared to that before the administration, and the body weight of the mice in the onv-60 mg/kg group decreased by 19.5% ($p < 0.001$) compared to that before the administration. 5. Test conclusion: Compound 18 of the present invention has a significant inhibitory effect on tumor volume growth in mice when administered continuously for 7 days at a dose of 10 mg/kg. To achieve the same efficacy, the required dose of compound 18 is approximately 1/6 of that of the control drug onvansertib,

and its impact on animal body weight is less than that of the control drug.

## Claims

1. A compound of formula (I), or a pharmaceutically acceptable salt or stereoisomer thereof,

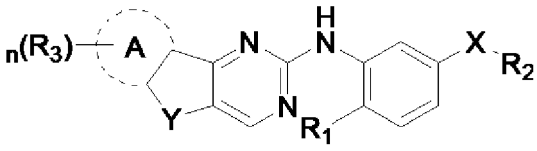

(I)

wherein X is independently selected from: divalent group of piperidine, piperazine, 3,8-diazabicyclo[3.2.1]octane, $-CH_2$-piperazine-, -NR-pyrrolidine-, -NR-azetidine-, or -NR-$CH_2$-pyrrolidine-; the R is independently selected from: H, D, or $C_1$-$C_6$ alkyl;

Y is independently selected from: divalent group of $-C(R_4)_2-C(R_4)_2-$, $-CR_4=CR_4-$, $-C(R_4)_2-NR_4-$, $-NR_4-C(R_4)_2-$, $-CR_4=N-$ or $-N=CR_4-$, each of the $R_4$ is independently selected from: H, D, or Ci-Ce alkyl;

ring A is selected from a structure represented by formula (A):

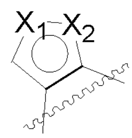

(A) ;

$X_1$ is independently selected from: C, N, O or S atom;

$X_2$ is independently selected from: C, N, O or S atoms;

$R_1$ is independently selected from: H, D, $C_1$-$C_6$ alkyl, $C_1$-$C_6$ haloalkyl, $C_1$-$C_6$ alkoxy, or $C_1$-$C_6$ haloalkoxy;

$R_2$ is independently selected from: H, D, or $C_1$-$C_6$ alkyl, wherein the $C_1$-$C_6$ alkyl is optionally substituted with one or more halogen, OH, $NH_2$, or CN groups;

each $R_3$ is independently selected from: H, D, $C_1$-$C_6$ alkyl, $C_3$-$C_6$ cycloalkyl, or $-C(O)NHR_5$, wherein the $C_1$-$C_6$ alkyl is optionally substituted with one or more halogen, OH, $NH_2$, CN, or $C_1$-$C_6$ alkoxy, the $R_5$ is independently selected from: H, D, $C_1$-$C_6$ alkyl, $C_1$-$C_6$ alkoxy, HO-$C_1$-$C_6$ alkoxy, OH, $NH_2$, $-NHC(O)NH_2$, or $R_6-S(O)_2-$, and the $R_6$ is independently selected from: $C_1$-$C_6$ alkyl, $C_1$-$C_6$ haloalkyl, or $C_3$-$C_6$ cycloalkyl.

2. A compound of formula (II), or a pharmaceutically acceptable salt or stereoisomer thereof,

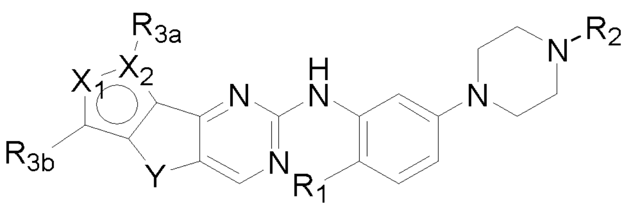

(II)

wherein:

Y is independently selected from: divalent group of $-C(R_4)_2-C(R_4)_2-$, $-CR_4 = CR_4-$, $-C(R_4)_2-NR_4-$, $-NR_4-C(R_4)_2-$, $-CR_4=N-$ or $-N=CR_4-$, each of the $R_4$ is independently selected from: H, D, or Ci-Ce alkyl;

$X_1$ is independently selected from: C, N, O or S atoms;

$X_2$ is independently selected from: C, N, O or S atoms;

$R_1$ is independently selected from: H, D, $C_1$-$C_6$ alkyl, $C_1$-$C_6$ haloalkyl, $C_1$-$C_6$ alkoxy, or $C_1$-$C_6$ haloalkoxy;

$R_2$ is independently selected from: H, D, or $C_1$-$C_6$ alkyl, wherein the $C_1$-$C_6$ alkyl is optionally substituted with

one or more halogen, OH, $NH_2$, or CN group;
$R_{3a}$ is independently selected from: H, D, $C_1$-$C_6$ alkyl, or $C_3$-$C_6$ cycloalkyl, wherein the Ci-Ce alkyl is optionally substituted with one or more halogen, OH, $NH_2$, CN, or $C_1$-$C_6$ alkoxy;
$R_{3b}$ is independently selected from: -C(O)NHR$_5$,
the $R_5$ is independently selected from: H, D, $C_1$-$C_6$ alkyl, $C_1$-$C_6$ alkoxy, HO-$C_1$-$C_6$ alkoxy, OH, $NH_2$, - NHC(O)$NH_2$, or $R_6$-S(O)$_2$-, the $R_6$ is independently selected from: $C_1$-$C_6$ alkyl, $C_1$-$C_6$ haloalkyl, or $C_3$-$C_6$ cycloalkyl.

**3.** A compound of formula (IIa), formula (IIb), formula (IIc), formula (IId) or formula (IIe), or a pharmaceutically acceptable salt or stereoisomer thereof,

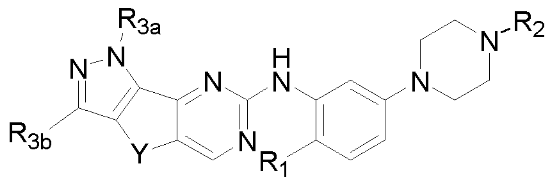
(IIa)
,
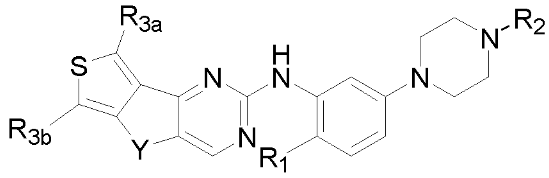
(IIb)
,
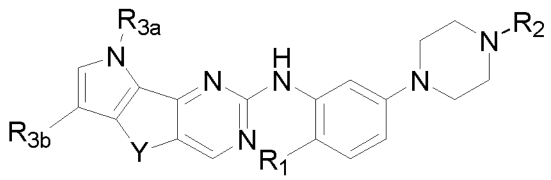
(IIc)
,
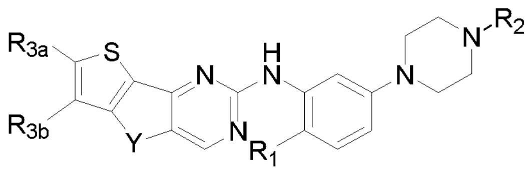
(IId)
,
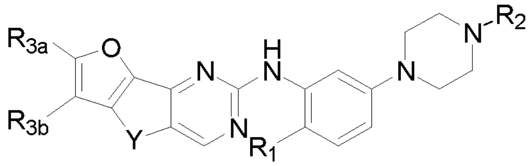
(IIe)
,

wherein:

Y is independently selected from: divalent group of -C($R_4$)$_2$-C($R_4$)$_2$-, -CR$_4$ = CR$_4$-, -C($R_4$)$_2$-NR$_4$-, -NR$_4$-C($R_4$)$_2$-, -CR$_4$=N- or -N=CR$_4$-, each of the $R_4$ is independently selected from: H, D, or Ci-Ce alkyl;
$R_1$ is independently selected from: H, D, $C_1$-$C_6$ alkyl, $C_1$-$C_6$ haloalkyl, $C_1$-$C_6$ alkoxy, or $C_1$-$C_6$ haloalkoxy;
$R_2$ is independently selected from: H, D, $C_1$-$C_6$ alkyl, wherein the $C_1$-$C_6$ alkyl is optionally substituted with one or more halogen, OH, $NH_2$, or CN group;
$R_{3a}$ is independently selected from: H, D, $C_1$-$C_6$ alkyl, $C_3$-$C_6$ cycloalkyl, wherein the $C_1$-$C_6$ alkyl is optionally substituted with one or more halogen, OH, $NH_2$, CN, or $C_1$-$C_6$ alkoxy,
$R_{3b}$ is independently selected from: -C(O)NHR$_5$,
the $R_5$ is independently selected from: H, D, $C_1$-$C_6$ alkyl, $C_1$-$C_6$ alkoxy, HO-$C_1$-$C_6$ alkoxy, OH, $NH_2$, - NHC(O)$NH_2$, or $R_6$-S(O)$_2$-, the $R_6$ is independently selected from: $C_1$-$C_6$ alkyl, $C_1$-$C_6$ haloalkyl, or $C_3$-$C_6$ cycloalkyl.

**4.** The compound, or the pharmaceutically acceptable salt or stereoisomer thereof according to claim 1, wherein

X is preferably independently selected from:

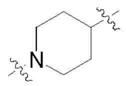, 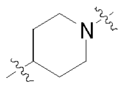, 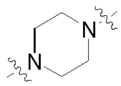, 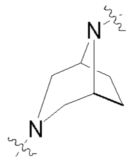, 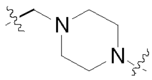,

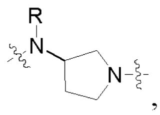, 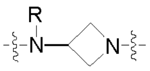, or 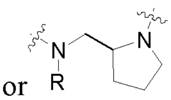;

the R is independently selected from: H, D, methyl, ethyl, n-propyl, or isopropyl;
ring A is preferably selected from a structure represented by formula (A-1), formula (A-2), formula (A-3), formula (A-4) or formula (A-5):

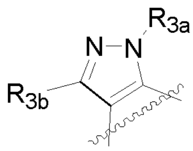
(A-1),

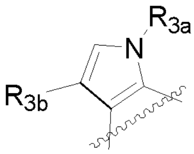
(A-2),

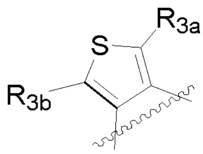
(A-3),

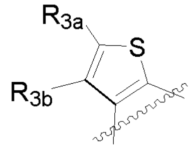
(A-4),

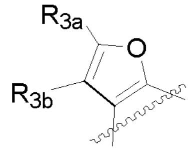
(A-5);

$R_{3a}$ is independently selected from: H, D, $C_1$-$C_6$ alkyl, $C_3$-$C_6$ cycloalkyl, wherein the $C_1$-$C_6$ alkyl is optionally substituted with one or more halogen, OH, $NH_2$, CN, or $C_1$-$C_6$ alkoxy;
$R_{3b}$ is independently selected from: -C(O)NHR$_5$, the $R_5$ is independently selected from: H, D, $C_1$-$C_6$ alkyl, $C_1$-$C_6$ alkoxy, HO-$C_1$-$C_6$ alkoxy, OH, $NH_2$, -NHC(O)$NH_2$, or $R_6$-S(O)$_2$-, the $R_6$ is independently selected from: $C_1$-$C_6$ alkyl, $C_1$-$C_6$ haloalkyl, or $C_3$-$C_6$ cycloalkyl.

**5.** The compound, or the pharmaceutically acceptable salt or stereoisomer thereof according to any one of claims 2-4, wherein, Y is preferably independently selected from: divalent group of -$CH_2$-$CH_2$-, - CH=CH-, -$CH_2$-NH-, -NH-$CH_2$-, -$CH_2$-N($CH_3$)-, -N($CH_3$)-$CH_2$-, -CH=N- or -N=CH-.

**6.** The compound or the pharmaceutically acceptable salt or stereoisomer thereof according to any one of claims 2-4, wherein, $R_1$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, n-propoxy, isopropoxy, n-butoxy, sec-butoxy, tert-butoxy, n-pentyloxy, S-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, or pentachloroethoxy.

**7.** The compound or the pharmaceutically acceptable salt or stereoisomer thereof according to any one of claims 2-4, wherein, $R_2$ is preferably independently selected from: H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl, wherein the terminal position of the methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, or 2-ethylbutyl is optionally substituted with one or more F, Cl, Br, OH, $NH_2$, or CN groups.

**8.** The compound or the pharmaceutically acceptable salt or stereoisomer thereof according to any one of claims 2-4, wherein, $R_{3a}$ is preferably selected from: methyl, ethyl, n-propyl, n-butyl, n-pentyl, n-hexyl, cyclopropyl, cyclobutyl, cyclopentyl or cyclohexyl, wherein the terminal position of the methyl, ethyl, n-propyl, n-butyl, n-pentyl or n-hexyl is optionally substituted with one or more F, Cl, Br, OH, $NH_2$, CN, methoxy or ethoxy.

**9.** The compound or the pharmaceutically acceptable salt or stereoisomer thereof according to any one of claims 2-4,

wherein, $R_{3b}$ is independently selected from: -C(O)NHR$_5$, the $R_5$ is particularly preferably selected from: H, D, methyl, ethyl, n-propyl, n-butyl, n-pentyl, n-hexyl, methoxy, ethoxy, n-propoxy, n-butoxy, n-pentyloxy, n-hexyloxy, HO-methoxy, HO-ethoxy, HO-n-propoxy, HO-n-butoxy, HO-n-pentyloxy, HO-n-hexyloxy, OH, $NH_2$, -NHC(O)$NH_2$, or $R_6$-S(O)$_2$-, and the $R_6$ is independently selected from: methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, cyclopropyl, cyclobutyl, cyclopentyl, or cyclohexyl.

**10.** A compound, or a pharmaceutically acceptable salt or stereoisomer thereof, selected from the following:

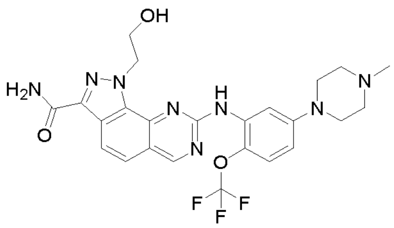

1

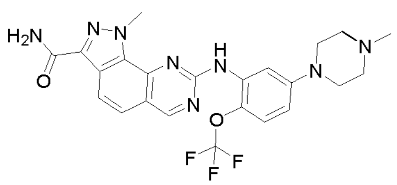

3

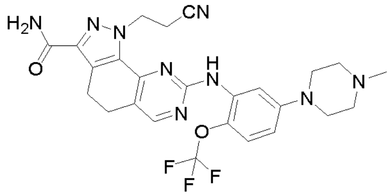

4

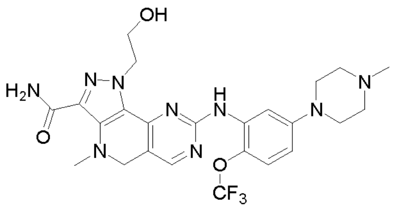

5

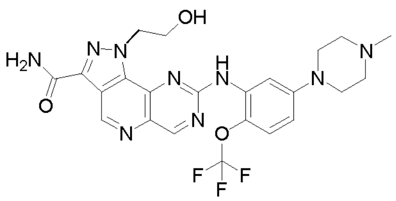

6

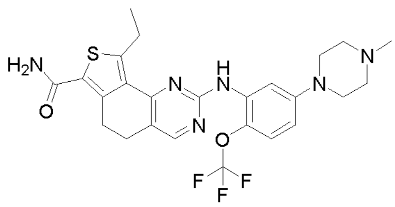

7

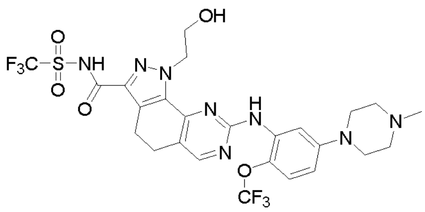

8

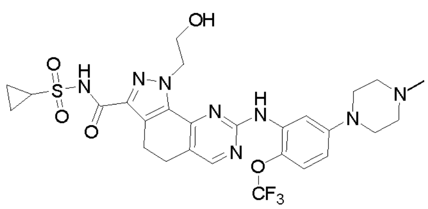

9

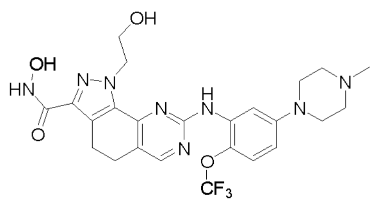

10

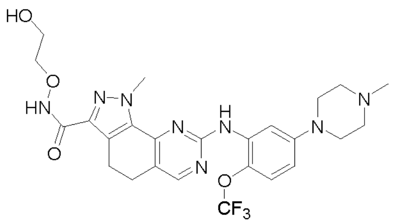

11

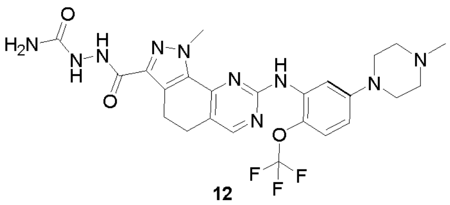

12

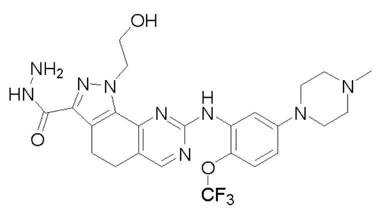

13

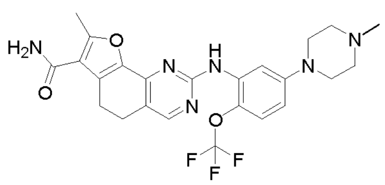

14

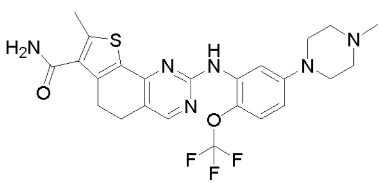

15

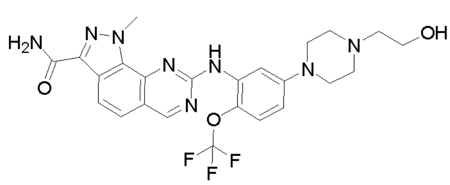

16

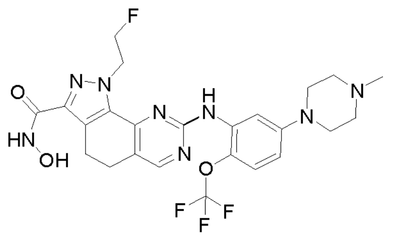

17

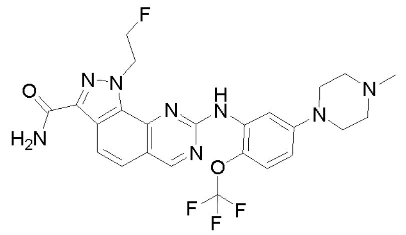

18

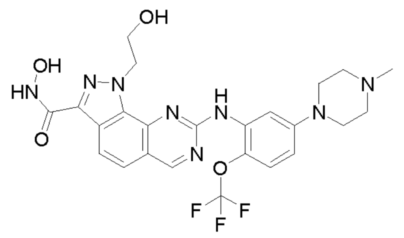

19

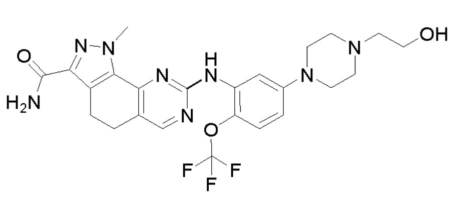

20

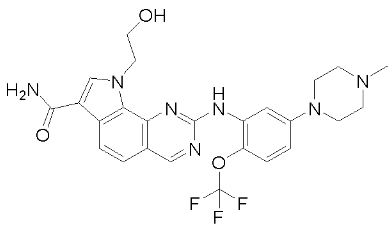

21

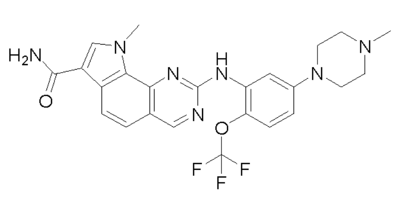

22

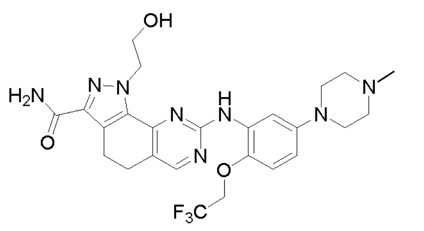

24

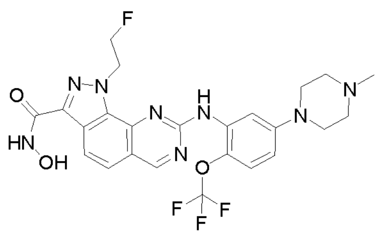

25

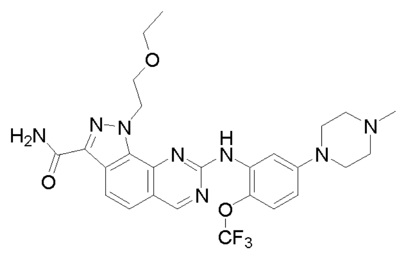

26

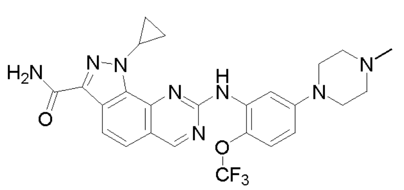

27

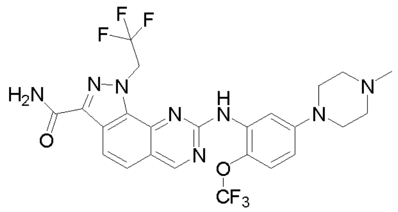

28

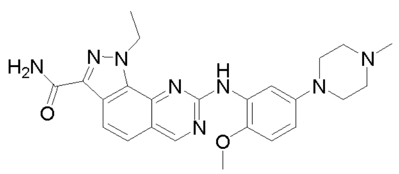

29

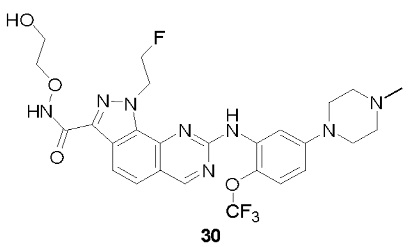

30

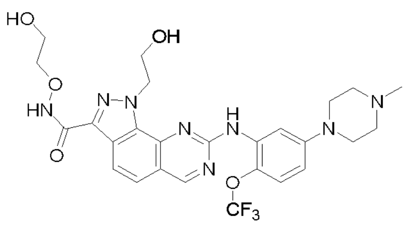

31

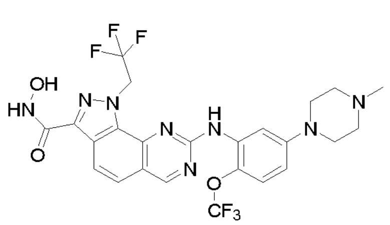

32

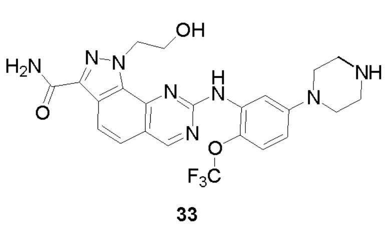

33

**11.** A pharmaceutical composition, comprising the compound, or the pharmaceutically acceptable salt or stereoisomer thereof according to any one of claims 1-10 and a pharmaceutically acceptable carrier.

**12.** Use of the compound, or the pharmaceutically acceptable salt or stereoisomer thereof according to any one of claims 1-10 or the pharmaceutical composition according to claim 11 in the preparation of a drug for the prevention or treatment of a disease caused by and/or related to dysregulation of protein kinase activity.

**13.** Use of the compound, or the pharmaceutically acceptable salt or stereoisomer thereof according to any one of claims 1-10 or the pharmaceutical composition according to claim 11 in the preparation of a drug for the prevention or treatment of a disease caused by and/or related to dysregulation of PLK1 kinase activity.

**14.** The use according to claim 12 or 13, wherein the disease includes: a cancer, a cell proliferative disease, a viral infection, an autoimmune disease and a neurodegenerative disease.

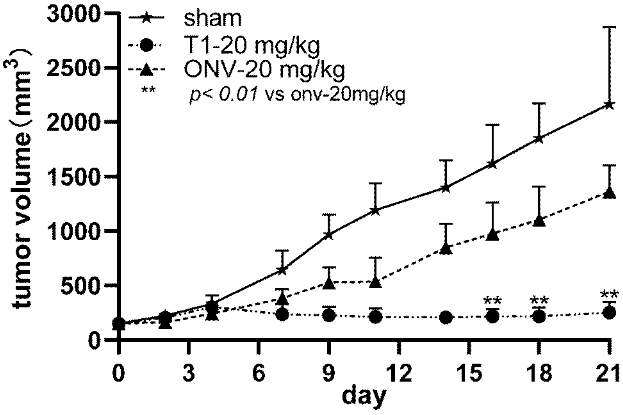


Fig. 1

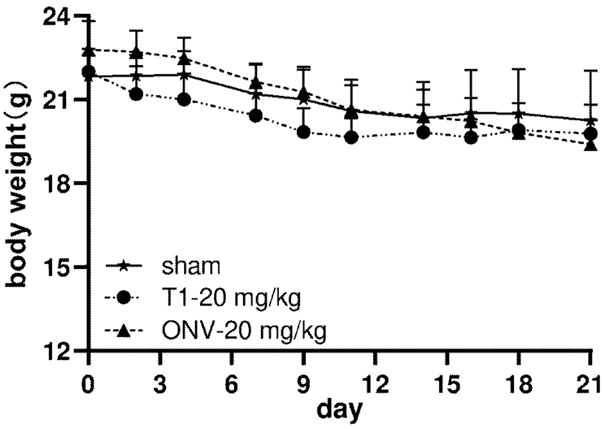


Fig. 2

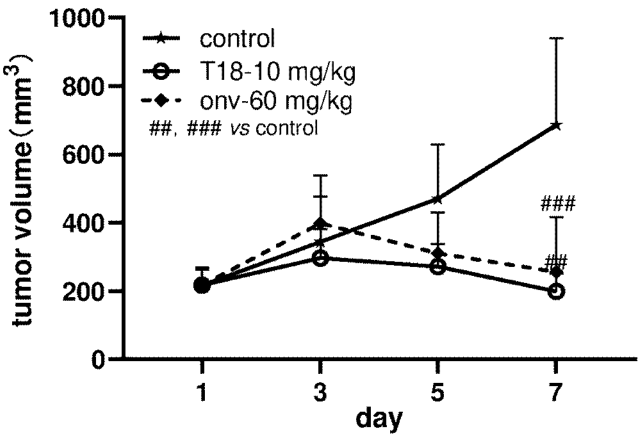


Fig. 3

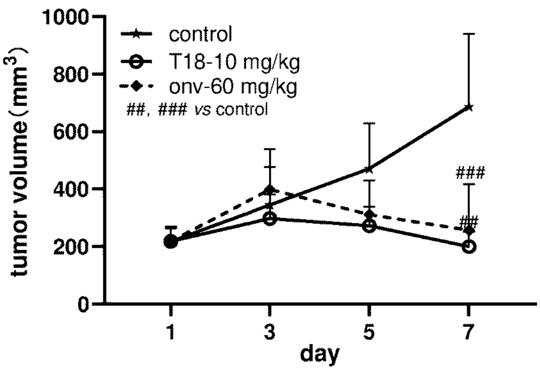
1000
800
600
400
200
0
tumor volume (mm3)
control
T18-10 mg/kg
onv-60 mg/kg
##, ### vs control
###
##
1
3
5
7
day

Fig. 4

**INTERNATIONAL SEARCH REPORT**

International application No.
**PCT/CN2022/137824**

**A. CLASSIFICATION OF SUBJECT MATTER**

C07D487/04(2006.01)i;C07D487/14(2006.01)i;C07D491/048(2006.01)i;C07D495/04(2006.01)i;C07D495/14(2006.01)i; A61K31/517(2006.01)i;A61K31/519(2006.01)i;A61P35/00(2006.01)i;A61P37/00(2006.01)i;A61P25/00(2006.01)i;A61P31/12(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNPAT, WPI, CNKI, 万方, WANFANG, STN(CAPLUS, REGISTRY, MARPAT): 山东绿叶制药有限公司, PLK1, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 101563351 A (NERVIANO MEDICAL SCIENCES S.R.L.) 21 October 2009 (2009-10-21)<br>description, p. 4, paragraphs 4-5, and pp. 32-38, embodiments 13-45, and claims 1-6 | 1-14 |
| X | CN 102079746 A (NERVIANO MEDICAL SCIENCES S.R.L.) 01 June 2011 (2011-06-01)<br>claims 1-10 and 15, and description, paragraphs [0020] and [0352]-[0389], and embodiments 1-71 | 1-14 |
| X | CN 102470136 A (NERVIANO MEDICAL SCIENCES S.R.L. et al.) 23 May 2012 (2012-05-23)<br>description, paragraphs [0079]-[0080] | 1-14 |
| X | WO 2021194320 A1 (UPPTHERA, INC.) 30 September 2021 (2021-09-30)<br>description, paragraphs [60]-[61], and embodiment 5 | 1-14 |
| PX | WO 2022166725 A1 (MEDSHINE DISCOVERY INC.) 11 August 2022 (2022-08-11)<br>claims 1-13 | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 February 2023** | **08 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/137824**

**C. DOCUMENTS CONSIDERED TO BE RELEVANT**

| Category* | Citation of document, with indication, where appropriate, of the relevant passages | Relevant to claim No. |
|---|---|---|
| PX | CN 114685520 A (WUHAN YUXIANG MEDICAL TECHNOLOGY CO., LTD.) 01 July 2022 (2022-07-01) claims 1-10 | 1-14 |
| PX | WO 2022143576 A1 (NEWSOARA BIOPHARMA CO., LTD.) 07 July 2022 (2022-07-07) abstract, and description, embodiments 1-20, and claims 1-32 | 1-14 |
| X | ANANT, A. et al. "A Computational approach to discover potential quinazoline derivatives against CDK4/6 kinase" *Journal of Molecular Structure,* Vol. 1245, 13 July 2021 (2021-07-13), 131079 | 1-14 |
| X | JOSHI, A. J. et al. "Strategies to select the best pharmacophore model: a case study in pyrazoloquinazoline class of PLK-1 inhibitors" *Med. Chem. Res.,* Vol. 27, No. 1, 12 September 2017 (2017-09-12), pp. 234-260 | 1-14 |
| X | HE, Lufen et al. "Identification of potent virtual leads to design novel PLK1 inhibitors: pharmacophore modelling, virtual screening and molecular docking studies" *Molecular Simulation,* Vol. 39, No. 8, 06 March 2013 (2013-03-06), pp. 640-650 | 1-14 |
| X | BERIA, I. et al. "NMS-P937, a 4,5-dihydro-1H-pyrazolo[4,3-h]quinazoline derivative as potent and selective Polo-like kinase 1 inhibitor" *Bioorganic Medicinal Chemistry Letters,* Vol. 21, 21 March 2011 (2011-03-21), pp. 2969-2974 | 1-14 |
| X | REGISTRY. "RN: 2650909-28-5, 2650907-15-4, 2650899-59-3, 2650818-68-9, 2650598-27-7, 2650469-82-0, 2650469-78-4, 2650416-71-8, 2650337-17-8, 2650316-22-4" *STN,* 08 July 2021 (2021-07-08), pp. 1-10 | 1-10 |

Form PCT/ISA/210 (second sheet) (July 2022)

**INTERNATIONAL SEARCH REPORT**
**Information on patent family members**

International application No.
**PCT/CN2022/137824**

| Patent document cited in search report | Publication date (day/month/year) | Patent family member(s) | Publication date (day/month/year) |
|---|---|---|---|
| CN 101563351 A | 21 October 2009 | UA 102219 C2 | 25 June 2013 |
| | | ZA 200904912 A | 29 September 2010 |
| CN 102079746 A | 01 June 2011 | US 2017050972 A1 | 23 February 2017 |
| | | US 9637497 B2 | 02 May 2017 |
| | | RS 52899 B | 28 February 2014 |
| | | US 2015158876 A1 | 11 June 2015 |
| | | US 9464090 B2 | 11 October 2016 |
| | | BR 122019010200 B1 | 15 December 2020 |
| | | BR 122019010200 B8 | 27 July 2021 |
| | | US 2007185143 A1 | 09 August 2007 |
| | | US 7482354 B2 | 27 January 2009 |
| | | MY 142019 A | 16 August 2010 |
| | | HRP 20050967 A2 | 31 May 2007 |
| | | HRP 20050967 B1 | 28 March 2014 |
| | | HRP 20050967 B8 | 25 April 2014 |
| | | US 2013338148 A1 | 19 December 2013 |
| | | US 8981089 B2 | 17 March 2015 |
| | | GEP 20094664 B | 10 April 2009 |
| | | CR 8102 A | 09 August 2006 |
| | | US 2019194214 A1 | 27 June 2019 |
| | | ECSP 056194 A | 19 April 2006 |
| | | SI 1636236 T1 | 31 January 2014 |
| | | TW 200505924 A | 16 February 2005 |
| | | TWI 349672 B | 01 October 2011 |
| | | EP 1636236 A1 | 22 March 2006 |
| | | EP 1636236 B1 | 09 October 2013 |
| | | OA 13170 A | 13 December 2006 |
| | | JP 2007502851 A | 15 February 2007 |
| | | JP 5043432 B2 | 10 October 2012 |
| | | AU 2004240772 A1 | 02 December 2004 |
| | | AU 2004240772 B2 | 28 April 2011 |
| | | BRPI 0410563 A | 20 June 2006 |
| | | BRPI 0410563 B1 | 01 December 2020 |
| | | BRPI 0410563 B8 | 25 May 2021 |
| | | US 2021300935 A1 | 30 September 2021 |
| | | TNSN 05298 A1 | 10 July 2007 |
| | | MXPA 05012573 A | 22 February 2006 |
| | | CY 1114708 T1 | 05 October 2016 |
| | | NZ 543661 A | 31 May 2009 |
| | | MEP 25908 A | 10 June 2010 |
| | | ME 00142 B | 10 October 2010 |
| | | WO 2004104007 A1 | 02 December 2004 |
| | | RS 20050944 A | 05 June 2008 |
| | | ES 2436524 T3 | 02 January 2014 |
| | | UA 80763 C2 | 25 October 2007 |
| | | AR 044543 A1 | 21 September 2005 |
| | | IS 8132 A | 18 November 2005 |
| | | IS 2939 B | 15 March 2016 |
| | | PT 1636236 E | 16 December 2013 |
| | | NO 20055496 D0 | 21 November 2005 |

Form PCT/ISA/210 (patent family annex) (July 2022)

**INTERNATIONAL SEARCH REPORT**
**Information on patent family members**

International application No.
**PCT/CN2022/137824**

| Patent document cited in search report | Publication date (day/month/year) | Patent family member(s) | Publication date (day/month/year) |
|---|---|---|---|
| | | NO 20055496 L | 14 February 2006 |
| | | NO 334992 B1 | 18 August 2014 |
| | | ZA 200509486 B | 25 September 2008 |
| | | EA 200501849 A1 | 30 June 2006 |
| | | EA 010904 B1 | 30 December 2008 |
| | | IL 172046 A | 30 March 2017 |
| | | DK 1636236 T3 | 09 December 2013 |
| | | US 2017190714 A1 | 06 July 2017 |
| | | US 10280176 B2 | 07 May 2019 |
| | | PL 1636236 T3 | 28 February 2014 |
| | | CO 5721006 A2 | 31 January 2007 |
| | | AR 102722 A2 | 22 March 2017 |
| | | AP 200503452 A0 | 31 December 2005 |
| | | AP 2005003452 A0 | 30 October 2009 |
| | | CA 2526578 A1 | 02 December 2004 |
| | | CA 2526578 C | 24 January 2012 |
| | | US 2009124605 A1 | 14 May 2009 |
| | | US 8541429 B2 | 24 September 2013 |
| | | KR 20060015294 A | 16 February 2006 |
| | | KR 101084871 B1 | 21 November 2011 |
| CN 102470136 A | 23 May 2012 | JP 2013500299 A | 07 January 2013 |
| | | JP 5851990 B2 | 03 February 2016 |
| | | TW 201109334 A | 16 March 2011 |
| | | AR 077422 A1 | 24 August 2011 |
| | | WO 2011012534 A1 | 03 February 2011 |
| | | US 2012157468 A1 | 21 June 2012 |
| | | US 8648078 B2 | 11 February 2014 |
| | | EP 2459196 A1 | 06 June 2012 |
| | | EP 2459196 B1 | 09 September 2015 |
| | | ES 2553114 T3 | 04 December 2015 |
| WO 2021194320 A1 | 30 September 2021 | KR 20210150341 A | 10 December 2021 |
| | | KR 102313752 B1 | 19 October 2021 |
| | | KR 102313753 B1 | 19 October 2021 |
| | | EP 3911654 A1 | 24 November 2021 |
| | | EP 3911654 A4 | 11 May 2022 |
| | | KR 20210122164 A | 08 October 2021 |
| | | KR 20210122162 A | 08 October 2021 |
| | | KR 102337029 B1 | 09 December 2021 |
| | | KR 102319264 B1 | 01 November 2021 |
| | | WO 2021194318 A1 | 30 September 2021 |
| | | WO 2021194319 A1 | 30 September 2021 |
| | | WO 2021194321 A1 | 30 September 2021 |
| | | KR 20210122163 A | 08 October 2021 |
| | | KR 20210122165 A | 08 October 2021 |
| | | JP 2022539579 A | 12 September 2022 |
| WO 2022166725 A1 | 11 August 2022 | None | |
| CN 114685520 A | 01 July 2022 | None | |
| WO 2022143576 A1 | 07 July 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101300251 B
- CN 101563351 B